(19) Europäisches Patentamt
European Patent Office
Office européen des brevets

(11) EP 4 427 763 A1

(12) EUROPEAN PATENT APPLICATION

(43) Date of publication:
**11.09.2024 Bulletin 2024/37**

(21) Application number: **23305290.1**

(22) Date of filing: **06.03.2023**

(51) International Patent Classification (IPC):
***A61K 47/68*** *(2017.01)* ***A61P 35/00*** *(2006.01)*
***A61K 39/395*** *(2006.01)*

(52) Cooperative Patent Classification (CPC):
**A61K 47/6853; A61K 47/68033; A61P 35/00; C07K 16/2818; C07K 16/2863; C07K 16/3007;**
C07K 2317/76

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Sanofi**
**75017 Paris (FR)**
- **ELI LILLY AND CO.**
**Indianapolis, IN 46285 (US)**

(72) Inventor: **The designation of the inventor has not yet been filed**

(74) Representative: **Cabinet Nony**
**11 rue Saint-Georges**
**75009 Paris (FR)**

Remarks:
The complete document including Reference Table(s) and the Sequence Listing(s) can be downloaded from the EPO website

# (54) ANTITUMOR COMBINATIONS CONTAINING ANTI-CEACAM5 ANTIBODY-DRUG CONJUGATES, ANTI-VEGFR-2 ANTIBODIES AND ANTI-PD1/PD-L1 ANTIBODIES

(57) The disclosure relates to a combination of (i) an antibody-drug conjugate (ADC) comprising an anti-CEACAM5 antibody, (ii) an anti-VEGFR2 antibody, and (iii) an anti-PD-1 antibody or anti-PD-L1 antibody, for use in the treatment of a cancer

EP 4 427 763 A1

## Description

### [REFERENCE TO SEQUENCE LISTING]

The instant application contains a Sequence Listing which has been submitted electronically in.xml format and is hereby incorporated by reference in its entirety. Said.xml copy, created on January 23, 2023, is named PR94533_EP_SANOFI.xml.

### [TECHNICAL FIELD]

The present disclosure relates to the field of therapeutic treatment of cancers that express CEACAM5. Certain aspects of the disclosure relate to combination therapies with an immunoconjugate comprising an anti-CEACAM5 antibody, with an anti-VEGFR-2 antibody, and with an anti-PD-1 or anti-PD-L1 agent, to treat cancer, including lung, gastric, gastroesophageal junction, esophageal, and pancreatic cancers.

### [TECHNICAL BACKGROUND]

According to the World Health Organization, cancer was the second leading cause of death globally and responsible for approx. 9.6 million in 2018. Thus, there is a continued need for providing improved drug combinations and regimens for the treatment of cancer.

Carcino-embryonic antigen (CEA) is a glycoprotein involved in cell adhesion. CEA was first identified in 1965 (Gold and Freedman, J Exp Med, 121, 439, 1965) as a protein normally expressed by fetal gut during the first six months of gestation, and found in cancers of the pancreas, liver, and colon. The CEA family belongs to the immunoglobulin superfamily. The CEA family, which consists of 18 genes, is sub-divided in two sub-groups of proteins: the carcinoembryonic antigen-related cell adhesion molecule (CEACAM) sub-group and the pregnancy-specific glycoprotein subgroup (Kammerer & Zimmermann, BMC Biology 2010, 8:12).

In humans, the CEACAM sub-group consists of 7 members: CEACAM1, CEACAM3, CEACAM4, CEACAM5, CEACAM6, CEACAM7 and CEACAM8. Numerous studies have shown that CEACAM5, identical to the originally identified CEA, is highly expressed on the surface of colorectal, gastric, lung, breast, prostate, ovary, cervix, and bladder tumor cells and weakly expressed in few normal epithelial tissues such as columnar epithelial and goblet cells in colon, mucous neck cells in the stomach and squamous epithelial cells in esophagus and cervix (Hammarström et al, 2002, in "Tumor markers, Physiology, Pathobiology, Technology and Clinical Applications" Eds. Diamandis E. P. et al., AACC Press, Washington pp 375). Thus, CEACAM5 may constitute a therapeutic target suitable for tumor specific targeting approaches, such as antibody-drug conjugates (ADCs).

Antibody-drug conjugates (ADCs) comprise an antibody attached to a chemotherapeutic agent such as a cytotoxic agent or a growth inhibitory agent or a cytostatic agent. The chemotherapeutic agent is typically attached to the antibody via a chemical linker. These antibody-drug conjugates (ADCs) have great potential in cancer chemotherapy and enable selective delivery of a potent chemotherapeutic agent to target cancer cells, resulting in improved efficacy, reduced systemic toxicity, and improved pharmacokinetics, pharmacodynamics and biodistribution compared to traditional chemotherapy. To date, hundreds of diverse antibody-drug conjugates (ADCs) have been developed against various cancers, of which several have been approved for human use.

The mechanism of action of antibody drug conjugates (ADCs) begins with its binding to a specific antigen, sufficiently expressed on the tumor cells in order to achieve a selective and efficient internalization of the drug. Selectively targeting potent cytotoxic agents to tumor cells using ADCs has now been shown to be an effective strategy for the treatment of cancer, as demonstrated by the recent approvals of brentuximab vedotin for the treatment of Hodgkin lymphoma and trastuzumab emtansine (T-DM1) for the treatment of relapsed metastatic HER2+ breast cancer (Younes A, Gopal AK, Smith SE, Ansell SM, Rosenblatt JD, Savage KJ, et al. Results of a pivotal phase II study of brentuximab vedotin for patients with relapsed or refractory Hodgkin's lymphoma. J Clin Oncol. 2012;30(18):2183-9; Verma S, Miles D, Gianni L, Krop IE, Welslau M, Baselga J, et al. Trastuzumab emtansine for HER2-positive advanced breast cancer. N Engl J Med. 2012;19:1783-91). Many other malignant diseases with unmet medical needs, such as solid tumor cancers, could benefit from such therapeutic options.

The international patent application published as WO 2014/079886 discloses an antibody binding to the A3-B3 domain of human and *Macaca fascicularis* CEACAM5 proteins and which does not significantly cross-react with human CEACAM1, human CEACAM6, human CEACAM7, human CEACAM8, *Macaca fascicularis* CEACAM1, *Macaca fascicularis* CEACAM6, and *Macaca fascicularis* CEACAM8. This antibody has been conjugated to a maytansinoid, thereby providing an antibody-drug conjugate having a significant cytotoxic activity on MKN45 human gastric cancer cells, with $IC_{50}$ values ≤ 1 nM.

huMAb2-3-SPDB-DM4 disclosed in WO 2014/079886 is an immunoconjugate (antibody-drug conjugate, ADC)

comprising a humanized anti-CEACAM5 antibody linked to maytansinoid derivative 4 (DM4), a potent antimitotic agent that inhibits microtubule assembly. DM4 is covalently bound to the antibody through an optimized linker SPDB [N-succinimidyl-4-(2-pyridyldithio)butanoic acid] that is stable in plasma and cleavable inside cells. After binding and internalization in targeted cancer cells, huMAb2-3-SPDB-DM4 is degraded, releasing cytotoxic DM4 metabolites. huMAb2-3-SPDB-DM4 is also known as tusamitamab ravtansine.

WO 2020/161214, the entire content of which is incorporated herein by reference, discloses use of anti-CEACAM5 immunoconjugates (ADCs) for treating lung cancer.

The Vascular Endothelial Growth Factor (VEGF) is regarded as the key pro-angiogenic factor that drives tumor angiogenesis. VEGF expression is highly deregulated in primary tumors and in metastatic lesions. In tumors, VEGF is expressed at high levels and by a multiplicity of cell types including cancer cells, tumor stroma and invading myeloid cells leading to endothelial cells hyperproliferation and loss of the guidance mechanisms of angiogenic sprouting.

The activation of Vascular Endothelial Growth Factor Receptor 2 (VEGFR-2) by VEGF is regarded as the most critical driver of tumor angiogenesis. VEGF has been shown to be expressed at high levels in many different types of carcinomas. The auto-phosphorylation of the VEGFR-2 kinase is one of the earliest events upon VEGF binding and is critical for activation of the kinase and subsequent phosphorylation events on the VEGFR-2 receptor. In consequence, antagonist antibodies to VEGFR-2 were produced and studied. In particular, ramucirumab (CAS number 947687-13-0) is a fully human IgG1 monoclonal antibody that binds to the ligand-binding site of VEGFR-2 and prevents its activation. Ramucirumab received approval for use as a monotherapy for hepatocellular carcinoma; as a monotherapy and in combination with chemotherapy in metastatic gastric, gastroesophageal junction adenocarcinoma (GEJ) or metastatic colorectal cancer; and in combination with chemotherapy for metastatic non-small cell lung cancer.

Immune checkpoint inhibitors (ICIs), administered alone or in combination with other anticancer therapies, have shown activity in treating a variety of tumor types, including non-small-cell lung cancer (NSCLC), with a manageable safety profile when combined with cytotoxic agents. Pembrolizumab (KEYTRUDA®), a humanized IgG4 monoclonal antibody against programmed cell death protein 1 (PD-1), is indicated as a single agent as the first-line treatment of patients with NSCLC expressing programmed death-ligand 1 protein (PD-L1).

Lung cancer is one of the most commonly diagnosed cancers and is the leading cause of cancer-related mortality worldwide. Non-small-cell lung cancer (NSCLC) accounts for 85% of all lung cancers and comprises several histopathological subtypes, of which adenocarcinoma (60%) and squamous-cell carcinoma (15%) are the most common.

The majority of patients with NSCLC presents an advanced stage at the time of diagnosis. These patients have a median overall survival of up to 8 to 12 months, and in 2015, a 5 year survival rate of approximately 25%. About 15% to 20% of patients with NSCLC have tumors with key genomic alterations that are amenable to targeted therapy, which include epidermal growth factor receptor (EGFR) mutations and ROS receptor tyrosine kinase 1 (ROS1) and anaplastic lymphoma kinase (ALK) rearrangements.

Until recently, the only available treatment option for advanced or metastatic NSQ NSCLC lacking targetable mutations was chemotherapy. Systemic therapy with platinum-based doublet regimens, with or without maintenance therapy, is the current first-line treatment for patients with advanced NSCLC. The standard second-line treatment for NSCLC has been docetaxel; docetaxel's activity was found to be enhanced by the addition of ramucirumab.

More recently, immunotherapy has initiated a new paradigm for the treatment of NSCLC. In particular, monoclonal antibodies targeting the programmed death-1 receptor (PD-1)/PD ligand 1 (PD -L1) pathway have emerged as powerful new therapeutic tools in several clinical trials.

Three drugs targeting the PD-1 pathway (nivolumab, pembrolizumab and atezolizumab) have been approved for the treatment of both chemotherapy-naïve and previously treated advanced stage NSCLC, however only small subset (20% to 30%) of patients responds to these treatments. Despite improvement in outcomes with newer lines therapy, including anti-PD-1/PD-L1 antibodies, the disease often progresses. Additional therapeutic approaches are therefore needed to improve the clinical efficacy and health-related quality of life (HRQOL) in patients with advanced/metastatic NSCLC.

Despite recent progress in the treatment of cancer such as advanced NSCLC, there remains a need for effective new treatment at the time of disease progression.

## [SUMMARY]

According to one of its objects, the present disclosure relates to a combination of (i) an antibody-drug conjugate (ADC) comprising an anti-CEACAM5 antibody, (ii) an anti-VEGFR2 antibody, and (iii) an anti-PD-1 antibody or anti-PD-L1 antibody, for use in the treatment of a cancer.

The present disclosure relates to an anti-VEGFR-2 antibody which is for use in combination with an antibody-drug conjugate (ADC) comprising an anti-CEACAM5-antibody and a chemotherapeutic agent, and an anti-PD-1 antibody or anti-PD-L1 antibody, for the treatment of cancer

The present disclosure relates to an anti-PD-1 antibody or anti-PD-L1 antibody which is for use in combination

with an antibody-drug conjugate (ADC) comprising an anti-CEACAM5-antibody and a chemotherapeutic agent, and an anti-VEGFR-2 antibody, for the treatment of cancer.

In some embodiments, the present disclosure relates to an antibody-drug conjugate (ADC) comprising an anti-CEACAM5-antibody for use for treating cancer in combination with an anti-VEGFR2 antibody and an anti-PD-1 antibody or anti-PD-L1 antibody.

In the disclosure, "antibody-drug conjugate comprising an anti-CEACAM5-antibody" and "ADC" are used interchangeably.

In some embodiments, the ADC, the anti-VEGFR2 antibody, and the anti-PD-1 antibody or anti-PD-L1 antibody may be each in an effective amount.

In some embodiments, the use may be in a subject in need thereof.

In some embodiments, the cancer may be a CEACAM5-positive cancer, i.e., the cancer (or tumor) cells express CEACAM5.

In some embodiments, the anti-CEACAM5 antibody may comprise a CDR-H1 having the amino acid sequence of SEQ ID NO: 1, a CDR-H2 having the amino acid sequence of SEQ ID NO: 2, a CDR-H3 having the amino acid sequence of SEQ ID NO: 3, a CDR-L1 having the amino acid sequence of SEQ ID NO: 4, a CDR-L2 having the amino acid sequence NTR, and a CDR-L3 having the amino acid sequence of SEQ ID NO: 5.

In some embodiments, the anti-CEACAM5 antibody may comprise a variable domain of a heavy chain (VH) consisting of SEQ ID NO: 6 and a variable domain of a light chain (VL) consisting of SEQ ID NO: 7.

In some embodiments, the anti-CEACAM5 antibody may be tusamitamab.

In some embodiments, the antibody-drug conjugate may comprise at least one chemotherapeutic agent.

In some embodiments, the chemotherapeutic agent may be selected from the group consisting of radioisotopes, protein toxins, small molecule toxins, and any combination thereof.

In some embodiments, the small molecule toxin may be selected from antimetabolites, DNA-alkylating agents, DNA-cross-linking agents, DNA-intercalating agents, anti-microtubule agents, topoisomerase inhibitors, and any combination thereof.

In some embodiments, the anti-microtubule agent may be selected from taxanes, vinca alkaloids, maytansinoids, colchicine, podophyllotoxin, griseofulvin, and any combination thereof.

In some embodiments, the chemotherapeutic agent may be a maytansinoid.

In some embodiments, the maytansinoid may be selected from N2'-deacetyl-N2'-(3-mercapto-1-oxopropyl)-maytansine (DM1), N2'-deacetyl-N2'-(4-methyl-4-mercapto-1-oxopentyl)-maytansine (DM4), and combinations thereof.

In some embodiments, the anti-CEACAM5 antibody may be covalently attached via a cleavable or non-cleavable linker to the at least one chemotherapeutic agent.

In some embodiments, linker may be selected from N-succinimidyl pyridyldithiobutyrate (SPDB), 4-(pyridin-2-yldisulfanyl)-2-sulfo-butyric acid (sulfo-SPDB), and succinimidyl(N-maleimidomethyl) cyclohexane-1-carboxylate (SMCC).

In some embodiments, the anti-CEACAM5 antibody may be covalently attached via a cleavable linker to the at least one chemotherapeutic agent, the linker being selected from N-succinimidyl pyridyldithiobutyrate (SPDB), 4-(pyridin-2-yldisulfanyl)-2-sulfo-butyric acid (sulfo-SPDB), and succinimidyl(N-maleimidomethyl) cyclohexane-1-carboxylate (SMCC).

In some embodiments, the CEACAM5-antibody may comprise a heavy chain (VH) consisting of SEQ ID NO: 8 and a light chain (VL) consisting of SEQ ID NO: 9 (huMAb2-3), and which may be covalently linked to N2'-deacetyl-N-2'(4-methyl-4-mercapto-1-oxopentyl)-maytansine (DM4) via N-succinimidyl pyridyldithiobutyrate (SPDB).

In some embodiments, the antibody-drug conjugate may be characterized by a drug-to-antibody ratio (DAR) ranging from 1 to 10.

In some embodiments, the antibody-drug conjugate may be tusamitamab ravtansine.

In some embodiments, the cancer may be a CEACAM5 positive cancer.

In some embodiments, the cancer expresses CEACAM5 with moderate or high intensity defined by immunohistochemistry.

In some embodiments, the cancer may be a CEACAM5 positive cancer having a CEACAM5 immunohistochemical intensity ≥ 2+ in ≥ 1% and < 50% of cancer cells. Such cancer expresses CEACAM5 with moderate intensity.

In some embodiments, the cancer may be a CEACAM5 positive cancer having a CEACAM5 immunohistochemical intensity ≥ 2+ in ≥ 50% of cancer cells. Such cancer expresses CEACAM5 with high intensity.

In some embodiments, the cancer may be selected from hepatocellular carcinoma, colorectal cancer, gastric cancer, gastroesophageal junction adenocarcinoma (GEJ), esophageal cancer, lung cancer, uterine cervix cancer, pancreatic cancer, ovarian cancer, thyroid cancer, bladder cancer, endometrial cancer, breast cancer, liver cancer, biliary tract cancer (e.g., cholangiocarcinoma), prostate cancer, neuroendocrine cancer, and skin cancer.

In some embodiments, the cancer may be selected from gastric cancer, gastroesophageal junction (GEJ)

adenocarcinoma, esophageal cancer, pancreatic cancer and lung cancer.

In some embodiments, the cancer may be a gastric cancer, a gastroesophageal junction (GEJ) adenocarcinoma, or a esophageal cancer.

In some embodiments, the cancer may be a pancreatic cancer.

In some embodiments, the cancer may be a lung cancer.

In some embodiments, the lung cancer may be a non-squamous non-small cell lung cancer (NSQ NSCLC).

In some embodiments, the non-squamous non-small cell lung cancer may be an advanced or metastatic NSQ NSCLC.

In some embodiments, the non-squamous non-small cell lung cancer has no epidermal growth factor receptor (EGFR) sensitizing mutation or v-raf murine sarcoma viral oncogene homolog B1 (BRAF) mutation or anaplastic lymphoma kinase/c-ros oncogene 1 (ALK/ROS) alterations.

In some embodiments, the combination may be administered to a subject having not received any prior systemic chemotherapy for treatment of the cancer.

In some embodiments, (i) the antibody-drug conjugate, (ii) the anti-VEGFR2 antibody, and (iii) the anti-PD-1 antibody or anti-PD-L1 antibody may be formulated in the form of three separate pharmaceutical compositions, wherein (a) a first pharmaceutical composition may comprise the antibody-drug conjugate, (b) a second pharmaceutical composition may comprise the anti-VEGFR2 antibody, and (c) a third pharmaceutical composition may comprise the anti-PD-1 antibody or anti-PD-L1 antibody.

In some embodiments, (i) the antibody-drug conjugate, (ii) the anti-VEGFR2 antibody, and (iii) the anti-PD-1 antibody or anti-PD-L1 antibody may be administered simultaneously, separately or sequentially to a subject in need thereof.

In some embodiments, (i) the antibody-drug conjugate, (ii) the anti-VEGFR2 antibody, and (iii) the anti-PD-1 antibody or anti-PD-L1 antibody may be sequentially administered to a subject in need thereof.

In some embodiments, the anti-VEGFR-2 antibody may be administered before the antibody-drug conjugate.

In some embodiments, the antibody-drug conjugate may be administered before the anti-PD-1 antibody or anti-PD-L1 antibody.

In some embodiments, (i) the antibody-drug conjugate, (ii) the anti-VEGFR2 antibody, and (iii) the anti-PD-1 antibody or anti-PD-L1 antibody may be administered for at least one cycle of treatment.

In some embodiments, (i) the antibody-drug conjugate, (ii) the anti-VEGFR2 antibody, and (iii) the anti-PD-1 antibody or anti-PD-L1 antibody may be administered at day 1 of a first cycle of treatment.

In some embodiments, (i) the antibody-drug conjugate, (ii) the anti-VEGFR2 antibody, and (iii) the anti-PD-1 antibody or anti-PD-L1 antibody may be administered at day 1 of at least one additional cycle of treatment.

In some embodiments, a cycle of treatment may be 2 weeks or 3 weeks

In some embodiments, a cycle of treatment may be 3 weeks.

In some embodiments, the antibody-drug conjugate may be administered at a dose of 80 $mg/m^2$ to 170 $mg/m^2$, or at a dose of 80 $mg/m^2$ to 150 $mg/m^2$, or at a dose of 100 $mg/m^2$ to 120 $mg/m^2$.

In some embodiments, the antibody-drug conjugate may be administered at a dose of 150 $mg/m^2$ or 120 $mg/m^2$.

In some embodiments, the anti-VEGFR2 antibody may be administered at a dose of 6 mg/kg to 10 mg/kg, or at a dose of 8 mg/k to 10 mg/kg, or at a dose of 6 mg/kg, or at a dose of 8 mg/kg, or at a dose of 10 mg/kg.

In some embodiments, the anti-PD-1 antibody or the anti-PD-L1 antibody may be administered at a dose of 150 mg to 400 mg, or at a dose of 150 mg to 300 mg, or at a dose of 200 mg.

In some embodiments, the antibody-drug conjugate may be administered at a dose of 120 $mg/m^2$ to 150 $mg/m^2$, the anti-VEGFR2 antibody may be administered at a dose of 6 mg/kg to 10 mg/kg, and the anti-PD-1 antibody or the anti-PD-L1 antibody may be administered at a dose of 200 mg.

In some embodiments, at day 1 of a first and of at least one additional cycles of treatment, the antibody-drug conjugate may be administered at a dose of 120 $mg/m^2$ or 150 $mg/m^2$, and the cycles of treatment may be 3 weeks.

In some embodiments, at day 1 of a first and of at least one additional cycles of treatment, the antibody-drug conjugate may be administered at a dose of 150 $mg/m^2$, and the cycles of treatment may be 3 weeks.

In some embodiments, at day 1 of a first cycle of treatment the antibody-drug conjugate may be administered at a dose of 150 $mg/m^2$ and at day 1 of at least one additional cycle of treatment the antibody-drug conjugate may be administered at a dose of 120 $mg/m^2$, and the cycles of treatment may be 3 weeks.

In some embodiments, at day 1 of a first cycle of treatment, the anti-VEGFR-2 antibody may be administered at a dose of 6 mg/kg to 10 mg/kg, and the cycle of treatment may be 3 weeks.

In some embodiments, at day 1 of a first cycle of treatment, the anti-VEGFR-2 antibody may be administered at a dose of 10 mg/kg, and the cycle of treatment may be 3 weeks.

In some embodiments, at day 1 of a subsequent (or additional) cycle of treatment, the anti-VEGFR-2 antibody may be administered at a dose of 6 mg/kg to 10 mg/kg, and the cycle of treatment may be 3 weeks.

In some embodiments, at day 1 of a subsequent cycle of treatment, the anti-VEGFR-2 antibody may be administered at a dose of 6 mg/kg, 8 mg/kg, or 10 mg/kg, and the cycle of treatment may be 3 weeks.

In some embodiments, at day 1 of a first cycle of treatment the anti-VEGFR-2 antibody may be administered at a dose of 10 mg/kg, and at day 1 of at least one additional cycle of treatment the anti-VEGFR-2 antibody may be administered at a dose of 10 mg/kg, 8 mg/kg, or 6 mg/kg, and the cycle of treatment may be 3 weeks.

In some embodiments, at day 1 of a first and at least one subsequent cycles of treatment, the anti-PD-1 antibody or the anti-PD-L1 antibody may be administered at a dose of 200 mg, and the cycle of treatment may be 3 weeks.

In some embodiments, at day 1 of a first cycle of treatment and at day 1 of an at least one additional cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and the cycle of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$ or 150 mg/$m^2$, (ii) the anti-VEGFR-2 antibody may be administered at a dose of 6 mg/kg, 8 mg/kg, or 10 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of 6 mg/kg or 8 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of 6 mg/kg, and (iii) the anti-PD-1 antibody or the anti-PD-L1 antibody may be administered at a dose of 200 mg, and the cycles of treatment may be 3 weeks.

In some embodiments, the cancer may be a lung cancer, where the cancer may be a CEACAM5-positive cancer having a CEACAM5 immunohistochemical intensity ≥ 2+ in ≥ 50% of cancer cells or a CEACAM5 immunohistochemical intensity ≥ 2+ in ≥ 1% and < 50% of cancer cells, wherein (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, on day 1 of a first cycle, and the cycle may be 3 weeks.

In some embodiments, (i) the antibody-drug conjugate may be administered at a subsequent dose of 150 mg/$m^2$, (ii) the anti-VEGFR-2 antibody may be administered at a subsequent dose of 10 mg/kg, and (iii) the anti-PD-1 antibody or the anti-PD-L1 antibody may be administered at a subsequent dose of 200 mg, on day 1 of subsequent cycle(s), and the cycle(s) is/are 3 weeks.

In some embodiments, (i) the antibody-drug conjugate may be administered at a subsequent dose of 120 mg/$m^2$, (ii) the anti-VEGFR-2 antibody may be administered at a subsequent dose of 6 or 8 mg/kg, and (iii) the anti-PD-1 antibody or the anti-PD-L1 antibody may be administered at a subsequent dose of 200 mg, on day 1 of subsequent cycle(s), and the cycle(s) is/are 3 weeks.

In some embodiments, the anti-VEGFR-2 antibody may be administered before the antibody-drug conjugate, and the antibody-drug conjugate may be administered before the anti-PD-1 antibody or the anti-PD-L1 antibody.

In some embodiments, the anti-VEGFR-2 antibody may be ramucirumab.

In some embodiments, the anti-PD-1 antibody may be selected from the group consisting of pembrolizumab, nivolumab, cemiplimab, sintilimab, dostarlimab, and tislelizumab.

In some embodiments, the anti-PD-1 antibody may be pembrolizumab or sintilimab.

In some embodiments, the anti-PD-L1 antibody may be selected from the group consisting of atezolizumab, avelumab, and durvalumab.

In some embodiments, (i) the antibody-drug conjugate may be tusamitamab ravtansine, (ii) the anti-VEGFR-2 antibody may be ramucirumab, and (iii) the anti-PD-1 antibody may be pembrolizumab.

According to one of its objects, the present disclosure relates to a pharmaceutical composition comprising (i) an antibody-drug conjugate as disclosed herein, (ii) an anti-VEGFR-2 antibody, (iii) an anti-PD-1 antibody or an anti-PD-L1 antibody, and a pharmaceutically acceptable excipient.

In some embodiments, in a pharmaceutical composition disclosed herein, the anti-VEGFR-2 antibody may be ramucirumab.

In some embodiments, in a pharmaceutical composition disclosed herein, the anti-PD-1 antibody may be

pembrolizumab.

According to one of its objects, the present disclosure relates to a kit comprising (i) a pharmaceutical composition comprising an antibody-drug conjugate as disclosed herein and a pharmaceutically acceptable excipient, (ii) a pharmaceutical composition comprising an anti-VEGFR2 antibody and a pharmaceutically acceptable excipient, and (iii) a pharmaceutical composition comprising an anti-PD-1 antibody or an anti-PD-L1 antibody and a pharmaceutically acceptable excipient.

According to one of its objects, the present disclosure relates to a pharmaceutical composition as disclosed herein, or a kit as disclosed herein, for a use for treating a cancer.

According to one of its objects, the present disclosure relates to a use of a combination of (i) an antibody-drug conjugate as disclosed herein, (ii) an anti-VEGFR2 antibody, and (iii) an anti-PD-1 antibody or anti-PD-L1 antibody, for the manufacture of a pharmaceutical composition or a kit, for the treatment of a cancer.

According to one of its objects, the present disclosure relates to a method for treating a cancer in a subject in need thereof, the method comprising at least a step of administering sequentially, a combination of (i) an antibody-drug conjugate as disclosed herein, (ii) an anti-VEGFR2 antibody, and (iii) an anti-PD-1 antibody or anti-PD-L1 antibody.

**[DESCRIPTION OF THE FIGURES]**

**Figure 1:** represents the decision tree for triplet cohort for tusamitamab ravtansine.
**Figure 2:** represents a graphical description of study design.

**[BRIEF DESCRIPTION OF THE SEQUENCES]**

SEQ ID NO: 1-5 show the sequences CDR-H1, CDR-H2, CDR-H3, CDR-L1 and CDR-L3 of the anti-CEACAM5-antibody (huMAb2-3).

SEQ ID NO: 6 shows the sequence of the variable domain of the heavy chain (VH) of the anti-CEACAM5-antibody (huMAb2-3).

SEQ ID NO: 7 shows the sequence of the variable domain of the light chain (VL) of the anti-CEACAM5-antibody (huMAb2-3).

SEQ ID NO: 8 shows the heavy chain sequence of the anti-CEACAM5-antibody (huMAb2-3).

SEQ ID NO: 9 shows the light chain sequence of the anti-CEACAM5-antibody (huMAb2-3).

SEQ ID NO: 10 shows the heavy chain sequence of the anti-VEGFR-2 antibody Ramucirumab.

SEQ ID NO: 11 shows the light chain sequence of the anti-VEGFR-2 antibody ramucirumab.

SEQ ID NO: 12-17 show the sequences CDR-H1, CDR-H2, CDR-H3, CDR-L1, CDR-L2 and CDR-L3 of the anti-VEGFR-2 antibody ramucirumab.

**[DETAILED DESCRIPTION]**

**Definitions**

Unless otherwise defined herein, scientific and technical terms used in connection with the present disclosure shall have the meanings that are commonly understood by those of ordinary skill in the art. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, may provide one of skill with a general dictionary of many of the terms used in this disclosure. Exemplary methods and materials are described below, although methods and materials similar or equivalent to those described herein can also be used in the practice or testing of the present disclosure. In case of conflict, the present specification, including definitions, will control. Generally, nomenclature used in connection with,

and techniques of, cell and tissue culture, molecular biology, virology, immunology, microbiology, genetics, analytical chemistry, synthetic organic chemistry, medicinal and pharmaceutical chemistry, and protein and nucleic acid chemistry and hybridization described herein are those well-known and commonly used in the art. Enzymatic reactions and purification techniques are performed according to manufacturer's specifications, as commonly accomplished in the art or as described herein. Further, unless otherwise required by context, singular terms shall include pluralities and plural terms shall include the singular.

Units, prefixes, and symbols are denoted in their International System Units (Systéme International des Unités (SI)) accepted form. Numeric ranges are inclusive of the numbers defining the range. Unless otherwise indicated, amino acid sequences are written left to right in amino to carboxy orientation. The headings provided herein are not limitations of the various aspects of the disclosure. Accordingly, the terms defined immediately below are more fully defined by reference to the specification in its entirety.

All publications and other references mentioned herein are incorporated by reference in their entirety. Although a number of documents are cited herein, this citation does not constitute an admission that any of these documents forms part of the common general knowledge in the art.

Throughout this specification and embodiments, the words **"have"** and **"comprise,"** or variations such as **"has," "having," "comprises,"** or **"comprising,"** will be understood to imply the inclusion of a stated integer or group of integers but not the exclusion of any other integer or group of integers. It is understood that wherever aspects are described herein with the language **"comprising,"** otherwise analogous aspects described in terms of "consisting of" and/or "consisting essentially of" are also provided.

It is to be noted that the term **"a"** or **"an"** entity refers to one or more of that entity; for example, "a nucleotide sequence," is understood to represent one or more nucleotide sequences. As such, the terms "a" (or "an"), "one or more," and "at least one" can be used interchangeably herein.

Furthermore, **"and/or"** where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The term **"approximately"** or **"about"** is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, *e.g.,* 10 percent, up or down (higher or lower). In some embodiments, the term indicates deviation from the indicated numerical value by ±10%, ±5%, ±4%, ±3%, ±2%, ±1%, ±0.9%, ±0.8%, ±0.7%, ±0.6%, ±0.5%, ±0.4%, ±0.3%, ±0.2%, ±0.1%, ±0.05%, or ±0.01%. In some embodiments, "about" indicates deviation from the indicated numerical value by ±10%. In some embodiments, "about" indicates deviation from the indicated numerical value by ±5%. In some embodiments, "about" indicates deviation from the indicated numerical value by ±4%. In some embodiments, "about" indicates deviation from the indicated numerical value by ±3%. In some embodiments, "about" indicates deviation from the indicated numerical value by ±2%. In some embodiments, "about" indicates deviation from the indicated numerical value by ±1%. In some embodiments, "about" indicates deviation from the indicated numerical value by ±0.9%. In some embodiments, "about" indicates deviation from the indicated numerical value by ±0.8%. In some embodiments, "about" indicates deviation from the indicated numerical value by ±0.7%. In some embodiments, "about" indicates deviation from the indicated numerical value by ±0.6%. In some embodiments, "about" indicates deviation from the indicated numerical value by ±0.5%. In some embodiments, "about" indicates deviation from the indicated numerical value by ±0.4%. In some embodiments, "about" indicates deviation from the indicated numerical value by ±0.3%. In some embodiments, "about" indicates deviation from the indicated numerical value by ±0.1%. In some embodiments, "about" indicates deviation from the indicated numerical value by ±0.05%. In some embodiments, "about" indicates deviation from the indicated numerical value by ±0.01%.

An **"antibody"** may be a natural or conventional antibody in which two heavy chains are linked to each other by disulfide bonds and each heavy chain is linked to a light chain by a disulfide bond. There are two types of light chain, lambda (l) and kappa (k). There are five main heavy chain classes (or isotypes) which determine the functional activity of an antibody molecule: IgM, IgD, IgG, IgA and IgE. Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties, such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are

primarily from the hypervariable or complementarity determining regions (CDRs). Occasionally, residues from non-hypervariable or framework regions (FR) influence the overall domain structure and hence the combining site. Complementarity Determining Regions or CDRs therefore refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody antigen-binding site, therefore, includes six CDRs, comprising the CDR set from each of a heavy and a light chain V region.

**"Framework Regions"** (FRs) refer to amino acid sequences interposed between CDRs, i.e., to those portions of immunoglobulin light and heavy chain variable regions that are relatively conserved among different immunoglobulins in a single species. The light and heavy chains of an immunoglobulin each have four FRs, designated FR1-L, FR2-L, FR3-L, FR4-L, and FR1-H, FR2-H, FR3-H, FR4-H, respectively. A human framework region is a framework region that is substantially identical (about 85%, or more, in particular 90%, 95%, 97%, 99% or 100%) to the framework region of a naturally occurring human antibody.

In the context of the disclosure, CDR/FR definition in an immunoglobulin light or heavy chain is to be determined based on IMGT definition (Lefranc et al. Dev. Comp. Immunol., 2003, 27(1):55-77; www.imgt.org).

As used herein, the term **"antibody"** denotes conventional antibodies and fragments thereof, as well as single domain antibodies and fragments thereof, in particular variable heavy chain of single domain antibodies, and chimeric, humanized, bispecific or multispecific antibodies.

As used herein, antibody or immunoglobulin also includes **"single domain antibodies"** which have been more recently described and which are antibodies whose complementary determining regions are part of a single domain polypeptide. Examples of single domain antibodies include heavy chain antibodies, antibodies naturally devoid of light chains, single domain antibodies derived from conventional four-chain antibodies, engineered single domain antibodies. Single domain antibodies may be derived from any species including, but not limited to mouse, human, camel, llama, goat, rabbit, bovine. Single domain antibodies may be naturally occurring single domain antibodies known as heavy chain antibody devoid of light chains. In particular, camelidae species, for example camel, dromedary, llama, alpaca and guanaco, produce heavy chain antibodies naturally devoid of light chain. Camelid heavy chain antibodies also lack the CH1 domain.

The variable heavy chain of these single domain antibodies devoid of light chains are known in the art as **"VHH"** or "**Nanobody**®". Similar to conventional VH domains, VHHs contain four FRs and three CDRs. VHH have advantages over conventional antibodies: they are about ten times smaller than IgG molecules, and as a consequence properly folded functional VHH can be produced by in vitro expression while achieving high yield. Furthermore, VHH are very stable, and resistant to the action of proteases. The properties and production of VHH have been reviewed by Harmsen and De Haard HJ (Appl. Microbiol. Biotechnol. 2007 Nov;77(1):13-22).

The term **"monoclonal antibody"** or **"mAb"** as used herein refers to an antibody molecule of a single amino acid sequence, which is directed against a specific antigen, and is not to be construed as requiring production of the antibody by any particular method. A monoclonal antibody may be produced by a single clone of B cells or hybridoma, but may also be recombinant, i.e., produced by protein engineering.

The term **"humanized antibody"** refers to an antibody which is wholly or partially of non-human origin, and which has been modified to replace certain amino acids, in particular in the framework regions of the VH and VL domains, in order to avoid or minimize an immune response in humans. The constant domains of a humanized antibody are most of the time human CH and CL domains.

**"Fragments"** of (conventional) antibodies comprise a portion of an intact antibody, in particular the antigen binding region or variable region of the intact antibody. Examples of antibody fragments include Fv, Fab, F(ab')2, Fab', dsFv, (dsFv)2, scFv, sc(Fv)2, diabodies, bispecific and multispecific antibodies formed from antibody fragments. A fragment of a conventional antibody may also be a single domain antibody, such as a heavy chain antibody or VHH.

The term **"Fab"** denotes an antibody fragment having a molecular weight of about 50,000 and antigen binding activity, in which about a half of the N-terminal side of the heavy chain and the entire light chain are bound together through a disulfide bond. It is usually obtained among fragments by treating IgG with a protease, such as papain.

The term **"F(ab')2"** refers to an antibody fragment having a molecular weight of about 100,000 and antigen binding activity, which is slightly larger than 2 identical Fab fragments bound via a disulfide bond of the hinge region. It is usually obtained among fragments by treating IgG with a protease, such as pepsin.

The term **"Fab'"** refers to an antibody fragment having a molecular weight of about 50,000 and antigen binding activity, which is obtained by cutting a disulfide bond of the hinge region of the F(ab')2.

A single chain Fv **("scFv")** polypeptide is a covalently linked VH::VL heterodimer which is usually expressed from a gene fusion including VH and VL encoding genes linked by a peptide-encoding linker. The human scFv fragment of the disclosure includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)2. "dsFv" is a

VH::VL heterodimer stabilized by a disulphide bond. "(dsFv)2" denotes two dsFv coupled by a peptide linker.

The term **"bispecific antibody"** or **"BsAb"** denotes an antibody which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP 2 050 764 A1.

The term **"multispecific antibody"** denotes an antibody which combines the antigen-binding sites of two or more antibodies within a single molecule.

The term **"diabodies"** refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains of the same chain, the domains are forced to pair with the complementary domains of another chain and create two antigen-binding sites.

An amino acid sequence **"at least 85% identical to a reference sequence"** is a sequence having, on its entire length, 85%, or more, in particular 90%, 91%, 92%, 93%, 94%, 95%, 96%, 97%, 98% or 99% sequence identity with the entire length of the reference amino acid sequence.

A percentage of **"sequence identity"** between amino acid sequences may be determined by comparing the two sequences, optimally aligned over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Optimal alignment of sequences for comparison is conducted by global pairwise alignment, e.g., using the algorithm of Needleman and Wunsch J. Mol. Biol. 48:443 (1970). The percentage of sequence identity can be readily determined for instance using the program Needle, with the BLOSUM62 matrix, and the following parameters gap-open=10, gap-extend=0.5.

A **"conservative amino acid substitution"** is one in which an amino acid residue is substituted by another amino acid residue having a side chain R group with similar chemical properties (e.g., charge, size or hydrophobicity). In general, a conservative amino acid substitution will not substantially change the functional properties of a protein. Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Conservative amino acids substitution groups can also be defined on the basis of amino acid size.

By **"purified"** and **"isolated"** it is meant, when referring to a polypeptide (i.e., the antibody of the disclosure) or a nucleotide sequence, that the indicated molecule is present in the substantial absence of other biological macromolecules of the same type. The term "purified" as used herein in particular means at least 75%, 85%, 95%, or 98% by weight, of biological macromolecules of the same type are present. An "isolated" nucleic acid molecule which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

As used herein, the term **"subject"** or **"patient"** denotes a mammal, such as a rodent, a feline, a canine, and a primate. In particular, a subject according to the disclosure is a human.

**"Administer"** or **"administering,"** as used herein refers to delivering to a subject a composition described herein. The composition can be administered to a subject using methods known in the art. In particular, the composition can be administered intravenously, subcutaneously, intramuscularly, intradermally, or via any mucosal surface, e.g., orally, sublingually, buccally, nasally, rectally, vaginally or via pulmonary route. In some embodiments, the administration is intravenous. In some embodiments, the administration is subcutaneous.

The terms **"treat"** or **"treatment"** or **"therapy"** refers to the administration of a compound or a composition according to the disclosure with the purpose to cure, heal, alleviate, relieve, alter, remedy, ameliorate, improve, or affect a disorder, the symptoms of the condition, or to prevent or delay the onset of the symptoms, complications, or otherwise arrest or inhibit further development of the disorder in a statistically significant manner. More particularly, **"treating"** or **"treatment"** includes any approach for obtaining beneficial or desired results in a subject's cancer condition. Beneficial or desired clinical results can include, but are not limited to, alleviation or amelioration of one or more cancer symptoms or conditions, diminishment or reduction of the extent of a cancer disease or of a cancer symptom, stabilizing, i.e., not worsening, the state of a cancer disease or of a cancer symptom, prevention of a cancer disease or of a cancer symptom's spread, delay or slowing of cancer disease or cancer symptom progression, amelioration or palliation of the cancer disease state, diminishment of the reoccurrence of cancer disease, and remission, whether partial or total and whether

detectable or undetectable. In other words, "treatment" as used herein includes any cure, amelioration, or reduction of a cancer disease or symptom. A "reduction" of a symptom or a disease means decreasing of the severity or frequency of the disease or symptom, or elimination of the disease or symptom.

As used herein, the terms **"effective amount"** refer to an amount that provides a therapeutic benefit in the treatment, prevention, or management of pathological processes considered. The specific amount that is therapeutically effective can be readily determined by an ordinary medical practitioner and may vary depending on factors such as the type and stage of pathological processes considered, the patient's medical history and age, and the administration of other therapeutic agents.

The unit "**mg/m$^2$**" indicates the amount of compound in mg per $m^2$ of subject body surface administered per dose. The person skilled in the art is aware how to determine the required amount of compound for the subject to be treated based on his body surface, which in turn may be calculated based on height and body weight.

The unit **"mg/kg"** indicates the amount of compound in mg per kg of subject body administered per dose. The person skilled in the art is aware how to determine the required amount of compound for the subject to be treated based on his body weight.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure. All publications mentioned herein are incorporated herein by reference to disclose and describe the methods and/or materials in connection with which the publications are cited.

The list of sources, ingredients, and components as described hereinafter are listed such that combinations and mixtures thereof are also contemplated and within the scope herein.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

All lists of items, such as, for example, lists of ingredients, are intended to and should be interpreted as Markush groups. Thus, all lists can be read and interpreted as items "selected from the group consisting of' the list of items "and combinations and mixtures thereof."

Referenced herein may be trade names for components including various ingredients utilized in the present disclosure. The inventors herein do not intend to be limited by materials under any particular trade name. Equivalent materials (e.g., those obtained from a different source under a different name or reference number) to those referenced by trade name may be substituted and utilized in the descriptions herein.

## Antibody-drug conjugate comprising an anti-CEACAM5-antibody

The present disclosure relates to an antibody-drug conjugate (ADC) comprising an anti-CEACAM5-antibody, or a fragment thereof, which is used in combination with an anti-VEGFR-2 antibody, or a fragment thereof, and an anti-PD-1 antibody or anti-PD-L1 antibody, or a fragment thereof, for the treatment of cancer.

The antibody-drug conjugate typically comprises an anti-CEACAM5-antibody and at least one chemotherapeutic agent. An antibody-drug conjugate (ADC) comprises an anti-CEACAM5-antibody conjugated to at least one chemotherapeutic agent. In the antibody-drug conjugate, the anti-CEACAM5-antibody may be covalently attached via a cleavable or non-cleavable linker to at least one chemotherapeutic agent.

### *Anti-CEACAM5-antibody*

According to an embodiment, the antibody-drug conjugate comprises an anti-CEACAM5-antibody or a fragment thereof.

According to an embodiment, the antibody-drug conjugate comprises a humanized anti-CEACAM5-antibody or a fragment thereof.

According to an embodiment, the anti-CEACAM5-antibody or a fragment thereof comprises a CDR-H1 consisting of SEQ ID NO: 1, CDR-H2 consisting of SEQ ID NO: 2, CDR-H3 consisting of SEQ ID NO: 3, CDR-L1 consisting of SEQ ID NO: 4, CDR-L2 consisting of amino acid sequence NTR, and CDR-L3 consisting of SEQ ID NO: 5.

In a further embodiment, the anti-CEACAM5-antibody or a fragment thereof comprises a variable domain of a heavy chain (VH) having at least 90% identity to SEQ ID NO: 6, and a variable domain of a light chain (VL) having at least 90% identity to SEQ ID NO: 7, wherein CDR1-H consists of SEQ ID NO: 1, CDR2-H consists of SEQ ID NO: 2, CDR3-H consists of SEQ ID NO: 3, CDR1-L consists of SEQ ID NO: 4, CDR2-L consists of amino acid sequence NTR, and CDR3-L consists of SEQ ID NO: 5.

In a further embodiment, the anti-CEACAM5-antibody or a fragment thereof comprises a variable domain of a heavy chain (VH) having at least 92%, at least 95%, at least 98% identity to SEQ ID NO: 6, and a variable domain of a light chain (VL) having at least 92%, at least 95%, at least 98% identity to SEQ ID NO: 7, wherein CDR1-H consists of SEQ ID NO: 1, CDR2-H consists of SEQ ID NO: 2, CDR3-H consists of SEQ ID NO: 3, CDR1-L consists of SEQ ID NO: 4, CDR2-L consists of amino acid sequence NTR, and CDR3-L consists of SEQ ID NO: 5.

In a further embodiment, the anti-CEACAM5-antibody or a fragment thereof comprises a variable domain of a heavy chain (VH) consisting of SEQ ID NO: 6 and a variable domain of a light chain (VL) consisting of SEQ ID NO: 7.

The anti-CEACAM5-antibody or a fragment thereof comprises in a further embodiment:

- a variable domain of heavy chain consisting of sequence EVQLQESGPGLVKPGGSLSLSCAAS**GFVFSSYD**MSWVRQTPERGLEWVAY**ISSGGGIT**YA PSTVKGRFTVSRDNAKNTLYLQMNSLTSEDTAVYYC**AAHYFGSSGPFAY**WGQGTLVTVSS (SEQ ID NO: 6, with CDRs shown in bold characters) in which FR1-H spans amino acid positions 1 to 25, CDR1-H spans amino acid positions 26 to 33 (SEQ ID NO: 1), FR2-H spans amino acid positions 34 to 50, CDR2-H spans amino acid positions 51 to 58 (SEQ ID NO: 2), FR3-H spans amino acid positions 59 to 96, CDR3-H spans amino acid positions 97 to 109 (SEQ ID NO: 3), and FR4-H spans amino acid positions 110 to 120, and

- a variable domain of light chain consisting of sequence DIQMTQSPASLSASVGDRVTITCRAS**ENIFSY**LAWYQQKPGKSPKLLVY**NTR**TLAEGVPSFS GSGSGTDFSLTISSLQPEDFATYYC**QHHYGTPFT**FGSGTKLEIK (SEQ ID NO: 7, with CDRs shown in bold characters) in which FR1-L spans amino acid positions 1 to 26, CDR1-L spans amino acid positions 27 to 32 (SEQ ID NO: 4), FR2-L spans amino acid positions 33 to 49, CDR2-L spans amino acid positions 50 to 52, FR3-L spans amino acid positions 53 to 88, CDR3-L spans amino acid positions 89 to 97 (SEQ ID NO: 5), and FR4-L spans amino acid positions 98 to 107.

In a further embodiment, the anti-CEACAM5-antibody or a fragment thereof comprises a heavy chain (HC) having at least 90% sequence identity to SEQ ID NO: 8 and a light chain (LC) having at least 90% sequence identity to SEQ ID NO: 9, wherein CDR1-H consists of SEQ ID NO: 1, CDR2-H consists of SEQ ID NO: 2, CDR3-H consists of SEQ ID NO: 3, CDR1-L consists of SEQ ID NO: 4, CDR2-L consists of amino acid sequence NTR, and CDR3-L consists of SEQ ID NO: 5.

In a further embodiment, the anti-CEACAM5-antibody or a fragment thereof comprises a heavy chain (HC) having at least 92%, at least 95%, at least 98% identity to SEQ ID NO: 8 and a light chain (LC) having at least 92%, at least 95%, at least 98% identity to SEQ ID NO: 9, wherein CDR1-H consists of SEQ ID NO: 1, CDR2-H consists of SEQ ID NO: 2, CDR3-H consists of SEQ ID NO: 3, CDR1-L consists of SEQ ID NO: 4, CDR2-L consists of amino acid sequence NTR, and CDR3-L consists of SEQ ID NO: 5.

In a further embodiment, the anti-CEACAM5-antibody or a fragment thereof comprises a heavy chain (HC) consisting of SEQ ID NO: 8 and a light chain (LC) consisting of SEQ ID NO: 9.

The anti-CEACAM5-antibody may also be a single domain antibody or a fragment thereof. In particular, a single domain antibody fragment may consist of a variable heavy chain (VHH) which comprises the CDR1-H, CDR2-H and CDR3-H of the antibodies as described above. The antibody may also be a heavy chain antibody, i.e., an antibody devoid of light chain, which may or may not contain a CH1 domain.

The single domain antibody or a fragment thereof may also comprise the framework regions of a camelid single domain antibody, and optionally the constant domain of a camelid single domain antibody.

The anti-CEACAM5-antibody may also be an antibody fragment, in particular a humanized antibody fragment, selected from the group consisting of Fv, Fab, F(ab')2, Fab', dsFv, (dsFv)2, scFv, sc(Fv)2, and diabodies.

The antibody may also be a bispecific or multispecific antibody formed from antibody fragments, at least one antibody fragment being an antibody fragment according to the disclosure. Multispecific antibodies are polyvalent protein complexes as described for instance in EP 2 050 764 A1 or US 2005/0003403 A1.

The anti-CEACAM5-antibody and fragments thereof can be produced by any technique well known in the art. In particular, said antibodies are produced by techniques as hereinafter described.

The anti-CEACAM5-antibody and fragments thereof can be used in an isolated (e.g., purified) from or contained in a vector, such as a membrane or lipid vesicle (e.g., a liposome).

The anti-CEACAM5-antibody and fragments thereof may be produced by any technique known in the art, such as, without limitation, any chemical, biological, genetic, or enzymatic technique, either alone or in combination.

Knowing the amino acid sequence of the desired sequence, one skilled in the art can readily produce the anti-CEACAM5-antibody and fragments thereof, by standard techniques for production of polypeptides. For instance, they can be synthesized using well-known solid phase method, in particular using a commercially available peptide synthesis apparatus (such as that made by Applied Biosystems, Foster City, California) and following the manufacturer's instructions. Alternatively, anti-CEACAM5-antibody and fragments thereof can be synthesized by recombinant DNA techniques as is well-known in the art. For example, these fragments can be obtained as DNA expression products after incorporation of DNA sequences encoding the desired (poly)peptide into expression vectors and introduction of such vectors into suitable eukaryotic or prokaryotic hosts that will express the desired polypeptide, from which they can be later isolated using well-known techniques.

Anti-CEACAM5-antibody and fragments thereof are suitably separated from the culture medium by conventional immunoglobulin purification procedures such as, for example, protein A-Sepharose, hydroxyapatite chromatography, gel electrophoresis, dialysis, or affinity chromatography.

Methods for producing humanized antibodies based on conventional recombinant DNA and gene transfection techniques are well known in the art (See, e. g., Riechmann L. et al. 1988; Neuberger MS. et al. 1985). Antibodies can be humanized using a variety of techniques known in the art including, for example, the technique disclosed in the application WO2009/032661, CDR-grafting (EP 239,400; PCT publication WO91/09967; U.S. Pat. Nos. 5,225,539; 5,530,101; and 5,585,089), veneering or resurfacing (EP 592,106; EP 519,596; Padlan EA (1991); Studnicka GM et al. (1994); Roguska MA. et al. (1994)), and chain shuffling (U.S. Pat. No.5,565,332). The general recombinant DNA technology for preparation of such antibodies is also known (see European Patent Application EP 125023 and International Patent Application WO 96/02576).

The Fab of the anti-CEACAM5-antibody can be obtained by treating an antibody which specifically reacts with CEACAM5 with a protease, such as papain. Also, the Fab of the anti-CEACAM5-antibody can be produced by inserting DNA sequences encoding both chains of the Fab of the anti-CEACAM5-antibody into a vector for prokaryotic expression, or for eukaryotic expression, and introducing the vector into prokaryotic or eukaryotic cells (as appropriate) to express the Fab of the anti-CEACAM5-antibody.

The F(ab')2 of the anti-CEACAM5-antibody can be obtained treating an antibody which specifically reacts with CEACAM5 with a protease, pepsin. Also, the F(ab')2 of the anti-CEACAM5-antibody can be produced by binding Fab' described below via a thioether bond or a disulfide bond.

The Fab' of the anti-CEACAM5-antibody can be obtained treating F(ab')2 which specifically reacts with CEACAM5 with a reducing agent, such as dithiothreitol. Also, the Fab' of the anti-CEACAM5-antibody can be produced by inserting DNA sequences encoding Fab' chains of the antibody into a vector for prokaryotic expression, or a vector for eukaryotic expression, and introducing the vector into prokaryotic or eukaryotic cells (as appropriate) to perform its expression.

The scFv of the anti-CEACAM5-antibody can be produced by taking sequences of the CDRs or VH and VL domains as previously described, constructing a DNA encoding an scFv fragment, inserting the DNA into a prokaryotic or eukaryotic expression vector, and then introducing the expression vector into prokaryotic or eukaryotic cells (as appropriate) to express the scFv. To generate a humanized scFv fragment, a well-known technology called CDR grafting may be used, which involves selecting the complementary determining regions (CDRs) according to the disclosure and grafting them onto a human scFv fragment framework of known three-dimensional structure (see, e. g., WO98/45322; WO 87/02671; US5,859,205; US5,585,089; US4,816,567; EP0173494).

In an embodiment, the anti-CEACAM5 antibody is tusamitamab (CAS [2349294-95-5].

### *Chemotherapeutic agents*

The antibody-drug conjugate for the use according to the present disclosure typically comprises at least one chemotherapeutic agent (also referred herein to cytotoxic agent). A chemotherapeutic agent as used herein refers to an agent that kills cells, including cancer cells. Such agents favorably stop cancer cells from dividing and growing and cause tumors to shrink in size. The expression "chemotherapeutic agent" is used herein interchangeably with the expressions "cytotoxic agent", "growth inhibitory agent" or "cytostatic drug".

The term "chemotherapeutic agent" as used herein refers to a substance that inhibits or prevents the function of cells and/or causes destruction of cells. The term "chemotherapeutic agent" is intended to include radioisotopes, enzymes, antibiotics, and toxins such as small molecule toxins or enzymatically active toxins of bacterial, fungal, plant or animal origin, including fragments and/or variants thereof, and the various antitumor or anticancer agents disclosed below. In some embodiments, the chemotherapeutic agent is an antimetabolite.

In a further embodiment, the chemotherapeutic agent is selected from the group consisting of radioisotopes, protein toxins, small molecule toxins, and combinations thereof.

Radioisotopes include radioactive isotopes suitable for treating cancer. Such radioisotopes generally emit mainly beta-radiation. In a further embodiment, the radioisotopes are selected from the group consisting of $At^{211}$, $Bi^{212}$,

$Er^{169}$, $I^{131}$, $I^{125}$, $Y^{90}$, $In^{111}$, $P^{32}$, $Re^{186}$, $Re^{188}$, $Sm^{153}$, $Sr^{89}$, radioactive isotopes of Lu, and combinations thereof. In an embodiment, the radioactive isotope is alpha-emitter isotope, more specifically $Th^{227}$, which emits alpha-radiation.

In a further embodiment, the small molecule toxins are selected from antimetabolites, DNA-alkylating agents, DNA-cross-linking agents, DNA-intercalating agents, anti-microtubule agents, topoisomerase inhibitors, and combinations thereof.

In a further embodiment, the anti-microtubule agent is selected from the group consisting of taxanes, vinca alkaloids, maytansinoids, colchicine, podophyllotoxin, gruseofulvin, and combinations thereof.

In some embodiment a chemotherapeutic agent may be a maytansinoid.

According to an embodiment, maytansinoids are selected from maytansinol, maytansinol analogs, and combinations thereof.

Examples of suitable maytansinol analogues include those having a modified aromatic ring and those having modifications at other positions. Such suitable maytansinoids are disclosed in U.S. Patent Nos. 4,424,219; 4,256,746; 4,294,757; 4,307,016; 4,313,946; 4,315,929; 4,331,598; 4,361,650; 4,362,663; 4,364,866; 4,450,254; 4,322,348; 4,371,533; 6,333,410; 5,475,092; 5,585,499; and 5,846,545.

In a further embodiment, the cytotoxic conjugates of the present disclosure utilize the thiol-containing maytansinoid (DM1), formally termed N2'-deacetyl-N2'-(3-mercapto-1-oxopropyl)-maytansine, as the cytotoxic agent. DM1 is represented by the following structural formula (I):

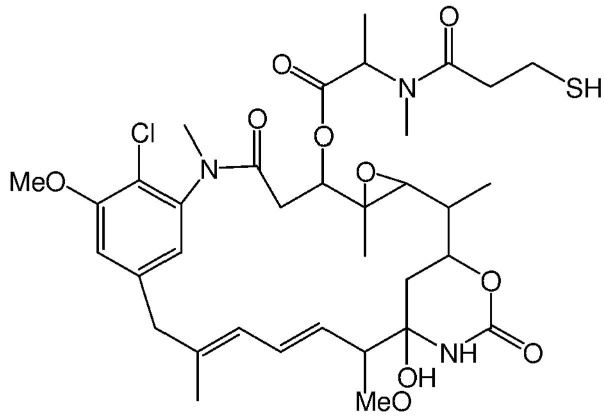

(I).

In a further embodiment, the cytotoxic conjugates of the present disclosure utilize the thiol-containing maytansinoid DM4, formally termed *N*2'-deacetyl-*N*-2'(4-methyl-4-mercapto-1-oxopentyl)-maytansine, as the cytotoxic agent. DM4 is represented by the following structural formula (II):

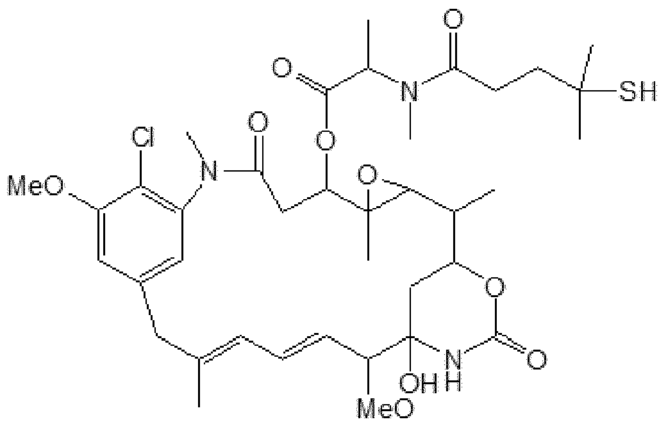

(II).

In further embodiments of the disclosure other maytansines, including thiol and disulfide-containing maytansinoids bearing a mono or di-alkyl substitution on the carbon atom bearing the sulfur atom, may be used. These include a maytansinoid having, at C-3, C-14 hydroxymethyl, C-15 hydroxy, or C-20 desmethyl, an acylated amino acid side chain with an acyl group bearing a hindered sulfhydryl group, wherein the carbon atom of the acyl group bearing the thiol functionality has one or two substituents, said substituents being $CH_3$, $C_2H_5$, linear or branched alkyl or alkenyl

having from 1 to 10 reagents and any aggregate which may be present in the solution.

Examples of these cytotoxic agents and of methods of conjugation are further given in the application WO 2008/010101 which is incorporated by reference.

The immunoconjugates according to the present disclosure can be prepared as described in the application WO 2004/091668, the entire content of which is incorporated herein by reference.

Accordingly, in a further embodiment, the maytansinoids are selected from the group consisting of *N*2'-deacetyl-*N*2'-(3-mercapto-1-oxopropyl)-maytansine (DM1) or *N*2'-deacetyl-*N*-2'(4-methyl-4-mercapto-1-oxopentyl)-maytansine (DM4), and combinations thereof.

In a further embodiment, in the antibody-drug conjugate, the anti-CEACAM5-antibody is covalently attached via a cleavable or non-cleavable linker to the at least one chemotherapeutic agent.

In a further embodiment, the linker is selected from the group consisting of N-succinimidyl pyridyldithiobutyrate (SPDB), 4-(pyridin-2-yldisulfanyl)-2-sulfo-butyric acid (sulfo-SPDB), and succinimidyl(N-maleimidomethyl) cyclohexane-1-carboxylate (SMCC).

In a further embodiment, the linker binds to a lysine or cysteine residue in the Fc region of the anti-CEACAM5 antibody. In a further embodiment, the linker forms a disulfide bond or a thioether bond with the maytansine.

In particular, the anti-CEACAM5-antibody-drug conjugate may be selected from the group consisting of:

the anti-CEACAM5-SPDB-DM4-antibody-drug conjugate of formula (III):

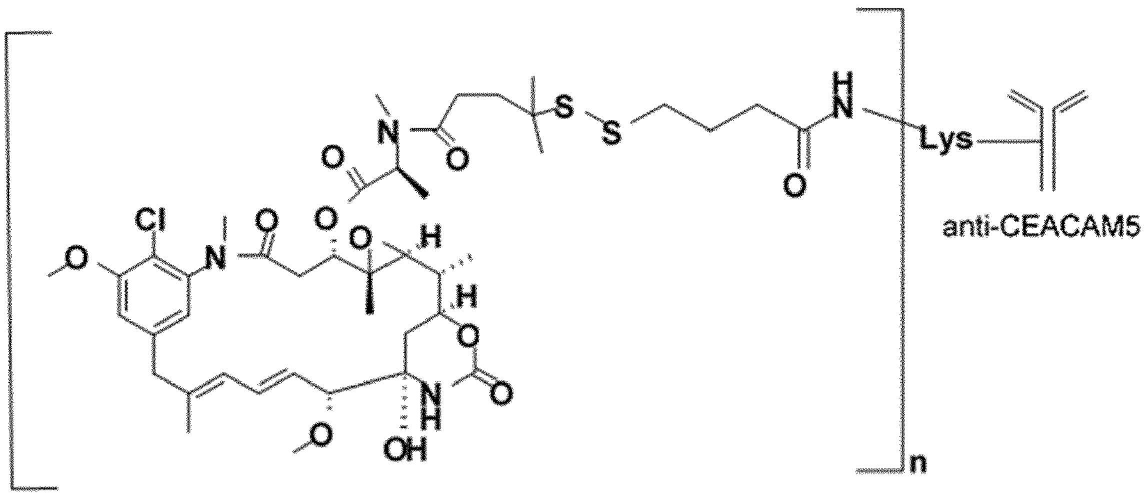

anti-CEACAM5-SPDB-DM4 ;

the anti-CEACAM5-sulfo-SPDB-DM4-antibody-drug conjugate of formula (IV):

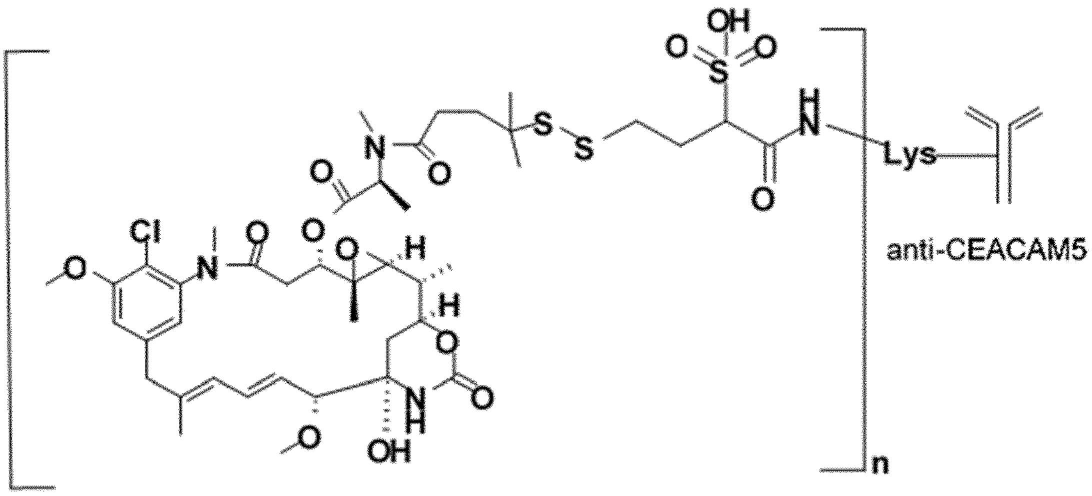

anti-CEACAM5-sulfo-SPDB-DM4 (IV);

and

an anti-CEACAM5-SMCC-DM1-antibody-drug conjugate of formula (V):

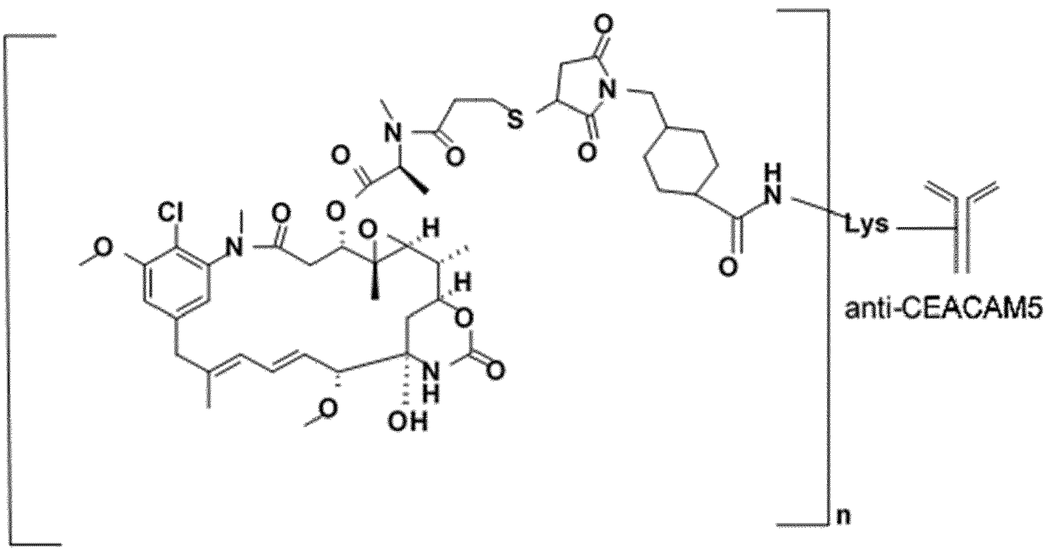

anti-CEACAM5-SMCC-DM1 (V).

In formulas (III), (IV) and (V) above, "n" corresponds to the number of molecules of chemotherapeutic agent conjugated per molecule of antibody. It corresponds to the "drug-to-antibody ratio" (or "DAR") defined below and may range from 1 to 10.

In a further embodiment, the antibody-drug conjugate of the present disclosure comprises an anti-CEACAM5-antibody, which comprises a heavy chain (VH) of SEQ ID NO: 8 and a light chain (VL) of SEQ ID NO: 9 (tusamitamab), wherein tusamitamab is covalently linked to N2'-deacetyl-N-2'(4-methyl-4-mercapto-1-oxopentyl)-maytansine (DM4) via N-succinimidyl pyridyldithiobutyrate (SPDB). Thereby, the antibody-drug conjugate tusamitamab ravtansine (huMAb2-3-SPDB-DM4) is obtained.

In an embodiment, the antibody-drug conjugate of the present disclosure is tusamitamab ravtansine (CAS [2254086-60-5]).

**"Linker",** as used herein, means a chemical moiety comprising a covalent bond or a chain of atoms that covalently attaches the antibody to the chemotherapeutic agent moiety (e.g., a cytostatic agent, a cytotoxic agent or a growth inhibitory agent). Suitable linkers are well known in the art and include disulfide groups, thioether groups, acid labile groups, photolabile groups, peptidase labile groups and esterase labile groups.

The conjugates may be prepared by *in vitro* methods. In order to link a drug or prodrug to the antibody, e.g., a chemotherapeutic agent, a linking group is used. Suitable linking groups are well known in the art and include disulfide groups, thioether groups, acid labile groups, photolabile groups, peptidase labile groups and esterase labile groups. Conjugation of an antibody with a chemotherapeutic agent of the disclosure, such as a cytotoxic agent, may be made using a variety of bifunctional protein coupling agents including but not limited to N-succinimidyl pyridyldithiobutyrate (SPDB), butanoic acid 4-[(5-nitro-2-pyridinyl)dithio]-2,5-dioxo-1-pyrrolidinyl ester (nitro-SPDB), 4-(pyridin-2-yldisulfanyl)-2-sulfo-butyric acid (sulfo-SPDB), N-succinimidyl (2-pyridyldithio) propionate (SPDP), succinimidyl (N-maleimidomethyl) cyclohexane-1-carboxylate (SMCC), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl)-hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4- dinitrobenzene). For example, a ricin immunotoxin can be prepared as described in Vitetta et al (1987). Carbon labeled 1-isothiocyanatobenzyl methyldiethylene triaminepentaacetic acid (MX-DTPA) is an exemplary chelating agent for conjugation of radionucleotide to the antibody (WO 94/11026).

The linker may be a "cleavable linker" facilitating release of the chemotherapeutic agent in the cell. For example, an acid-labile linker, a peptidase-sensitive linker, an esterase labile linker, a photolabile linker or a disulfide-containing linker (See e.g., U.S. Patent No. 5,208,020) may be used. The linker may be also a "non-cleavable linker" (for example SMCC linker) that might led to better tolerance in some cases.

In general, the conjugate can be obtained by a process comprising the steps of:

(i) bringing into contact an optionally-buffered aqueous solution of a cell-binding agent (e.g., an antibody according to the disclosure) with solutions of a linker and a chemotherapeutic agent, such as a cytotoxic compound (or agent);

(ii) then optionally separating the conjugate which was formed in (i) from the unreacted cell-binding agent (e.g., antibody of the disclosure) and unreacted chemotherapeutic agent, such as unreacted cytotoxic compound (or agent).

The aqueous solution of cell-binding agent can be buffered with buffers such as, e.g., potassium phosphate, acetate, citrate or N-2-Hydroxyethylpiperazine-N'-2-ethanesulfonic acid (Hepes buffer). The buffer depends upon the nature of the cell-binding agent (e.g., antibody of the disclosure). The chemotherapeutic agent, such as the cytotoxic compound (or agent), is in solution in an organic polar solvent, e.g., dimethyl sulfoxide (DMSO) or dimethylacetamide (DMA).

The reaction temperature is usually comprised between 20°C and 40°C. The reaction time can vary from 1 hour to 24 hours. The reaction between the cell-binding agent and the chemotherapeutic agent, such as the cytotoxic agent, can be monitored by size exclusion chromatography (SEC) with a refractometric and/or UV detector. If the conjugate yield is too low, the reaction time can be extended.

A number of different chromatography methods can be used by the person skilled in the art in order to perform the separation of step (ii): the conjugate can be purified, for example from aggregates, e.g., by SEC, adsorption chromatography (such as ion exchange chromatography, IEC), hydrophobic interaction chromatography (HIC), affinity chromatography, mixed-support chromatography such as hydroxyapatite chromatography, or high-performance liquid chromatography (HPLC). Purification by dialysis or diafiltration can also be used.

As used herein, the term "aggregates" means the associations which can be formed between two or more cell-binding agents, said agents being modified or not by conjugation. The aggregates can be formed under the influence of a great number of parameters, such as a high concentration of cell-binding agent (e.g., antibody of the disclosure) in the solution, the pH of the solution, high shearing forces, the number of bonded dimers and their hydrophobic character, the temperature (see Wang & Gosh, 2008, J. Membrane Sci., 318: 311-316, and references cited therein); note that the relative influence of some of these parameters is not clearly established. In the case of proteins and antibodies, the person skilled in the art will refer to Cromwell et al. (2006, AAPS Journal, 8(3): E572-E579). The content in aggregates can be determined with techniques well known to the skilled person, such as SEC (see Walter et al., 1993, Anal. Biochem., 212(2): 469-480).

After step (i) or (ii), the conjugate-containing solution can be submitted to an additional step (iii) of chromatography, ultrafiltration and/or diafiltration.

The conjugate is recovered at the end of these steps in an aqueous solution.

In a further embodiment, the antibody-drug conjugate according to the disclosure is characterized by a "drug-to-antibody ratio" (or "DAR") ranging from 1 to 10, or from 2 to 5, or from 3 to 4. This is generally the case of conjugates including maytansinoid molecules.

This DAR number can vary with the nature of the antibody and of the drug (i.e. the chemotherapeutic agent, such as a cytotoxic agent or a growth-inhibitory agent) used along with the experimental conditions used for the conjugation (like the ratio chemotherapeutic agent (e.g., growth-inhibitory agent)/antibody, the reaction time, the nature of the solvent and of the cosolvent if any).Thus the contact between the antibody and the chemotherapeutic agent, such as a cytotoxic agent or a growth-inhibitory agent, leads to a mixture comprising several conjugates differing from one another

by different drug-to-antibody ratios; optionally the naked antibody; optionally aggregates. The DAR that is determined is thus a mean value.

A method which can be used to determine the DAR consists in measuring spectrophotometrically the ratio of the absorbance at of a solution of substantially purified conjugate at λD and 280 nm. 280 nm is a wavelength generally used for measuring protein concentration, such as antibody concentration. The wavelength λD is selected so as to allow discriminating the drug from the antibody, i.e., as readily known to the skilled person, λD is a wavelength at which the drug (i.e., chemotherapeutic agent) has a high absorbance and λD is sufficiently remote from 280 nm to avoid substantial overlap in the absorbance peaks of the drug and antibody. λD may be selected as being 252 nm in the case of maytansinoid molecules. A method of DAR calculation may be derived from Antony S. Dimitrov (ed), LLC, 2009, Therapeutic Antibodies and Protocols, vol 525, 445, Springer Science:

The absorbances for the conjugate at λD (AλD) and at 280 nm (A280) are measured either on the monomeric peak of the size exclusion chromatography (SEC) analysis (allowing to calculate the "DAR(SEC)" parameter) or using a classic spectrophotometer apparatus (allowing to calculate the "DAR(UV)" parameter). The absorbances can be expressed as follows:

$$A\lambda D = (cD \times \varepsilon D\lambda D) + (cA \times \varepsilon A\lambda D)$$

$$A280 = (cD \times \varepsilon D280) + (cA \times \varepsilon A280)$$

wherein:

cD and cA are respectively the concentrations in the solution of the drug (i.e., chemotherapeutic agent) and of the antibody

εDλD and εD280 are respectively the molar extinction coefficients of the drug at λD and 280 nm

εAλD and εA280 are respectively the molar extinction coefficients of the antibody at λD and 280 nm.

Resolution of these two equations with two unknowns leads to the following equations:

$$cD = [(\varepsilon A280 \times A\lambda D) - (\varepsilon A\lambda D \times A280)] / [(\varepsilon D\lambda D \times \varepsilon A280) - (\varepsilon A\lambda D \times \varepsilon D280)]$$

$$cA = [A280 - (cD \times \varepsilon D280)] / \varepsilon A280$$

The average DAR is then calculated from the ratio of the drug concentration to that of the antibody: DAR = cD / cA.

**Anti-VEGFR-2 antibody**

The combination of the disclosure comprises an anti-VEGFR-2 antibody, or a fragment thereof, for the treatment of cancer.

In one embodiment, the anti-VEGFR-2 antibody is a monoclonal antibody, or a fragment thereof having antagonist activity to VEGFR-2. In one embodiment, the anti-VEGFR-2 antibody is an IgG.

The anti-VEGFR-2 antibody is preferably adapted to the patient. For example, an anti-mouse VEGFR-2 antibody such as DC-101 is preferably used on mice, and an antihuman VEGFR-2 antibody on humans.

In one embodiment, the anti-VEGFR-2 antibody is ramucirumab (CAS number 947687-13-0). It is a fully human monoclonal IgG1 antibody against human VEGFR-2.

In one embodiment, the anti-VEGFR-2 antibody or fragment thereof comprises the light chain and heavy chain CDRs of ramucirumab.

In one embodiment, the anti-VEGFR-2 antibody or fragment thereof comprises the variable domain of heavy chain (VH) and the variable domain of light chain (VL) of ramucirumab.

In a further embodiment, the anti-VEGFR-2 antibody or fragment thereof comprises a heavy chain (HC) having at least 92%, at least 95%, at least 98% identity to SEQ ID NO: 10 and a light chain (LC) having at least 92%, at least

95%, at least 98% identity to SEQ ID NO: 11.

The anti-VEGFR-2 antibody or fragment thereof may also be a single domain antibody or a fragment thereof. In particular, a single domain antibody fragment may consist of a variable heavy chain (VHH) which comprises the CDR1-H, CDR2-H and CDR3-H of the antibodies as described above. The antibody may also be a heavy chain antibody, i.e., an antibody devoid of light chain, which may or may not contain a CH1 domain.

The single domain antibody or a fragment thereof may also comprise the framework regions of a camelid single domain antibody, and optionally the constant domain of a camelid single domain antibody.

The anti-VEGFR-2 antibody may also be an antibody fragment, in particular a humanized antibody fragment, selected from the group consisting of Fv, Fab, F(ab')2, Fab', dsFv, (dsFv)2, scFv, sc(Fv)2, and diabodies.

The antibody may also be a bispecific or multispecific antibody formed from antibody fragments, at least one antibody fragment being an antibody fragment according to the disclosure. The anti-VEGFR-2 antibody and fragments thereof can be produced by any technique well known in the art. In particular, said antibodies are produced by techniques as already described.

The anti-VEGFR-2 antibody and fragments thereof can be used in an isolated (e.g., purified) from or contained in a vector, such as a membrane or lipid vesicle (e.g., a liposome).

The anti-VEGFR-2 antibody and fragments thereof may be produced by any technique known in the art, such as, without limitation, any chemical, biological, genetic, or enzymatic technique, either alone or in combination.

**Anti-PD-1 antibodies; anti-PD-L1 antibodies**

The combination of the disclosure comprises an anti-PD-1 antibody or an anti-PD-L1 antibody, or a fragment thereof, for the treatment of cancer.

In one embodiment, the anti-PD-1 antibody or the anti-PD-L1 antibody, or a fragment thereof, is a monoclonal antibody having interfering activity with interaction between PD-1 and PD-L1. In one embodiment, the anti-PD-1 antibody or the anti-PD-L1 antibody is an IgG.

Anti-PD-1 antibodies and anti-PD-L1 antibodies capable of interfering with interaction between PD-1, which is expressed on the surface of immune cells, and PD-L1, which is expressed on the surface of cancer cells, are useful as immune checkpoint inhibitors, thereby blocking a pathway that shields tumor cells from immune system components able and poised to fight cancer. When PD-1 and PD-L1 interact, they form a biochemical "shield" protecting tumor cells from being destroyed by the immune system. Thus, blockade of either PD-1 or PD-L1 leading to blockade of interaction between PD-1 and PD-L1 prevents or unmasks the biochemical "shield" protecting tumor cells from being destroyed by the immune system.

A number of anti-PD-1 antibodies have been approved for clinical use in the treatment of cancer. These include pembrolizumab (KEYTRUDA®), nivolumab (OPDIVO®), cemiplimab (LIBTAYO®), sintilimab (TYVYT®), dostarlimab (JEMPERLI®), and tislelizumab.

Similarly, a number of anti-PD-L1 antibodies have been approved for clinical use in the treatment of cancer. These include atezolizumab (TECENTRIQ®), avelumab (BAVENCIO®), and durvalumab (IMFINZI®).

In one embodiment, the anti-PD-1 antibody is pembrolizumab or sintilimab.

In one embodiment, the anti-PD-1 antibody is pembrolizumab. It is a fully human monoclonal IgG1 antibody against human PD-1.

In one embodiment, the anti-PD-1 antibody, or a fragment thereof, comprises the light chain and heavy chain CDRs of pembrolizumab.

In one embodiment, the anti-PD-1 antibody or a fragment thereof comprises the variable domain of heavy chain (VH) and the variable domain of light chain (VL) of pembrolizumab.

In one embodiment, the anti-PD-1 antibody is sintilimab. In one embodiment, the anti-PD-1 antibody, or a fragment thereof, comprises the light chain and heavy chain CDRs of sintilimab. In one embodiment, the anti-PD-1 antibody or a fragment thereof comprises the variable domain of heavy chain (VH) and the variable domain of light chain (VL) of sintilimab.

The anti-PD-1 antibody or the anti-PD-L1 antibody, or a fragment thereof, may also be a single domain antibody or a fragment thereof. In particular, a single domain antibody fragment may consist of a variable heavy chain (VHH) which comprises the CDR1-H, CDR2-H and CDR3-H of the antibodies as described above. The antibody may also be a heavy chain antibody, i.e., an antibody devoid of light chain, which may or may not contain a CH1 domain.

The single domain antibody or a fragment thereof may also comprise the framework regions of a camelid single domain antibody, and optionally the constant domain of a camelid single domain antibody.

The anti-PD-1 antibody or the anti-PD-L1 antibody may also be an antibody fragment, in particular a humanized antibody fragment, selected from the group consisting of Fv, Fab, F(ab')2, Fab', dsFv, (dsFv)2, scFv, sc(Fv)2, and diabodies.

The antibody may also be a bispecific or multispecific antibody formed from antibody fragments, at least one

antibody fragment being an antibody fragment according to the disclosure. The anti-PD-1 antibody or the anti-PD-L1 antibody and fragments thereof can be produced by any technique well known in the art. In particular, said antibodies are produced by techniques as already described.

The anti-PD-1 antibody or the anti-PD-L1 antibody and fragments thereof can be used in an isolated (e.g., purified) from or contained in a vector, such as a membrane or lipid vesicle (e.g., a liposome).

The anti-PD-1 antibody or the anti-PD-L1 antibody and fragments thereof may be produced by any technique known in the art, such as, without limitation, any chemical, biological, genetic, or enzymatic technique, either alone or in combination.

## Cancer

In an embodiment, the cancer is a carcinoma, a sarcoma or a blastoma. In a further embodiment, the cancer is a carcinoma.

According to an embodiment, the cancer is a cancer expressing CEACAM5. A cancer expressing CEACAM5 may also be named CEACAM5 positive cancer.

In some embodiments, the cancer is a CEACAM5-positive cancer.

A CEACAM5-positive cancer is defined as cancer for which a CEACAM5 immunohistochemical [IHC] intensity is ≥ 2+ in ≥ 50% of cancer cells or ≥ 2+ intensity in ≥ 1% and < 50% of the cells tumors (or cancer cells).

In certain embodiments, the cancer has a negative or low CEACAM5 expression on tumor cells. A negative or low CEACAM5 expression on tumor cells defined as being a CEACAM5 immunohistochemical [IHC] intensity ≥ 2+ in < 1% of cells, as measured by immunohistochemistry (IHC).

In certain embodiments, the cancer has a moderate CEACAM5 expression on tumor cells. A moderate CEACAM5 expression on tumor cells may be defined as being a CEACAM5 immunohistochemical [IHC] intensity ≥ 2+ in ≥ 1% and in < 50% of cancer cells, as measured by immunohistochemistry.

In certain embodiments, the cancer has a high CEACAM5 expression on tumor cells. A high CEACAM5 expression on tumor cells may be defined as being a CEACAM5 immunohistochemical [IHC] intensity ≥ 2+ intensity in ≥ 50% of cancer cells, as measured by immunohistochemistry.

Immunohistochemical techniques for detecting antigens on cells or in tissue sections by means of immunological and chemical reactions are well-known in the field. Those techniques are highly sensitive and specific and can detect a wide variety of antigens. Immunohistochemistry methods comprise the following steps: binding of an antibody to a specific antigen; formation of an antibody-antigen complex by incubation with a secondary, enzyme-conjugated antibody, and generation of colored deposits at the sites of antibody-antigen binding in presence of substrate and chromogen catalyzed by the enzyme.

The CEACAM5 tumor expression may be determined by using Immunohistochemistry (IHC) assay. An assay may be done using anti-CEACAM5 antibody such as SANOFI's antibody clone 769. Anti-CEACAM5 clone 769 is a murine monoclonal antibody with the same specificity as tusamitamab ravtansine to the CEACAM5 target. The assay may be run on Techmate platformer or on a Dako/Agilent Autostainer Link 48 IHC. Interpretation of CEACAM5 reactivity is to be performed using semi-quantitative Percent Scores (calculated by summing the percentages of intensities ≥2+) or H-score for CEACAM5 plasma membrane staining (whole or polarized) in tumor cells.

According to an embodiment, the cancer is selected from hepatocellular carcinoma, colorectal cancer, gastric cancer, gastroesophageal junction adenocarcinoma (GEJ), esophageal cancer, lung cancer (e.g., non-squamous non-small cell lung cancer), uterus cervix cancer, pancreatic cancer, ovarian cancer, thyroid cancer, bladder cancer, endometrial cancer, breast cancer, liver cancer, biliary tract cancer (for instance cholangiocarcinoma), prostate cancer, neuroendocrine cancer and skin cancer.

In some embodiments, the cancer may be selected from gastric cancer, gastroesophageal junction (GEJ) adenocarcinoma, esophageal cancer, and lung cancer.

According to an embodiment, the cancer is gastric cancer or gastroesophageal junction adenocarcinoma (GEJ).

According to an embodiment, the cancer is a gastric cancer.

In some embodiments, the cancer may be a pancreatic cancer.

According to an embodiment, the cancer is a lung cancer. A lung cancer may be a non-squamous non-small-cell lung cancer (NSQ NSCLC).

Non-small cell lung cancer is a disease in which malignant (cancer) cells form in the tissues of the lung. Smoking is the major cause of the disease. This is a type of epithelial lung cancer other than small cell lung carcinoma. There are several types of non-small cell lung cancer. Each type of non-small cell lung cancer has different kinds of cancer cells. The cancer cells of each type grow and spread in different ways. The types of non-small cell lung cancer are named for the kinds of cells found in the cancer and how the cells look under a microscope: (1) squamous cell carcinoma: Cancer that begins in squamous cells, which are thin, flat cells that look like fish scales. This is also called epidermoid carcinoma. (2) large cell carcinoma: Cancer that may begin in several types of large cells. (3) adenocarcinoma: Cancer

that begins in the cells that line the alveoli and make substances such as mucus.

In some embodiments, the non-squamous non-small cell lung cancer may be an advanced or metastatic NSQ NSCLC.

According to an embodiment, the subject is a patient with malignant tumor, in particular with a malignant solid tumor, and more specifically with locally advanced or metastatic solid malignant tumor. A metastatic solid malignant tumor may be a metastatic cancer, for example a metastatic carcinoma. A cancer or a carcinoma may be as above indicated.

In some embodiments, the non-squamous non-small cell lung cancer has no epidermal growth factor receptor (EGFR) sensitizing mutation or v-raf murine sarcoma viral oncogene homolog B1 (BRAF) mutation or anaplastic lymphoma kinase/c-ros oncogene 1 (ALK/ROS) alterations.

**Combined treatment**

***Sequences of administration***

The disclosure relates to an antibody-drug conjugate comprising an anti-CEACAM5-antibody for use for treating cancer in a subject in need thereof who receives, simultaneously, separately, or sequentially an anti-VEGFR- 2 antibody and an anti-PD-1 antibody or anti-PD-L1 antibody.

According to the present disclosure, the ADC is for use for treating cancer in combination with an anti-VEGFR-2 antibody and an anti-PD-1 antibody or anti-PD-L1 antibody. The disclosure also relates to an anti-VEGFR-2 antibody for use for treating cancer in combination with the ADC and an anti-PD-1 antibody or anti-PD-L1 antibody. The disclosure also relates to an anti-PD-1 antibody or anti-PD-L1 antibody for use for treating cancer in combination with the ADC and an anti-VEGFR-2 antibody.

In some embodiments, (i) the antibody-drug conjugate, (ii) the anti-VEGFR2 antibody, and (iii) the anti-PD-1 antibody or anti-PD-L1 antibody may be administered simultaneously (or concurrently), separately or sequentially to a subject in need thereof.

As used herein, the expression "**in combination with**" means that the anti-VEGFR-2 antibody or the anti-PD-1 antibody or anti-PD-L1 antibody are administered before, after, or concurrent with the ADC. In some embodiments, the term "in combination with" includes sequential or concomitant administration of the ADC and the anti-VEGFR-2 antibody or the anti-PD-1 antibody or anti-PD-L1 antibody. In some embodiments, **"combination"** used with respect to the anti-VEGFR-2 antibody, the antibody-drug conjugate comprising an anti-CEACAM5-antibody, and the anti-PD-1 antibody or the anti-PD-L1 antibody intends to refer to antibodies formulated individually and administered sequentially to a subject in need thereof, for a cancer indication.

Methods to treat cancer (e.g., GC, GEJ cancer, NSQ NSCLC) includes administering an antibody-drug conjugate comprising an anti-CEACAM5-antibody (e.g., tusamitamab ravtansine) in combination with the anti-VEGFR-2 antibody and the anti-PD-1 antibody or anti-PD-L1 antibody for additive or synergistic activity.

In some embodiments, (i) the antibody-drug conjugate, (ii) the anti-VEGFR2 antibody, and (iii) the anti-PD-1 antibody or anti-PD-L1 antibody are administered sequentially to a subject in need thereof.

According to some embodiments, the ADC and the anti-VEGFR-2 antibody are administered simultaneously, separately, or sequentially to a subject in need thereof.

According to an embodiment, the ADC and the anti-VEGFR-2 antibody are simultaneously administered to a subject in need thereof. For example, ADC and the anti-VEGFR2 antibody are administered on day one of a cycle of treatment, approximatively at the same time. A simultaneous administration of the ADC and the anti-VEGFR-2 antibody may be by the same route.

According to an embodiment, the ADC and the anti-VEGFR-2 antibody are separately administered to a subject in need thereof. For example, the ADC and the anti-VEGFR2 antibody are administered on day one of a cycle, by separate routes or at separates location of the body of said subject. A separate administration of the ADC and the anti-VEGFR-2 antibody may be at the same time or at close times, e.g., 5 min or less.

According to an embodiment, the ADC and the anti-VEGFR-2 antibody are sequentially administered to a subject in need thereof. For example, the ADC and the anti-VEGFR-2 antibody are administered on day one of a cycle, at different times, for example the ADC is administered one to three hours after the anti-VEGFR-2 antibody. A sequential administration of the ADC and the anti-VEGFR-2 antibody may be by separate routes or by a same route. A sequential administration of the ADC and the anti-VEGFR-2 antibody may comprise administration of the ADC after the anti-VEGFR-2 antibody. The ADC may be administered about 0.5 hr, 1hr, 2hrs, 3hrs, 4hrs, 5hrs or about 6hrs after the anti-VEGFR-2 antibody. The ADC may be administered about 1hr after the anti-VEGFR-2 antibody.

In a sequential administration, the period of time between the administration of the anti-VEGFR-2 antibody and the antibody-drug conjugate comprising an anti-CEACAM5-antibody may last from about a few minutes to about several hours, days, or weeks. In some embodiments, the period of time may range from about 5 minutes to about 3 hours, for

example from 10 minutes to about 2.5 hours, from about 30 minutes to about 2 hours, or from about 1 hour to about 1.5 hours. A period of time between may last about 5 minutes, about 10 minutes, about 30 minutes, about 1 hour, 1.5 hours, about 2 hours, about 2.5 hours or about 3 hours.

In some embodiments, the anti-VEGFR-2 is administered over one hour.

In some embodiments, the antibody-drug conjugate comprising an anti-CEACAM5-antibody is administered over 1.5 hours.

In some embodiments, in a sequential administration, the period of time between the administration of the anti-VEGFR-2 antibody and the antibody-drug conjugate comprising an anti-CEACAM5-antibody may range from about 5 minutes to about 3 hours, for example from 10 minutes to about 2.5 hours, from about 30 minutes to about 2 hours, or from about 1 hour to about 1.5 hours. In a sequential administration on a same day of a cycle, a period of time between may last about 5 minutes, about 10 minutes, about 30 minutes, about 1 hour, 1.5 hours, about 2 hours, about 2.5 hours or about 3 hours.

The period of time between an administration of the anti-VEGFR-2 antibody and an administration of the antibody-drug conjugate comprising an anti-CEACAM5-antibody may range from about 20 minutes to about 5 hours, from about 30 minutes to about 3 hours, from about 40 minutes to about 2 hours, from about 50 minutes to about 1.5 hours, or may last about 1 hour.

In an embodiment, in a sequential administration, the period of time between the administration of the anti-VEGFR-2 antibody and the antibody-drug conjugate comprising an anti-CEACAM5-antibody may be at least one hour.

In a sequential administration, the antibody-drug conjugate (ADC) comprising an anti-CEACAM5-antibody may be administered after or before the anti-VEGFR-2 antibody.

In some embodiments, the antibody-drug conjugate comprising an anti-CEACAM5-antibody may be administered before the anti-VEGFR-2 antibody.

In some embodiments, the antibody-drug conjugate comprising an anti-CEACAM5-antibody may be administered after the anti-VEGFR-2 antibody.

In some embodiments, the sequence of administration of the antibody-drug conjugate comprising an anti-CEACAM5-antibody and of the anti-VEGFR-2 antibody may be the same for all cycles of treatment.

When administered "before" the ADC, the anti-VEGFR-2 antibody may be administered about 72 hours, about 60 hours, about 48 hours, about 36 hours, about 24 hours, about 12 hours, about 10 hours, about 8 hours, about 6 hours, about 4 hours, about 2 hours, about 1 hour, about 30 minutes, about 15 minutes, or about 10 minutes prior to the administration of the ADC.

When administered "after" the ADC, the anti-VEGFR-2 antibody may be administered about 10 minutes, about 15 minutes, about 30 minutes, about 1 hour, about 2 hours, about 4 hours, about 6 hours, about 8 hours, about 10 hours, about 12 hours, about 24 hours, about 36 hours, about 48 hours, about 60 hours, or about 72 hours after the administration of the antibody-drug conjugate comprising an anti-CEACAM5-antibody.

In some embodiments, the anti-VEGFR-2 antibody may be administered before the antibody-drug conjugate.

In some embodiments, the method of treating cancer is administering to a subject in need thereof an effective amount of an antibody-drug conjugate comprising an anti-CEACAM5-antibody before administering the anti-VEGFR-2 antibody.

In some embodiments, the method of treating cancer is administering to a subject in need thereof an effective amount of an antibody-drug conjugate comprising an anti-CEACAM5-antibody comprising an anti-CEACAM5 antibody after administering the anti-VEGFR-2 antibody.

According to some embodiments, the ADC and the anti-PD-1 antibody or the anti-PD-L1 antibody are administered simultaneously, separately, or sequentially to a subject in need thereof.

According to an embodiment, the ADC and the anti-PD-1 antibody or the anti-PD-L1 antibody are simultaneously administered to a subject in need thereof. For example, ADC and the anti-PD-1 antibody or the anti-PD-L1 antibody are administered on day one of a cycle of treatment, approximatively at the same time. A simultaneous administration of the ADC and the anti-PD-1 antibody or the anti-PD-L1 antibody may be by the same route.

According to an embodiment, the ADC and the anti-PD-1 antibody or the anti-PD-L1 antibody are separately administered to a subject in need thereof. For example, the ADC and the anti-PD-1 antibody or the anti-PD-L1 antibody are administered on day one of a cycle, by separate routes or at separates location of the body of said subject. A separate administration of the ADC and the anti-PD-1 antibody or the anti-PD-L1 antibody may be at the same time or at close times, e.g., 5 min or less.

According to an embodiment, the ADC and the anti-PD-1 antibody or the anti-PD-L1 antibody are sequentially administered to a subject in need thereof. For example, the ADC and the anti-PD-1 antibody or the anti-PD-L1 antibody are administered on day one of a cycle, at different times, for example the ADC is administered one to three hours after the anti-PD-1 antibody or the anti-PD-L1 antibody. A sequential administration of the ADC and the anti-PD-1 antibody or the anti-PD-L1 antibody may be by separate routes or by a same route. A sequential administration of the ADC and the anti-PD-1 antibody or the anti-PD-L1 antibody may comprise administration of the ADC after the anti-PD-1 antibody

or the anti-PD-L1 antibody. The ADC may be administered about 0.5 hr, 1hr, 2hrs, 3hrs, 4hrs, 5hrs or about 6hrs after the anti-PD-1 antibody or the anti-PD-L1 antibody. The ADC may be administered about 1hr after the anti-PD-1 antibody or the anti-PD-L1 antibody.

In a sequential administration, the period of time between the administration of the anti-PD-1 antibody or the anti-PD-L1 antibody and the antibody-drug conjugate comprising an anti-CEACAM5-antibody may last from about a few minutes to about several hours, days, or weeks. In some embodiments, the period of time may range from about 5 minutes to about 3 hours, for example from 10 minutes to about 2.5 hours, from about 30 minutes to about 2 hours, or from about 1 hour to about 1.5 hours. A period of time between may last about 5 minutes, about 10 minutes, about 30 minutes, about 1 hour, 1.5 hours, about 2 hours, about 2.5 hours or about 3 hours.

In some embodiments, the anti-PD-1 antibody or the anti-PD-L1 antibody is administered over one hour.

In some embodiments, the antibody-drug conjugate comprising an anti-CEACAM5-antibody is administered over 1.5 hours.

In some embodiments, in a sequential administration, the period of time between the administration of the anti-PD-1 antibody or the anti-PD-L1 antibody and the antibody-drug conjugate comprising an anti-CEACAM5-antibody may range from about 5 minutes to about 3 hours, for example from 10 minutes to about 2.5 hours, from about 30 minutes to about 2 hours, or from about 1 hour to about 1.5 hours. In a sequential administration on a same day of a cycle, a period of time between may last about 5 minutes, about 10 minutes, about 30 minutes, about 1 hour, 1.5 hours, about 2 hours, about 2.5 hours or about 3 hours.

In an embodiment, in a sequential administration, the period of time between the administration of the anti-PD-1 antibody or the anti-PD-L1 antibody and the antibody-drug conjugate comprising an anti-CEACAM5-antibody may be at least one hour.

In a sequential administration, the antibody-drug conjugate (ADC) comprising an anti-CEACAM5-antibody may be administered after or before the anti-PD-1 antibody or the anti-PD-L1 antibody.

In some embodiments, the antibody-drug conjugate comprising an anti-CEACAM5-antibody may be administered before the anti-PD-1 antibody or the anti-PD-L1 antibody.

In some embodiments, the antibody-drug conjugate comprising an anti-CEACAM5-antibody may be administered after the anti-PD-1 antibody or the anti-PD-L1 antibody.

In some embodiments, the sequence of administration of the antibody-drug conjugate comprising an anti-CEACAM5-antibody and of the anti-PD-1 antibody or the anti-PD-L1 antibody may be the same for all cycles of treatment.

When administered "before" the ADC, the anti-PD-1 antibody or the anti-PD-L1 antibody may be administered about 72 hours, about 60 hours, about 48 hours, about 36 hours, about 24 hours, about 12 hours, about 10 hours, about 8 hours, about 6 hours, about 4 hours, about 2 hours, about 1 hour, about 30 minutes, about 15 minutes, or about 10 minutes prior to the administration of the ADC.

When administered "after" the ADC, the anti-PD-1 antibody or the anti-PD-L1 antibody may be administered about 10 minutes, about 15 minutes, about 30 minutes, about 1 hour, about 2 hours, about 4 hours, about 6 hours, about 8 hours, about 10 hours, about 12 hours, about 24 hours, about 36 hours, about 48 hours, about 60 hours, or about 72 hours after the administration of the antibody-drug conjugate comprising an anti-CEACAM5-antibody.

In some embodiments, the anti-PD-1 antibody or the anti-PD-L1 antibody may be administered before the antibody-drug conjugate.

In some embodiments, the method of treating cancer is administering to a subject in need thereof an effective amount of an antibody-drug conjugate comprising an anti-CEACAM5-antibody before administering the anti-PD-1 antibody or the anti-PD-L1 antibody.

In some embodiments, the method of treating cancer is administering to a subject in need thereof an effective amount of an antibody-drug conjugate comprising an anti-CEACAM5-antibody comprising an anti-CEACAM5 antibody after administering the anti-PD-1 antibody or the anti-PD-L1 antibody.

In some embodiments, the anti-PD-1 antibody or the anti-PD-L1 antibody and the ADC may be administered sequentially.

In some embodiments, the ADC may be administered before the anti-PD-1 antibody or anti-PD-L1 antibody.

The present disclosure also relates to a method of treatment of cancer in a subject in need thereof, comprising administering an anti-VEGFR-2 antibody, administering an antibody-drug conjugate comprising an anti-CEACAM5-antibody, and administering the anti-PD-1 antibody or an anti-PD-L1 antibody to the subject in need thereof.

The present disclosure also relates to a method of treatment of cancer in a subject in need thereof, comprising administering, sequentially, in the following order: (a) an anti-VEGFR-2 antibody, (b) an antibody-drug conjugate comprising an anti-CEACAM5-antibody, and (c) an anti-PD-1 antibody or the anti-PD-L1 antibody to the subject in need thereof.

The present disclosure also relates to a combination comprising an anti-VEGFR-2 antibody, an antibody-drug conjugate comprising an anti-CEACAM5-antibody, and an anti-PD-1 antibody or an anti-PD-L1 antibody for use for treating cancer.

The combination is for a sequential administration of the anti-VEGFR-2 antibody, the antibody-drug conjugate comprising an anti-CEACAM5-antibody, and the anti-PD-1 antibody or the anti-PD-L1 antibody.

A method of treatment or a use, as disclosed herein, may achieve a synergistic effect in reducing tumor size.

A method of treatment or a use, as disclosed herein, may achieve a synergistic effect in inhibiting tumor growth.

The present disclosure also relates to a combination for the manufacture of a medicament for the treatment of cancer, the combination comprising an antibody-drug conjugate comprising an anti-CEACAM5-antibody, an anti-VEGFR-2 antibody, and an anti-PD-1 antibody or an anti-PD-L1 antibody. The medicament may comprise the antibodies formulated individually, in separate containers.

In an embodiment, the present disclosure relates to a use of a combination of (i) an antibody-drug conjugate as disclosed herein, (ii) an anti-VEGFR2 antibody, and (iii) an anti-PD-1 antibody or anti-PD-L1 antibody, for the manufacture of a pharmaceutical composition or a kit, for the treatment of a cancer. The pharmaceutical composition or the kit may comprise the antibodies formulated individually, in separate containers

In an embodiment, the combination permits a simultaneous, separate or sequential administration of the antibody-drug conjugate comprising an anti-CEACAM5-antibody, the anti-VEGFR-2 antibody, and the anti-PD-1 antibody or the anti-PD-L1 antibody.

In an embodiment, the combination permits a simultaneous administration of the antibody-drug conjugate comprising an anti-CEACAM5-antibody, the anti-VEGFR-2 antibody, and the anti-PD-1 antibody or the anti-PD-L1 antibody.

In an embodiment, the combination permits a separate administration of the antibody-drug conjugate comprising an anti-CEACAM5-antibody, the anti-VEGFR-2 antibody, and the anti-PD-1 antibody or the anti-PD-L1 antibody.

In an embodiment, the combination permits a sequential administration of the antibody-drug conjugate comprising an anti-CEACAM5-antibody, the anti-VEGFR-2 antibody, and the anti-PD-1 antibody or the anti-PD-L1 antibody.

In a further embodiment, combinations according to the disclosure are pharmaceutical compositions.

In a further embodiment, combinations according to the disclosure are pharmaceutical kits-of-parts.

According to some embodiments, the ADC, the anti-VEGFR-2 antibody, and the anti-PD-1 antibody or the anti-PD-L1 antibody are administered simultaneously, separately, or sequentially to a subject in need thereof.

According to an embodiment, the ADC, the anti-VEGFR-2 antibody and the anti-PD-1 antibody or the anti-PD-L1 antibody are simultaneously administered to a subject in need thereof. For example, the ADC, the anti-VEGFR-2 antibody, and the anti-PD-1 antibody or the anti-PD-L1 antibody are administered on day one of a cycle of treatment, approximatively at the same time. A simultaneous administration of the ADC, the anti-VEGFR-2 antibody, and the anti-PD-1 antibody or the anti-PD-L1 antibody may be by the same route.

According to an embodiment, the ADC, the anti-VEGFR-2 antibody, and the anti-PD-1 antibody or the anti-PD-L1 antibody are separately administered to a subject in need thereof. For example, the ADC, the anti-VEGFR-2 antibody and the anti-PD-1 antibody or the anti-PD-L1 antibody are administered on day one of a cycle, by separate routes or at separates location of the body of said subject. A separate administration of the ADC, the anti-VEGFR-2 antibody, and the anti-PD-1 antibody or the anti-PD-L1 antibody may be at the same time or at close times, e.g., 5 min or less.

According to an embodiment, the ADC, the anti-VEGFR-2 antibody, and the anti-PD-1 antibody or the anti-PD-L1 antibody are sequentially administered to a subject in need thereof. For example, the ADC, the anti-VEGFR-2 antibody, and the anti-PD-1 antibody or the anti-PD-L1 antibody are administered on day one of a cycle, at different times, for example the ADC is administered one to three hours after the anti-VEGFR-2 antibody, and the anti-PD-1 antibody or the anti-PD-L1 antibody is administered one to three hours after the ADC. A sequential administration of the ADC, the anti-VEGFR-2 antibody, and the anti-PD-1 antibody or the anti-PD-L1 antibody may be by separate routes or by a same route. A sequential administration of the ADC, the anti-VEGFR-2 antibody, and the anti-PD-1 antibody or the anti-PD-L1 antibody may comprise administration of the ADC after the anti-VEGFR-2 antibody and administration of the anti-PD-1 antibody or the anti-PD-L1 antibody after the ADC. The ADC may be administered about 0.5 hr, 1hr, 2hrs, 3hrs, 4hrs, 5hrs or about 6hrs after the anti-VEGFR-2 antibody and the anti-PD-1 antibody or the anti-PD-L1 antibody may be administered about 0.5 hr, 1hr, 2hrs, 3hrs, 4hrs, 5hrs or about 6hrs after the ADC. The anti-CEACAM5-antibody may be administered about 1hr after the anti-PD-1 antibody or the anti-PD-L1 antibody.

In a sequential administration, the period of time between the administration of the anti-PD-1 antibody or the anti-PD-L1 antibody and the antibody-drug conjugate comprising an anti-CEACAM5-antibody and between the administration of the anti-VEGFR-2 antibody and the ADC may last from about a few minutes to about several hours, days, or weeks. In some embodiments, the period of time may range from about 5 minutes to about 3 hours, for example from 10 minutes to about 2.5 hours, from about 30 minutes to about 2 hours, or from about 1 hour to about 1.5 hours. A period of time between may last about 5 minutes, about 10 minutes, about 30 minutes, about 1 hour, 1.5 hours, about 2 hours, about 2.5 hours or about 3 hours.

In some embodiments, the anti-VEGFR-2 antibody may be administered for a period of time ranging from about 20 minutes to about 2.5 hours, or from about 30 minutes to about 2 hours, or from about 45 minutes to about 1.5 hours, or for about 1 hour. In some embodiments, the period of time may be of about 1 hour.

In some embodiments, the anti-VEGFR-2 antibody is administered over one hour.

In some embodiments, the antibody-drug conjugate comprising an anti-CEACAM5-antibody may be administered for a period of time ranging from about 30 minutes to about 3 hours, or from about 45 minutes to about 2.5 hours, or from about 1 hour to about 2 hours, or for about 1.5 hours. In some embodiments, the period of time may be of about 1.5 hours.

In some embodiments, the antibody-drug conjugate comprising an anti-CEACAM5-antibody is administered over 1.5 hours.

In some embodiments, the anti-PD-1 antibody or the anti-PD-L1 antibody may be administered for a period of time ranging from about 20 minutes to about 2.5 hours, or from about 30 minutes to about 2 hours, or from about 45 minutes to about 1.5 hours, or for about 1 hour. In some embodiments, the period of time may be of about 1 hour.

In some embodiments, the anti-PD-1 antibody or the anti-PD-L1 antibody is administered over one hour.

In some embodiments, (i) the antibody-drug conjugate, (ii) the anti-VEGFR2 antibody, and (iii) the anti-PD-1 antibody or anti-PD-L1 antibody are administered sequentially to a subject in need thereof.

In some embodiments, in a sequential administration, the period of time between the administration of the anti-PD-1 antibody or the anti-PD-L1 antibody and the antibody-drug conjugate comprising an anti-CEACAM5-antibody and between the administration of the anti-VEGFR-2 antibody and the ADC may range from about 5 minutes to about 3 hours, for example from 10 minutes to about 2.5 hours, from about 30 minutes to about 2 hours, or from about 1 hour to about 1.5 hours. In a sequential administration on a same day of a cycle, a period of time between may last about 5 minutes, about 10 minutes, about 30 minutes, about 1 hour, 1.5 hours, about 2 hours, about 2.5 hours or about 3 hours.

In an embodiment, in a sequential administration o, the period of time between the administration of the anti-PD-1 antibody or the anti-PD-L1 antibody and the antibody-drug conjugate comprising an anti-CEACAM5-antibody and between the administration of the anti-VEGFR-2 antibody and the ADC may be at least one hour.

In a sequential administration, the antibody-drug conjugate (ADC) comprising an anti-CEACAM5-antibody may be administered after or before the anti-VEGFR-2 antibody.

In some embodiments, the antibody-drug conjugate comprising an anti-CEACAM5-antibody may be administered after the anti-VEGFR-2 antibody.

In a sequential administration, the antibody-drug conjugate (ADC) comprising an anti-CEACAM5-antibody may be administered after or before the anti-PD-1 antibody or the anti-PD-L1 antibody.

In some embodiments, the antibody-drug conjugate comprising an anti-CEACAM5-antibody may be administered before the anti-PD-1 antibody or the anti-PD-L1 antibody.

In some embodiments, the sequence of administration of the antibody-drug conjugate comprising an anti-CEACAM5-antibody and of the anti-PD-1 antibody or the anti-PD-L1 antibody may be the same for all cycles of treatment.

In some embodiments, the anti-VEGFR-2 antibody may be administered before the antibody-drug conjugate, and the antibody-drug conjugate may be administered before the anti-PD-1 antibody or the anti-PD-L1 antibody.

In some embodiments, the sequence of administration of the antibody-drug conjugate comprising an anti-CEACAM5-antibody, of the anti-VEGFR-2 antibody and of the anti-PD-1 antibody or the anti-PD-L1 antibody may vary along the cycles of treatment. In some embodiments, one or more cycles of a treatment may comprise a first sequence of administration and one or more cycles of said treatment may comprise a second sequence of administration, the first and second sequences being different.

In some embodiments, for example in a use for treating a cancer, in a first cycle of treatment, the antibody-drug conjugate comprising an anti-CEACAM5-antibody may be administered after the anti-VEGFR-2 antibody and before the anti-PD-1 antibody or the anti-PD-L1 antibody, and in a subsequent cycle of treatment the ADC may be administered after the anti-VEGFR-2 antibody and before the anti-PD-1 antibody or the anti-PD-L1 antibody.

In some embodiments, for example in a use for treating a cancer, in a first cycle of treatment, the antibody-drug conjugate comprising an anti-CEACAM5-antibody may be administered after the anti-VEGFR-2 antibody and before the anti-PD-1 antibody or the anti-PD-L1 antibody, and in a subsequent cycle of treatment the ADC may be administered before the anti-VEGFR-2 antibody and before the anti-PD-1 antibody or the anti-PD-L1 antibody.

In some embodiments, for example in a use for treating a cancer, in a first cycle of treatment, the antibody-drug conjugate comprising an anti-CEACAM5-antibody may be administered before the anti-VEGFR-2 antibody and before the anti-PD-1 antibody or the anti-PD-L1 antibody, and in a subsequent cycle of treatment the ADC may be administered before the anti-VEGFR-2 antibody and before the anti-PD-1 antibody or the anti-PD-L1 antibody.

In some embodiments, the sequence of administration of the antibody-drug conjugate comprising an anti-CEACAM5-antibody, of the anti-VEGFR-2 antibody and of the anti-PD-1 antibody or the anti-PD-L1 antibody remains constant along the cycles of treatment. In some embodiments, the anti-VEGFR-2 antibody is administered before the antibody-drug conjugate, and the antibody-drug conjugate is administered before the anti-PD-1 antibody or the anti-PD-L1 antibody

In some embodiments, for example in a use for treating a cancer such as gastric cancer, a GEJ adenocarcinoma, a pancreatic cancer, or a lung cancer, the antibody-drug conjugate comprising an anti-CEACAM5-antibody may be

administered after the anti-VEGFR-2 antibody and before the anti-PD-1 antibody or the anti-PD-L1 antibody for the first and all subsequent cycles of treatment.

In some embodiments, the dosage regimen may comprise administration of a dose over a period of about 10 minutes to about 48 hours, or of about 1h to about 48h, such as over a period of 1h to 4h. In some embodiments, the dosage regimen may comprise administration of a dose over a period of about 1h.

In an aspect of this embodiment, the dose frequency varies from twice a week to once every three weeks, for example every 2 weeks or every 3 weeks.

In an aspect of this embodiment, a dose frequency may be once every three weeks.

In an embodiment, the treatment duration is of at least 4 or 6 months.

***Cycle of treatment***

A treatment, or course of treatment, may comprise at least one cycle of treatment.

In some embodiments, a treatment may comprise a first cycle of treatment, i.e., cycle 1, and at least one additional cycle of treatment, i.e., cycle(s) 2, 3, 4, 5, 6, 7, 8, 9, 10, 11, 12, 13, 14, 15, 16, or more.

In a further embodiment, the antibody-drug conjugate comprising an anti-CEACAM5-antibody, the anti-VEGFR-2 antibody, and the anti-PD-1 antibody or the anti-PD-L1 antibody may be administered for a treatment comprising from about 8 to about 16 cycles. According to an embodiment, the cycle may be selected from a 1-week cycle, a 2-weeks cycle, a 3-weeks cycle, a 4-weeks cycle, a 5-weeks cycle, a 6-weeks cycle, or more weeks cycle.

The first cycle and the additional cycle(s) may be identical or different.

For example, the first cycle may comprise an administration of a loading dose (or first dose), and the additional cycle(s) may comprise an administration of a subsequent dose (or second), i.e., different dosages for the loading and subsequent doses.

In the disclosure, "additional cycle" and "subsequent cycle" are used interchangeably.

Alternatively, the first and additional cycles may comprise an administration of a same dose, i.e., same dosage for the loading and subsequent doses. In such embodiment, "loading" and "subsequent" doses are named "dose".

In some embodiments, the antibody-drug conjugate comprising an anti-CEACAM5-antibody may be administered at a loading dose in a first cycle and at a subsequent dose in additional cycle(s), i.e., different dosages for the loading and subsequent doses.

In some embodiments, the antibody-drug conjugate comprising an anti-CEACAM5-antibody may be administered at a same dose in a first cycle and additional cycle(s), i.e., same dosage for the loading and subsequent doses.

In some embodiments, the anti-VEGFR-2 antibody may be administered at a loading dose in a first cycle and at a subsequent dose in additional cycle(s), i.e., different dosages for the loading and subsequent doses.

In some embodiments, the anti-VEGFR-2 antibody may be administered at a same dose in a first cycle and additional cycle(s), i.e., same dosage for the loading and subsequent doses.

In some embodiments, the anti-PD-1 antibody or the anti-PD-L1 antibody may be administered at a loading dose in a first cycle and at a subsequent dose in additional cycle(s), i.e., different dosages for the loading and subsequent doses.

In some embodiments, the anti-PD-1 antibody or the anti-PD-L1 antibody may be administered at a same dose in a first cycle and additional cycle(s), i.e., same dosage for the loading and subsequent doses.

In some embodiments, the antibody-drug conjugate comprising an anti-CEACAM5-antibody may be administered at a same dose in a first cycle and additional cycle(s), the anti-VEGFR-2 antibody, and the anti-PD-1 antibody or the anti-PD-L1 antibody may be administered at a same dose in a first cycle and additional cycle(s), i.e., same dosage for the loading and subsequent doses.

In some embodiments, the antibody-drug conjugate comprising an anti-CEACAM5-antibody may be administered at a loading dose in a first cycle and at a subsequent dose in additional cycle(s), i.e., different dosages for the loading and subsequent doses, the anti-VEGFR-2 antibody may be administered at a same dose in a first cycle and additional cycle(s), i.e., same dosage for the loading and subsequent doses, and the anti-PD-1 antibody or the anti-PD-L1 antibody may be administered at a same dose in a first cycle and additional cycle(s), i.e., same dosage for the loading and subsequent doses.

In some embodiments, the antibody-drug conjugate comprising an anti-CEACAM5-antibody may be administered at a loading dose in a first cycle and at a subsequent dose in additional cycle(s), i.e., different dosages for the loading and subsequent doses, the anti-VEGFR-2 antibody may be administered at a loading dose in a first cycle and at a subsequent dose in additional cycle(s), i.e., different dosages for the loading and subsequent doses, and the anti-PD-1 antibody or the anti-PD-L1 antibody may be administered at a same dose in a first cycle and additional cycle(s), i.e., same dosage for the loading and subsequent doses.

A cycle of treatment may last from about 1 to about 6 weeks, from 1 to 4 weeks, from 1 to 3 weeks.

In some embodiments, a cycle of treatment may last at least about two weeks.

In some embodiments, a cycle of treatment may last at least about three weeks.

In some embodiment, a cycle of treatment may comprise a period of treatment at least on day 1, for example on day 1, 2, 3, 4, 5 or 6, of the cycle and a period of rest lasting until the completion of said cycle. The periods of treatment and the periods of rest may be identical or different between a first cycle and an at least one additional cycle. In some embodiments, the periods of treatment and the periods of rest may be identical between a first cycle and an at least one additional cycle.

In some embodiments, the antibody-drug conjugate comprising an anti-CEACAM5-antibody, the anti-VEGFR-2 antibody, and the anti-PD-1 antibody or anti-PD-L1 antibody may be administered on the same day, or each at different days, one at a day the two others at a different day, of a cycle of treatment.

In some embodiments, one of the antibody-drug conjugate comprising an anti-CEACAM5-antibody, the anti-VEGFR-2 antibody, and the anti-PD-1 antibody or anti-PD-L1 antibody may be administered at day 1 of a cycle of treatment, and the others may be administered at one or two subsequent days of the cycle of treatment.

In some embodiment, a cycle of treatment, i.e., first and additional cycles, may comprise a period of treatment on day 1 of the cycle and a period of rest lasting until the completion of said cycle.

In some embodiments, the antibody-drug conjugate comprising an anti-CEACAM5-antibody, the anti-VEGFR-2 antibody, and the anti-PD-1 antibody or anti-PD-L1 antibody may be administered at day 1 of a first cycle of treatment and at day 1 of an at least one additional cycle(s) of treatment.

The antibody-drug conjugate comprising an anti-CEACAM5-antibody, the anti-VEGFR-2 antibody, and the anti-PD-1 antibody or anti-PD-L1 antibody may be administered at day 1 of each cycle of treatment.

A treatment (or course of treatment) may comprise at least a first cycle (cycle 1) of treatment and at least one additional (subsequent) cycle. A treatment may comprise from 2 to 16, from 3 to 15, from 4 to 14, from 5 to 13, from 6 to 12, from 7 to 11, from 8 to 10, or about 9 cycles. A treatment may comprise 2, 3, 4, 5, 6, 7, 8, 9, 10, 11, 12, 13, 14, 15, 16 or more cycles.

In some embodiments, the antibody-drug conjugate comprising an anti-CEACAM5-antibody, the anti-VEGFR-2 antibody, and the an anti-PD-1 antibody or anti-PD-L1 antibody may be administered once per cycle. The administration may be carried out on day one of each cycle.

***ADC dosages***

In some embodiments, it is disclosed an antibody-drug conjugate comprising an anti-CEACAM5-antibody and a chemotherapeutic agent for use for treating a cancer in combination with an anti-VEGFR-2 antibody and an anti-PD-1 antibody or anti-PD-L1 antibody, wherein the antibody-drug conjugate is administered at a dose of 60 $mg/m^2$ to 210 $mg/m^2$, or from about 80 to about 170 $mg/m^2$, or from about 100 to about 170 $mg/m^2$, or from about 120 to about 170 $mg/m^2$, or from about 135 to about 170 $mg/m^2$, or from about 150 to about 170 $mg/m^2$.

In some embodiments, the antibody-drug conjugate is administered at a dose of from about 60 to about 210 $mg/m^2$, or from about 80 to about 170 $mg/m^2$, or from about 100 to about 150 $mg/m^2$.

In various embodiments, the antibody-drug conjugate comprising the anti-CEACAM5 antibody is administered at a dose of about 60, 70, 80, 90, 100, 110, 120, 130, 135, 140, 145, 150, 155, 160, 165, 170, 175, 180, 185, 190, 195, 200, 205, or about 210 $mg/m^2$.

In various embodiments, the antibody-drug conjugate comprising the anti-CEACAM5 antibody is administered at a dose of about 60, 80, 100, 120, 135, 150, 170, 180, 190 or about 210 $mg/m^2$.

In some embodiments, the ADC may be administered at a dose of about 80 $mg/m^2$ to about 170 $mg/m^2$, or at a dose of about 80 $mg/m^2$ to about 150 $mg/m^2$, or at a dose of about 100 $mg/m^2$ to about 120 $mg/m^2$.

In some embodiments, the ADC may be administered at a dose of about 80 $mg/m^2$ or about 100 $mg/m^2$ or about 120 $mg/m^2$ or about 150 $mg/m^2$ or about 170 $mg/m^2$.

In some embodiments, the ADC may be administered at a dose of about 150 $mg/m^2$ or about 120 $mg/m^2$.

According to an embodiment, the antibody-drug conjugate comprising the anti-CEACAM5 antibody is administered at a dose of about 80 $mg/m^2$.

In some embodiments, the ADC may be administered at a dose of about 100 $mg/m^2$.

In some embodiments, the ADC may be administered at a dose of about 120 $mg/m^2$.

In some embodiments, the ADC may be administered at a dose of about 150 $mg/m^2$.

In some embodiments, the ADC may be administered at a dose of about 170 $mg/m^2$.

According to an embodiment, the antibody-drug conjugate comprising the anti-CEACAM5 antibody is administered at a dose of about 80, 100, 120, 150 or about 170 $mg/m^2$, as a loading dose (or first dose).

According to an embodiment, the antibody-drug conjugate comprising the anti-CEACAM5 antibody is administered at a dose of about 120 or about 150 $mg/m^2$, as a loading dose.

According to an embodiment, the antibody-drug conjugate comprising the anti-CEACAM5 antibody is administered at a dose of about 80 $mg/m^2$, as a loading dose.

According to an embodiment, the antibody-drug conjugate comprising the anti-CEACAM5 antibody is administered at a dose of about 100 $mg/m^2$, as a loading dose.

According to an embodiment, the antibody-drug conjugate comprising the anti-CEACAM5 antibody is administered at a dose of about 120 $mg/m^2$, as a loading dose.

According to an embodiment, the antibody-drug conjugate comprising the anti-CEACAM5 antibody is administered at a dose of about 150 $mg/m^2$, as a loading dose.

According to an embodiment, the antibody-drug conjugate comprising the anti-CEACAM5 antibody is administered at a dose of about 170 $mg/m^2$, as a loading dose.

According to an embodiment, the loading dose is for a cycle of treatment of 2 weeks.

According to an embodiment, the loading dose is for a cycle of treatment of 3 weeks.

A loading dose may be administered at day 1 of the first cycle.

On day 1 of a first cycle, the antibody-drug conjugate comprising an anti-CEACAM5-antibody may be administered at a dose of about 80 $mg/m^2$, 100 $mg/m^2$, 120 $mg/m^2$, 150 $mg/m^2$ or about 170 $mg/m^2$.

On day 1 of a first cycle, the antibody-drug conjugate comprising an anti-CEACAM5-antibody may be administered at a dose of about 120 $mg/m^2$ or about 150 $mg/m^2$.

On day 1 of a first cycle, the antibody-drug conjugate comprising an anti-CEACAM5-antibody may be administered at a dose of about 120 $mg/m^2$.

On day 1 of a first cycle, the antibody-drug conjugate comprising an anti-CEACAM5-antibody may be administered at a dose of about 150 $mg/m^2$.

A first cycle of treatment may be 3 weeks.

According to an embodiment, the antibody-drug conjugate comprising the anti-CEACAM5 antibody may be administered at a dose of about 80, 100, 120, 150 or about 170 $mg/m^2$, as a subsequent dose (or second dose).

According to an embodiment, the antibody-drug conjugate comprising the anti-CEACAM5 antibody may be administered at a dose of about 120 or about 150 $mg/m^2$, as a subsequent dose (or second dose).

According to an embodiment, the antibody-drug conjugate comprising the anti-CEACAM5 antibody may be administered at a dose of about 80 $mg/m^2$, as a subsequent dose.

According to an embodiment, the antibody-drug conjugate comprising the anti-CEACAM5 antibody may be administered at a dose of about 100 $mg/m^2$, as a subsequent dose.

According to an embodiment, the antibody-drug conjugate comprising the anti-CEACAM5 antibody may be administered at a dose of about 120 $mg/m^2$, as a subsequent dose.

According to an embodiment, the antibody-drug conjugate comprising the anti-CEACAM5 antibody may be administered at a dose of about 150 $mg/m^2$, as a subsequent dose.

According to an embodiment, the antibody-drug conjugate comprising the anti-CEACAM5 antibody may be administered at a dose of about 170 $mg/m^2$, as a subsequent dose.

A subsequent dose may be administered at day 1 of cycle(s) subsequent to the first cycle (the subsequent or additional cycles).

On day 1 of additional cycles (at least one cycle subsequent to the first cycle), the antibody-drug conjugate comprising an anti-CEACAM5-antibody may be administered at a dose of about 80 $mg/m^2$.

On day 1 of additional cycles (at least one cycle subsequent to the first cycle), the antibody-drug conjugate comprising an anti-CEACAM5-antibody may be administered at a dose of about 100 $mg/m^2$.

On day 1 of additional cycles (at least one cycle subsequent to the first cycle), the antibody-drug conjugate comprising an anti-CEACAM5-antibody may be administered at a dose of about 120 $mg/m^2$.

On day 1 of additional cycles (at least one cycle subsequent to the first cycle), the antibody-drug conjugate comprising an anti-CEACAM5-antibody may be administered at a dose of about 150 $mg/m^2$.

On day 1 of additional cycles (at least one cycle subsequent to the first cycle), the antibody-drug conjugate comprising an anti-CEACAM5-antibody may be administered at a dose of about 170 $mg/m^2$.

A subsequent (or additional) cycle of treatment may be 3 weeks.

According to an embodiment, the antibody-drug conjugate comprising the anti-CEACAM5 antibody is administered at a dose of about 80, 100, 120, 150 or about 170 $mg/m^2$, as a loading dose, on a first cycle of treatment, e.g., at day 1, and then at a dose of about 80, 100, 120, 150 or about 170 $mg/m^2$, as a subsequent dose, e.g., at day 1, on additional cycle(s).

According to an embodiment, the antibody-drug conjugate comprising the anti-CEACAM5 antibody is administered at a dose of about 120 or about 150 $mg/m^2$, as a loading dose, on a first cycle of treatment, e.g., at day 1, and then at a dose of about 120 or about 150 $mg/m^2$, as a subsequent dose, e.g., at day 1, on additional cycle(s).

According to an embodiment, the antibody-drug conjugate comprising the anti-CEACAM5 antibody is administered at a dose of about 150 $mg/m^2$, as a loading dose, on a first cycle of treatment, e.g., at day 1, and then at a dose of about 120 or about 150 $mg/m^2$, as a subsequent dose, e.g., at day 1, on additional cycle(s).

According to an embodiment, the antibody-drug conjugate comprising the anti-CEACAM5 antibody is administered at a dose of about 150 mg/$m^2$, as a loading dose, on day 1 of a first cycle of treatment, and then at a dose of about 120 or about 150 mg/$m^2$, as a subsequent dose, on day 1 of (an) additional cycle(s). The cycle may be about 3 weeks.

According to an embodiment, the antibody-drug conjugate comprising the anti-CEACAM5 antibody is administered at a dose of about 150 mg/$m^2$, as a loading dose, on day 1 of a first cycle of treatment, and then at a dose of about 150 mg/$m^2$, as a subsequent dose, on day 1 of (an) additional cycle(s). The cycle may be about 3 weeks.

According to an embodiment, the antibody-drug conjugate comprising the anti-CEACAM5 antibody is administered at a dose of about 150 mg/$m^2$, as a loading dose, on day 1 of a first cycle of treatment, and then at a dose of about 120 mg/$m^2$, as a subsequent dose, on day 1 of (an) additional cycle(s). The cycle may be about 3 weeks.

In certain embodiments, for subjects with a body surface area (BSA) >2.2 $m^2$, the dose of the antibody-drug conjugate comprising an anti-CEACAM5-antibody may be calculated based on a BSA of 2.2 $m^2$.

The antibody-drug conjugate may be tusamitamab ravtansine (huMAb2-3-SPDB-DM4).

***Anti-VEGFR-2 antibody dosage***

The anti-VEGFR-2 antibody is administered at a dose of 2 mg/kg to 20 mg/kg, or from about 4 to about 15 mg/kg, or from about 6 to about 10 mg/kg, or at about 8 mg/kg, or at about 10 mg/kg. The anti-VEGFR-2 antibody may be administered at a dose of about 2, 4, 6, 8, 10, 12, 14, 16, 18 or about 20 mg/kg.

In some embodiments, the anti-VEGFR2 antibody may be administered at a dose of 6 mg/kg, or at a dose of 8 mg/kg, or at a dose of 10 mg/kg.

In some embodiments, the anti-VEGFR2 antibody may be administered at a dose of 6 mg/kg.

In some embodiments, the anti-VEGFR2 antibody may be administered at a dose of 8 mg/kg.

In some embodiments, the anti-VEGFR2 antibody may be administered at a dose of 10 mg/kg.

According to an embodiment, the anti-VEGFR2 antibody is administered at a dose of about 6 mg/kg, or at a dose of 8 mg/kg, or at a dose of 10 mg/kg, as a loading dose (or first dose).

According to an embodiment, the anti-VEGFR2 antibody is administered at a dose of about 8 mg/kg, or about 10 mg/kg, as a loading dose.

According to an embodiment, the anti-VEGFR2 antibody is administered at a dose of about 6 mg/kg, as a loading dose.

According to an embodiment, the anti-VEGFR2 antibody is administered at a dose of about 8 mg/kg, as a loading dose.

According to an embodiment, the anti-VEGFR2 antibody is administered at a dose of about 10 mg/kg, as a loading dose.

According to an embodiment, the loading dose is for a cycle of treatment of 2 weeks.

According to an embodiment, the loading dose is for a cycle of treatment of 3 weeks.

A loading dose may be administered at day 1 of the first cycle.

On day 1 of a first cycle, the antibody-drug conjugate comprising an anti-CEACAM5-antibody may be administered at a dose of about 6 mg/kg, 8 mg/kg, or about 10 mg/kg.

On day 1 of a first cycle, the antibody-drug conjugate comprising an anti-CEACAM5-antibody may be administered at a dose of about 8 mg/kg or about 10 mg/kg.

On day 1 of a first cycle, the antibody-drug conjugate comprising an anti-CEACAM5-antibody may be administered at a dose of about 8 mg/kg.

On day 1 of a first cycle, the antibody-drug conjugate comprising an anti-CEACAM5-antibody may be administered at a dose of about 10 mg/kg.

A first cycle of treatment may be 3 weeks.

According to an embodiment, the anti-VEGFR2 antibody may be administered at a dose of about 6 mg/kg, 8 mg/kg, or about 10 mg/kg, as a subsequent dose (or second dose).

According to an embodiment, the anti-VEGFR2 antibody may be administered at a dose of about 8 or about 10 mg/kg, as a subsequent dose (or second dose).

According to an embodiment, the anti-VEGFR2 antibody may be administered at a dose of about 6 mg/kg, as a subsequent dose.

According to an embodiment, the anti-VEGFR2 antibody may be administered at a dose of about 8 mg/kg, as a subsequent dose.

According to an embodiment, the anti-VEGFR2 antibody may be administered at a dose of about 10 mg/kg, as a subsequent dose.

A subsequent dose may be administered at day 1 of cycle(s) subsequent to the first cycle (the subsequent or additional cycles).

On day 1 of additional cycles (at least one cycle subsequent to the first cycle), the antibody-drug conjugate comprising an anti-CEACAM5-antibody may be administered at a dose of about 6 mg/kg.

On day 1 of additional cycles (at least one cycle subsequent to the first cycle), the antibody-drug conjugate comprising an anti-CEACAM5-antibody may be administered at a dose of about 8 mg/kg.

On day 1 of additional cycles (at least one cycle subsequent to the first cycle), the antibody-drug conjugate comprising an anti-CEACAM5-antibody may be administered at a dose of about 10 mg/kg.

A subsequent cycle of treatment may be 3 weeks.

According to an embodiment, the anti-VEGFR2 antibody is administered at a dose of about 8 or about 10 mg/kg, as a loading dose, on a first cycle of treatment, e.g., at day 1, and then at a dose of about 6, 8, or about 10 mg/kg, as a subsequent dose, e.g., at day 1, on additional cycle(s).

According to an embodiment, the anti-VEGFR2 antibody is administered at a dose of about 8 or about 10 mg/kg, as a loading dose, on a first cycle of treatment, e.g., at day 1, and then at a dose of about 8 or about 10 mg/kg, as a subsequent dose, e.g., at day 1, on additional cycle(s).

According to an embodiment, the anti-VEGFR2 antibody is administered at a dose of about 10 mg/kg, as a loading dose, on a first cycle of treatment, e.g., at day 1, and then at a dose of about 6, or about 8, or about 10 mg/kg, as a subsequent dose, e.g., at day 1, on additional cycle(s).

According to an embodiment, the anti-VEGFR2 antibody is administered at a dose of about 10 mg/kg, as a loading dose, on a first cycle of treatment, e.g., at day 1, and then at a dose of about 8, or about 10 mg/kg, as a subsequent dose, e.g., at day 1, on additional cycle(s).

According to an embodiment, the anti-VEGFR2 antibody is administered at a dose of about 10 mg/kg, as a loading dose, on a first cycle of treatment, e.g., at day 1, and then at a dose of about 10 mg/kg, as a subsequent dose, e.g., at day 1, on additional cycle(s).

According to an embodiment, the anti-VEGFR2 antibody is administered at a dose of about 10 mg/kg, as a loading dose, on a first cycle of treatment, e.g., at day 1, and then at a dose of about 8 mg/kg, as a subsequent dose, e.g., at day 1, on additional cycle(s).

According to an embodiment, the anti-VEGFR2 antibody is administered at a dose of about 10 mg/kg, as a loading dose, on a first cycle of treatment, e.g., at day 1, and then at a dose of about 8 mg/kg, as a subsequent dose, e.g., at day 1, a second cycle, and then at a dose of about 6 mg/kg, as a subsequent dose, e.g., at day 1, on additional cycle(s).

According to an embodiment, the anti-VEGFR2 antibody is administered at a dose of about 8 or 10 mg/kg, as a loading dose, on day 1 of a first cycle of treatment, and then at a dose of about 6, 8 or about 10 mg/kg, as a subsequent dose, on day 1 of (an) additional cycle(s). The cycle may be about 3 weeks.

According to an embodiment, the anti-VEGFR2 antibody is administered at a dose of about 8 or 10 mg/kg, as a loading dose, on day 1 of a first cycle of treatment, and then at a dose of about 8 or 10 mg/kg, as a subsequent dose, on day 1 of (an) additional cycle(s). The cycle may be about 3 weeks.

According to an embodiment, the anti-VEGFR2 antibody is administered at a dose of about 10 mg/kg, as a loading dose, on day 1 of a first cycle of treatment, and then at a dose of about 6, 8, or 10 mg/kg, as a subsequent dose, on day 1 of (an) additional cycle(s). The cycle may be about 3 weeks.

According to an embodiment, the anti-VEGFR2 antibody is administered at a dose of about 10 mg/kg, as a loading dose, on day 1 of a first cycle of treatment, and then at a dose of about 8 or 10 mg/kg, as a subsequent dose, on day 1 of (an) additional cycle(s). The cycle may be about 3 weeks.

According to an embodiment, the anti-VEGFR2 antibody is administered at a dose of about 10 mg/kg, as a loading dose, on day 1 of a first cycle of treatment, and then at a dose of about 10 mg/kg, as a subsequent dose, on day 1 of (an) additional cycle(s). The cycle may be about 3 weeks.

According to an embodiment, the anti-VEGFR2 antibody is administered at a dose of about 10 mg/kg, as a loading dose, on day 1 of a first cycle of treatment, and then at a dose of about 8 mg/kg, as a subsequent dose, on day 1 of (an) additional cycle(s). The cycle may be about 3 weeks.

According to an embodiment, the anti-VEGFR2 antibody is administered at a dose of about 10 mg/kg, as a loading dose, on day 1 of a first cycle of treatment, and then at a dose of about 8 mg/kg, as a subsequent dose, on day 1 of a second cycle, and then at a dose of about 6 mg/kg, as a subsequent dose, on day 1 of (an) additional cycle(s). The cycle may be about 3 weeks.

The anti-VEGFR-2 antibody may be ramucirumab.

***Anti-PD-1 antibody or the anti-PD-L1 antibody dosage***

In some embodiments, the anti-PD-1 antibody or the anti-PD-L1 antibody may be administered at a dose of about 150 mg to about 400 mg, or at a dose of about 150 mg to about 300 mg or at a dose of about 200 mg. The anti-PD-1 antibody or the anti-PD-L1 antibody may be administered at a dose of about 150, 155, 160, 165, 170, 175, 185, 190, 195, 200, 205, 210, 215, 22, 225, 235, 240, 245, 250, 255, 260, 265, 270, 275, 280, 285, 290, 295, or about 300 mg.

In some embodiments, the anti-PD-1 antibody or the anti-PD-L1 antibody may be administered at a dose of

about 200 mg.

According to an embodiment, the anti-PD-1 antibody or the anti-PD-L1 antibody is administered a dose of about 150 mg to about 400 mg, or at a dose of about 150 mg to about 300 mg or at a dose of about 200 mg, as a loading dose (or first dose).

According to an embodiment, the anti-PD-1 antibody or the anti-PD-L1 antibody is administered a dose of about 200 mg, as a loading dose (or first dose).

According to an embodiment, the loading dose is for a cycle of treatment of 2 weeks.

According to an embodiment, the loading dose is for a cycle of treatment of 3 weeks.

A loading dose may be administered at day 1 of the first cycle.

On day 1 of a first cycle, the anti-PD-1 antibody or the anti-PD-L1 antibody is administered a dose of about 150 mg to about 400 mg, or at a dose of about 150 mg to about 300 mg or at a dose of about 200 mg.

On day 1 of a first cycle, the anti-PD-1 antibody or the anti-PD-L1 antibody is administered a dose of about 200 mg.

A first cycle of treatment may be 3 weeks.

According to an embodiment, the anti-PD-1 antibody or the anti-PD-L1 antibody is administered a dose of about 150 mg to about 400 mg, or at a dose of about 150 mg to about 300 mg or at a dose of about 200 mg, as a subsequent dose (or second dose).

According to an embodiment, the anti-PD-1 antibody or the anti-PD-L1 antibody is administered a dose of about 200 mg, as a subsequent dose (or second dose).

A subsequent dose may be administered at day 1 of cycle(s) subsequent to the first cycle (the subsequent or additional cycles).

On day 1 of additional cycles (at least one cycle subsequent to the first cycle), the anti-PD-1 antibody or the anti-PD-L1 antibody is administered a dose of about 200 mg.

A subsequent cycle of treatment may be 3 weeks.

According to an embodiment, the anti-PD-1 antibody or the anti-PD-L1 antibody is administered a dose of about 150 mg to about 400 mg, or at a dose of about 150 mg to about 300 mg or at a dose of about 200 mg, as a loading dose, on a first cycle of treatment, e.g., at day 1, and then at a dose of about 150 mg to about 400 mg, or at a dose of about 150 mg to about 300 mg or at a dose of about 200 mg, as a subsequent dose, e.g., at day 1, on additional cycle(s).

According to an embodiment, the anti-PD-1 antibody or the anti-PD-L1 antibody is administered a dose of about 200 mg, as a loading dose, on a first cycle of treatment, e.g., at day 1, and then at a dose of about 200 mg, as a subsequent dose, e.g., at day 1, on additional cycle(s).

According to an embodiment, the anti-PD-1 antibody or the anti-PD-L1 antibody is administered at a dose of about 150 mg to about 400 mg, or at a dose of about 150 mg to about 300 mg or at a dose of about 200 mg, as a loading dose, on day 1 of a first cycle of treatment, and then at a dose of about 150 mg to about 400 mg, or at a dose of about 150 mg to about 300 mg or at a dose of about 200 mg, as a subsequent dose, on day 1 of (an) additional cycle(s). The cycle may be about 3 weeks.

According to an embodiment, the anti-PD1 or anti-PDL1 antibody is administered at a dose of about 200 mg, as a loading dose, on day 1 of a first cycle of treatment, and then at a dose of about 200 mg, as a subsequent dose, on day 1 of (an) additional cycle(s). The cycle may be about 3 weeks.

In some embodiments, the anti-PD-1 antibody may be pembrolizumab.

***Combination dosages***

According to an embodiment, the antibody-drug conjugate comprising the anti-CEACAM5 antibody may be administered at a dose of about 80, 100, 120, 150 or about 170 $mg/m^2$, at day 1 on a first cycle of treatment, as a loading dose, the anti-VEGFR-2 antibody may be administered at a dose of about 8 or 10 mg/kg, at day 1 on a first cycle of treatment, as a loading dose, and the anti-PD-1 antibody or the anti-PD-L1 antibody may be administered at a dose of about 150 mg to about 400 mg, or at a dose of about 150 mg to about 300 mg or at a dose of about 200 mg at day 1 on a first cycle of treatment, as a loading dose.

According to an embodiment, the antibody-drug conjugate comprising the anti-CEACAM5 antibody may be administered at a dose of about 80, 100, 120, or about 150 $mg/m^2$, as a subsequent dose, e.g., at day 1, on additional cycle(s), the anti-VEGFR-2 antibody may be administered at a dose of about 6, 8 or 10 mg/kg, as a subsequent dose, e.g., at day 1, on additional cycle(s), and the anti-PD-1 antibody or the anti-PD-L1 antibody may be administered at a dose of about 150 mg to about 400 mg, or at a dose of about 150 mg to about 300 mg or at a dose of about 200 mg as a subsequent dose, e.g., at day 1, on additional cycle(s).

According to an embodiment, the antibody-drug conjugate comprising the anti-CEACAM5 antibody may be administered at a dose of about 80, 100, 120, 150 or about 170 $mg/m^2$, at day 1 on a first cycle of treatment, as a loading dose, and at a dose of about 80, 100, 120, or about 150 $mg/m^2$, as a subsequent dose, e.g., at day 1, on additional

cycle(s), the anti-VEGFR-2 antibody may be administered at a dose of about 8 or 10 mg/kg, at day 1 on a first cycle of treatment, as a loading dose, and at a dose of about 6, 8 or 10 mg/kg, as a subsequent dose, e.g., at day 1, on additional cycle(s), and the anti-PD-1 antibody or the anti-PD-L1 antibody may be administered at a dose of about 150 mg to about 400 mg, or at a dose of about 150 mg to about 300 mg or at a dose of about 200 mg at day 1 on a first cycle of treatment, as a loading dose, and at a dose of about 150 mg to about 400 mg, or at a dose of about 150 mg to about 300 mg or at a dose of about 200 mg as a subsequent dose, e.g., at day 1, on additional cycle(s). The cycle(s) may be about 2 or 3 weeks. The cycle(s) may be about 3 weeks.

The antibody-drug conjugate may be administered after the anti-VEGFR-2 antibody and before the anti-PD-1 antibody or the anti-PD-L1 antibody.

In some embodiments, at day 1 of a first cycle of treatment, (i) the antibody-drug conjugate is administered at a dose of 150 $mg/m^2$, (ii) the anti-VEGFR-2 antibody is administered at a dose of 10 mg/kg, and (iii) the anti-PD-1 antibody or the anti-PD-L1 antibody is administered at a dose of 200 mg.

In some embodiments, at day 1 of an at least one additional cycle of treatment, the antibody-drug conjugate is administered at a dose of 120 or 150 $mg/m^2$.

In some embodiments, at day 1 of an at least one additional cycle of treatment, the anti-VEGFR-2 antibody is administered at a dose of 6, 8 or 10 mg/kg.

In some embodiments, at day 1 of an at least one additional cycle of treatment, the anti-PD-1 antibody or the anti-PD-L1 antibody is administered at a dose of 200 mg.

In some embodiments, the antibody-drug conjugate comprising the anti-CEACAM5 antibody may be administered at a dose of about 120 or 150 $mg/m^2$, at day 1 on a first cycle of treatment, as a loading dose, and at a dose of about 120 or 150 $mg/m^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 6, 8 or 10 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 $mg/m^2$, at day 1 on a first cycle of treatment, as a loading dose, and at a dose of about 120 or 150 $mg/m^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 6, 8 or 10 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 $mg/m^2$, at day 1 on a first cycle of treatment, as a loading dose, and at a dose of about 120 or 150 $mg/m^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 8 or 10 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 $mg/m^2$, at day 1 on a first cycle of treatment, as a loading dose, and at a dose of about 120 or 150 $mg/m^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 8 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 $mg/m^2$, at day 1 on a first cycle of treatment, as a loading dose, and at a dose of about 120 or 150 $mg/m^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 10 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 $mg/m^2$, at day 1 on a first cycle of treatment, as a loading dose, and at a dose of about 120 or 150 $mg/m^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 6 mg/kg, as a subsequent dose, e.g., at day 1, on additional cycle(s), and the anti-PD-1 antibody or the anti-PD-L1 antibody

may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 mg/m$^2$, at day 1 on a first cycle of treatment, as a loading dose, and at a dose of about 150 mg/m$^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 6, 8 or 10 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 mg/m$^2$, at day 1 on a first cycle of treatment, as a loading dose, and at a dose of about 150 mg/m$^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 10 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 mg/m$^2$, at day 1 on a first cycle of treatment, as a loading dose, and at a dose of about 150 mg/m$^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 8 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 mg/m$^2$, at day 1 on a first cycle of treatment, as a loading dose, and at a dose of about 150 mg/m$^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 6 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 mg/m$^2$, at day 1 on a first cycle of treatment, as a loading dose, and at a dose of about 150 mg/m$^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 8 mg/kg, as a subsequent dose, on day 1 of a second cycle, and then at a dose of about 6 mg/kg, as a subsequent dose, on day 1 of (an) additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 mg/m$^2$, at day 1 on a first cycle of treatment, as a loading dose, and at a dose of about 120 mg/m$^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 6, 8 or 10 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 mg/m$^2$, at day 1 on a first cycle of treatment, as a loading dose, and at a dose of about 120 mg/m$^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 10 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 mg/m$^2$, at day 1 on a first cycle of treatment, as a loading dose, and at a dose of about 120 mg/m$^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 8 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of

about 200 mg as a subsequent dose, e.g., at day 1, on additional cycle(s).

In some embodiments, the antibody-drug conjugate comprising the anti-CEACAM5 antibody may be administered at a dose of about 150 mg/$m^2$, at day 1 on a first cycle of treatment, as a loading dose, and at a dose of about 120 mg/$m^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 6 mg/kg, as a subsequent dose, e.g., at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

In some embodiments, the antibody-drug conjugate comprising the anti-CEACAM5 antibody may be administered at a dose of about 150 mg/$m^2$, at day 1 on a first cycle of treatment, as a loading dose, and at a dose of about 120 mg/$m^2$, as a subsequent dose, e.g., at day 1, on additional cycle(s), the anti-VEGFR-2 antibody may be administered at a dose of about 10 mg/kg, at day 1 on a first cycle of treatment, as a loading dose, and at a dose of about 8 mg/kg, as a subsequent dose, on day 1 of a second cycle, and then at a dose of about 6 mg/kg, as a subsequent dose, on day 1 of (an) additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, e.g., at day 1, on additional cycle(s).

The cycle(s) are about 3 weeks.

The antibody-drug conjugate may be administered after the anti-VEGFR-2 antibody and before the anti-PD-1 antibody or the anti-PD-L1 antibody.

In some embodiments, at day 1 of a first cycle of treatment, (i) the antibody-drug conjugate is administered at a dose of 150 mg/$m^2$, (ii) the anti-VEGFR-2 antibody is administered at a dose of 10 mg/kg, and (iii) the anti-PD-1 antibody or the anti-PD-L1 antibody is administered at a dose of 200 mg, and the cycle of treatment may be 3 weeks.

In some embodiments, at day 1 of an at least one additional cycle of treatment, the antibody-drug conjugate is administered at a dose of 120 or 150 mg/$m^2$, and the cycle of treatment may be 3 weeks.

In some embodiments, at day 1 of an at least one additional cycle of treatment, the anti-VEGFR-2 antibody is administered at a dose of 6, 8 or 10 mg/kg, and the cycle of treatment may be 3 weeks.

In some embodiments, at day 1 of an at least one additional cycle of treatment, the anti-PD-1 antibody or the anti-PD-L1 antibody is administered at a dose of 200 mg, and the cycle of treatment may be 3 weeks.

In some embodiments, at day 1 of a first cycle of treatment, (i) the antibody-drug conjugate may be administered at a dose of 120 or 150 mg/$m^2$, (ii) the anti-VEGFR-2 antibody may be administered at a dose of 8 or 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 or 150 mg/$m^2$, (ii) the anti-VEGFR-2 antibody may be administered at a dose of 6 mg/kg, 8 mg/kg or 10 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$ or 150 mg/$m^2$, (ii) the anti-VEGFR-2 antibody may be administered at a dose of 6 mg/kg, 8 mg/kg, or 10 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment and at day 1 of an at least one additional cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and the cycle of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of 6 mg/kg or 8 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered

at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of 8 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of 6 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of about 8 mg/kg, as a subsequent dose, on day 1 of a second cycle, and then at a dose of about 6 mg/kg, as a subsequent dose, on day 1 of (an) additional cycle(s), and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of 8 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of 6 mg/kg, and (iii) the anti-PD-1 antibody or the anti-PD-L1 antibody may be administered at a dose of 200 mg, and the cycles of treatment may be 3 weeks.

In some embodiments, at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of about 8 mg/kg, as a subsequent dose, on day 1 of a second cycle, and then at a dose of about 6 mg/kg, as a subsequent dose, on day 1 of (an) additional cycle(s), and (iii) the anti-PD-1 antibody or the anti-PD-L1 antibody may be administered at a dose of 200 mg, and the cycles of treatment may be 3 weeks.

In some embodiments, the anti-VEGFR-2 antibody may be administered before the antibody-drug conjugate, and the antibody-drug conjugate may be administered before the anti-PD-1 antibody or the anti-PD-L1 antibody.

The antibody-drug conjugate may be tusamitamab ravtansine (huMAb2-3-SPDB-DM4).

In some embodiments, the anti-VEGFR-2 antibody may be ramucirumab.

In some embodiments, the anti-PD-1 antibody may be pembrolizumab.

***Combined treatment for lung cancer***

In some embodiments, the cancer may be a lung cancer.

In some embodiments, the lung cancer may be a non-squamous non-small cell lung cancer (NSQ NSCLC).

In some embodiments, the non-squamous non-small cell lung cancer may be an advanced or metastatic NSQ NSCLC.

In some embodiments, the non-squamous non-small cell lung cancer has no epidermal growth factor receptor (EGFR) sensitizing mutation or v-raf murine sarcoma viral oncogene homolog B1 (BRAF) mutation or anaplastic lymphoma kinase/c-ros oncogene 1 (ALK/ROS) alterations.

In some embodiments, the lung cancer may be a CEACAM5-positive cancer having a CEACAM5 immunohistochemical intensity ≥ 2+ in ≥ 50% of cancer cells or a CEACAM5 immunohistochemical intensity ≥ 2+ in ≥ 1% and < 50% of cancer cells, as measured by immunohistochemistry.

In some embodiments, the lung cancer may be a CEACAM5-positive cancer having a CEACAM5 immunohistochemical intensity ≥ 2+ in ≥ 50% of cancer cells, as measured by immunohistochemistry.

In some embodiments, the lung cancer may be a CEACAM5-positive cancer having a CEACAM5 immunohistochemical intensity ≥ 2+ in ≥ 50% of cancer cells or a CEACAM5 immunohistochemical intensity ≥ 2+ in ≥ 1% and < 50% of cancer cells, as measured by immunohistochemistry wherein (i) the antibody-drug conjugate may be administered at a dose of about 120 or 150 mg/$m^2$, (ii) the anti-VEGFR-2 antibody may be administered at a dose of about 8 or 10 mg/kg, and (iii) the anti-PD-1 antibody or the anti-PD-L1 antibody may be administered at a dose of 200 mg.

In some embodiments, the lung cancer may be a CEACAM5-positive cancer having a CEACAM5 immunohistochemical intensity ≥ 2+ in ≥ 50% of cancer cells or a CEACAM5 immunohistochemical intensity ≥ 2+ in ≥ 1% and < 50% of cancer cells, as measured by immunohistochemistry wherein (i) the antibody-drug conjugate may be administered at a dose of 120 or 150 mg/$m^2$, (ii) the anti-VEGFR-2 antibody may be administered at a dose of 8 or 10 mg/kg, and (iii) the anti-PD-1 antibody or the anti-PD-L1 antibody may be administered at a dose of 200 mg, on day 1 of a first cycle, and the cycle may be 3 weeks.

In some embodiments, the lung cancer may be a CEACAM5-positive cancer having a CEACAM5 immunohistochemical intensity ≥ 2+ in ≥ 50% of cancer cells or a CEACAM5 immunohistochemical intensity ≥ 2+ in ≥ 1% and < 50% of cancer cells, as measured by immunohistochemistry, wherein (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, on day 1 of a first cycle, and the cycle may be 3 weeks.

In some embodiments, at day 1 of an at least one additional cycle of treatment, the antibody-drug conjugate is administered at a dose of 120 or 150 mg/$m^2$, and the cycle of treatment may be 3 weeks.

In some embodiments, at day 1 of an at least one additional cycle of treatment, the anti-VEGFR-2 antibody is administered at a dose of 6, 8 or 10 mg/kg, and the cycle of treatment may be 3 weeks.

In some embodiments, at day 1 of an at least one additional cycle of treatment, the anti-PD-1 antibody or the anti-PD-L1 antibody is administered at a dose of 200 mg, and the cycle of treatment may be 3 weeks.

In some embodiments, (i) the antibody-drug conjugate may be administered at a subsequent dose of 150 mg/$m^2$, (ii) the anti-VEGFR-2 antibody may be administered at a subsequent dose of 10 mg/kg, and (iii) the anti-PD-1 antibody or the anti-PD-L1 antibody may be administered at a subsequent dose of 200 mg, on day 1 of subsequent cycle(s), and the cycle(s) is/are 3 weeks.

In some embodiments, (i) the antibody-drug conjugate may be administered at a subsequent dose of 120 mg/$m^2$, (ii) the anti-VEGFR-2 antibody may be administered at a subsequent dose of 6 or 8 mg/kg, and (iii) the anti-PD-1 antibody or the anti-PD-L1 antibody may be administered at a subsequent dose of 200 mg, on day 1 of subsequent cycle(s), and the cycle(s) is/are 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, the antibody-drug conjugate comprising the anti-CEACAM5 antibody may be administered at a dose of about 150 mg/$m^2$, at day 1 on a first cycle of treatment, as a loading dose, and at a dose of about 120 or 150 mg/$m^2$, as a subsequent dose, at day 1, on additional cycle(s), the anti-VEGFR-2 antibody may be administered at a dose of about 10 mg/kg, at day 1 on a first cycle of treatment, as a loading dose, and at a dose of about 6, 8 or 10 mg/kg, as a subsequent dose, at day 1, on additional cycle(s), and the anti-PD-1 antibody or the anti-PD-L1 antibody may be administered at a dose of about 200 mg at day 1 on a first cycle of treatment, as a loading dose, and at a dose of about 200 mg as a subsequent dose, at day 1, on additional cycle(s). The cycles of treatment are 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment and at day 1 of an at least one additional cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and the cycle of treatment may be 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of 6 mg/kg or 8 mg/kg, and (iii) the anti-PD-1 antibody or the anti-PD-L1 antibody may be administered at a dose of 200 mg, and the

cycles of treatment may be 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and the cycles of treatment may be 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of 8 mg/kg, and (iii) the anti-PD-1 antibody or the anti-PD-L1 antibody may be administered at a dose of 200 mg, and the cycles of treatment may be 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of 6 mg/kg, and (iii) the anti-PD-1 antibody or the anti-PD-L1 antibody may be administered at a dose of 200 mg, and the cycles of treatment may be 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 150 mg/$m^2$, (ii) the anti-VEGFR-2 antibody may be administered at a dose of about 8 mg/kg, as a subsequent dose, on day 1 of a second cycle, and then at a dose of about 6 mg/kg, as a subsequent dose, on day 1 of (an) additional cycle(s), and (iii) the anti-PD-1 antibody or the anti-PD-L1 antibody may be administered at a dose of 200 mg, and the cycles of treatment may be 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and the cycles of treatment may be 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of 8 mg/kg, and (iii) the anti-PD-1 antibody or the anti-PD-L1 antibody may be administered at a dose of 200 mg, and the cycles of treatment may be 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of 6 mg/kg, and (iii) the anti-PD-1 antibody or the anti-PD-L1 antibody may be administered at a dose of 200 mg, and the cycles of treatment may be 3 weeks.

In some embodiments, the lung cancer may be a CEACAM5-positive cancer having a CEACAM5 immunohistochemical intensity ≥ 2+ in ≥ 50% of cancer cells, as measured by immunohistochemistry, and at day 1 of a first cycle of treatment, (i) the antibody-drug conjugate may be administered at a dose of 150 mg/$m^2$, (ii) the anti-VEGFR-2 antibody may be administered at a dose of 10 mg/kg, and (iii) the anti-PD-1 antibody or the anti-PD-L1 antibody may be administered at a dose of 200 mg, and at day 1 of an at least one subsequent cycle of treatment (i) the antibody-drug conjugate may be administered at a dose of 120 mg/$m^2$, (ii) the anti-VEGFR-2 antibody may be administered at a dose of about 8 mg/kg, as a subsequent dose, on day 1 of a second cycle, and then at a dose of about 6 mg/kg, as a subsequent dose, on day 1 of (an) additional cycle(s), and (iii) the anti-PD-1 antibody or the anti-PD-L1 antibody may be administered at a dose of 200 mg, and the cycles of treatment may be 3 weeks.

According to some embodiments, in a use or method for treating a lung cancer, such as a non-small cell lung cancer, such as non-squamous non-small-cell lung cancer (NSQ NSCLC), one cycle (first and additional cycles) may comprise the steps of:

i) administering an anti-VEGFR-2 antibody at a dose from about 6 to about 10 mg/kg, once in the cycle, for example at day one of the cycle;

ii) administering the antibody-drug conjugate comprising an anti-CEACAM5-antibody at a dose of from of about 120 or 150 mg/$m^2$, for example at about 150 mg/$m^2$, once in the cycle, for example at day one of the cycle; and

iii) administering an anti-PD-1 antibody or the anti-PD-L1 antibody may be administered at a dose of 200 mg, for example at day one of the cycle.

The administration may be carried out on day one of the cycle.

The cycle is about three weeks.

The antibody-drug conjugate may be administered after the anti-VEGFR-2 antibody.

The antibody-drug conjugate may be administered before anti-PD-1 antibody or the anti-PD-L1 antibody.

According to some embodiments, in a use or method for treating a lung cancer, such as a non-small cell lung cancer, such as non-squamous non-small-cell lung cancer (NSQ NSCLC), one cycle (first and additional cycles) may comprise the steps of:

i) administering an anti-VEGFR-2 antibody at a dose from about 10 mg/kg, once in the cycle, for example at day one of the cycle;

ii) administering the antibody-drug conjugate comprising an anti-CEACAM5-antibody at a dose of from of about 150 mg/$m^2$, once in the cycle, for example at day one of the cycle; and

iii) administering an anti-PD-1 antibody or the anti-PD-L1 antibody may be administered at a dose of 200 mg, for example at day one of the cycle.

According to some embodiments, in a use or method for treating a lung cancer, such as a non-small cell lung cancer, such as non-squamous non-small-cell lung cancer (NSQ NSCLC), one cycle (first and additional cycles) may comprise the steps of:

i) administering an anti-VEGFR-2 antibody at a dose from about 8 mg/kg, once in the cycle, for example at day one of the cycle;

ii) administering the antibody-drug conjugate comprising an anti-CEACAM5-antibody at a dose of from of about 150 mg/$m^2$, once in the cycle, for example at day one of the cycle; and

iii) administering an anti-PD-1 antibody or the anti-PD-L1 antibody may be administered at a dose of 200 mg, for example at day one of the cycle.

According to some embodiments, in a use or method for treating a lung cancer, such as a non-small cell lung cancer, such as non-squamous non-small-cell lung cancer (NSQ NSCLC), one cycle (first and additional cycles) may comprise the steps of:

i) administering an anti-VEGFR-2 antibody at a dose from about 8 mg/kg, once in the cycle, for example at day one of the cycle;

ii) administering the antibody-drug conjugate comprising an anti-CEACAM5-antibody at a dose of from of about 120 mg/m$^2$, once in the cycle, for example at day one of the cycle; and

iii) administering an anti-PD-1 antibody or the anti-PD-L1 antibody may be administered at a dose of 200 mg, for example at day one of the cycle.

According to some embodiments, in a use or method for treating a lung cancer, such as a non-small cell lung cancer, such as non-squamous non-small-cell lung cancer (NSQ NSCLC), a first cycle may comprise the steps of:

i) administering an anti-VEGFR-2 antibody at a dose from about 10 mg/kg, once in the cycle, for example at day one of the cycle;

ii) administering the antibody-drug conjugate comprising an anti-CEACAM5-antibody at a dose of from of about 150 mg/m$^2$, once in the cycle, for example at day one of the cycle; and

iii) administering an anti-PD-1 antibody or the anti-PD-L1 antibody may be administered at a dose of 200 mg, once in the cycle, for example at day one of the cycle.

In such embodiment, such cycle may be a first cycle.

According to some embodiments, in a use or method for treating a lung cancer, such as a non-small cell lung cancer, such as non-squamous non-small-cell lung cancer (NSQ NSCLC), an additional cycle may comprise the steps of:

i) administering an anti-VEGFR-2 antibody at a dose from about 6, 8 or 10 mg/kg, once in the cycle, for example at day one of the cycle;

ii) administering the antibody-drug conjugate comprising an anti-CEACAM5-antibody at a dose of from of about 120 or 150 mg/m$^2$, once in the cycle, for example at day one of the cycle; and

iii) administering an anti-PD-1 antibody or the anti-PD-L1 antibody may be administered at a dose of 200 mg, once in the cycle, for example at day one of the cycle.

According to some embodiments, in a use or method for treating a lung cancer, such as a non-small cell lung cancer, such as non-squamous non-small-cell lung cancer (NSQ NSCLC), an additional cycle may comprise the steps of:

i) administering an anti-VEGFR-2 antibody at a dose from about 10 mg/kg, once in the cycle, for example at day one of the cycle;

ii) administering the antibody-drug conjugate comprising an anti-CEACAM5-antibody at a dose of from of about 150 mg/m$^2$, once in the cycle, for example at day one of the cycle and

iii) administering an anti-PD-1 antibody or the anti-PD-L1 antibody may be administered at a dose of 200 mg, once in the cycle, for example at day one of the cycle.

According to some embodiments, in a use or method for treating a lung cancer, such as a non-small cell lung cancer, such as non-squamous non-small-cell lung cancer (NSQ NSCLC), an additional cycle may comprise the steps of:

i) administering an anti-VEGFR-2 antibody at a dose from about 10 mg/kg, once in the cycle, for example at day one of the cycle;

ii) administering the antibody-drug conjugate comprising an anti-CEACAM5-antibody at a dose of from of about 120 mg/m$^2$, once in the cycle, for example at day one of the cycle; and

iii) administering an anti-PD-1 antibody or the anti-PD-L1 antibody may be administered at a dose of 200 mg, once in the cycle, for example at day one of the cycle.

According to some embodiments, in a use or method for treating a lung cancer, such as a non-small cell lung cancer, such as non-squamous non-small-cell lung cancer (NSQ NSCLC), an additional cycle may comprise the steps of:

i) administering an anti-VEGFR-2 antibody at a dose from about 8 mg/kg, once in the cycle, for example at day one of the cycle;

ii) administering the antibody-drug conjugate comprising an anti-CEACAM5-antibody at a dose of from of about 120 $mg/m^2$, once in the cycle, for example at day one of the cycle; and

iii) administering an anti-PD-1 antibody or the anti-PD-L1 antibody may be administered at a dose of 200 mg, once in the cycle, for example at day one of the cycle.

According to some embodiments, in a use or method for treating a lung cancer, such as a non-small cell lung cancer, such as non-squamous non-small-cell lung cancer (NSQ NSCLC), an additional cycle may comprise the steps of:

ia) administering an anti-VEGFR-2 antibody at a dose from about 8 mg/kg, once in a second cycle, for example at day one of the second cycle, and

ib) administering an anti-VEGFR-2 antibody at a dose from about 6 mg/kg, once in subsequent cycle(s), for example at day one of the subsequent cycle(s);

ii) administering the antibody-drug conjugate comprising an anti-CEACAM5-antibody at a dose of from of about 150 $mg/m^2$, once in the cycle, for example at day one of the cycle; and

iii) administering an anti-PD-1 antibody or the anti-PD-L1 antibody may be administered at a dose of 200 mg, for example at day one of the cycle.

According to some embodiments, in a use or method for treating a lung cancer, such as a non-small cell lung cancer, such as non-squamous non-small-cell lung cancer (NSQ NSCLC), an additional cycle may comprise the steps of:

ia) administering an anti-VEGFR-2 antibody at a dose from about 8 mg/kg, once in a second cycle, for example at day one of the second cycle, and

ib) administering an anti-VEGFR-2 antibody at a dose from about 6 mg/kg, once in subsequent cycle(s), for example at day one of the subsequent cycle(s),

ii) administering the antibody-drug conjugate comprising an anti-CEACAM5-antibody at a dose of from of about 120 $mg/m^2$, once in the cycle, for example at day one of the cycle;
and

iii) administering an anti-PD-1 antibody or the anti-PD-L1 antibody may be administered at a dose of 200 mg, for example at day one of the cycle.

A cycle may be 3 weeks.
In some embodiments, the anti-VEGFR-2 antibody may be administered before the antibody-drug conjugate, and the antibody-drug conjugate may be administered before the anti-PD-1 antibody or the anti-PD-L1 antibody.
The antibody-drug conjugate may be tusamitamab ravtansine (huMAb2-3-SPDB-DM4).
In some embodiments, the anti-VEGFR-2 antibody may be ramucirumab.
In one embodiment, the anti-PD-1 antibody is pembrolizumab or sintilimab.
In one embodiment, the anti-PD-1 antibody is sintilimab.
In some embodiments, the anti-PD-1 antibody may be pembrolizumab.

## Pharmaceutical compositions or combinations

In some embodiments, the present disclosure relates to a pharmaceutical composition comprising (i) an antibody-drug conjugate as disclosed herein, (ii) an anti-VEGFR-2 antibody, (iii) an anti-PD-1 antibody or an anti-PD-L1 antibody, and a pharmaceutically acceptable excipient.
In some embodiments, (i) the antibody-drug conjugate, (ii) the anti-VEGFR2 antibody, and (iii) the anti-PD-1 antibody or anti-PD-L1 antibody may be formulated in a single pharmaceutical composition.
In some embodiments, (i) the antibody-drug conjugate, (ii) the anti-VEGFR2 antibody, and (iii) the anti-PD-1 antibody or anti-PD-L1 antibody may be formulated in the form of two separate pharmaceutical compositions, wherein

(a) one pharmaceutical composition may comprise the antibody-drug conjugate and the anti-VEGFR2 antibody and the other pharmaceutical composition may comprise the anti-PD-1 antibody or anti-PD-L1 antibody, or (b) one pharmaceutical composition may comprise the antibody-drug conjugate and the other pharmaceutical composition may comprise and the anti-VEGFR2 antibody the anti-PD-1 antibody or anti-PD-L1 antibody, or (c) one pharmaceutical composition may comprise the antibody-drug conjugate and the anti-PD-1 antibody or anti-PD-L1 antibody and the other pharmaceutical composition may comprise the anti-VEGFR2 antibody.

In some embodiments, (i) the antibody-drug conjugate, (ii) the anti-VEGFR2 antibody, and (iii) the anti-PD-1 antibody or anti-PD-L1 antibody may be formulated in the form of three separate pharmaceutical compositions, wherein (a) a first pharmaceutical composition may comprise the antibody-drug conjugate, (b) a second pharmaceutical composition may comprise the anti-VEGFR2 antibody, and (c) a third pharmaceutical composition may comprise the anti-PD-1 antibody or anti-PD-L1 antibody.

In some embodiments, the present disclosure relates to a kit comprising (i) a pharmaceutical composition comprising an antibody-drug conjugate as disclosed herein and a pharmaceutically acceptable excipient, (ii) a pharmaceutical composition comprising an anti-VEGFR2 antibody and a pharmaceutically acceptable excipient, and (iii) a pharmaceutical composition comprising an anti-PD-1 antibody or an anti-PD-L1 antibody and a pharmaceutically acceptable excipient.

In some embodiments, the present disclosure relates to a pharmaceutical composition as disclosed herein, or a kit as disclosed herein, for a use for treating cancer.

In the case of formulation of the antibody-drug conjugate, the anti-VEGFR-2 antibody, and the anti-PD-1 antibody or the anti-PD-L1 antibody in three separate pharmaceutical compositions, the three separate pharmaceutical compositions may be administered simultaneously, separately, or sequentially, to a subject in need thereof. In some embodiments, the three separate pharmaceutical compositions may be sequentially administered to a subject in need thereof.

In some embodiments, pharmaceutical compositions or combinations of the present disclosure are such that the antibody-drug conjugate comprising an anti-CEACAM5-antibody, the anti-VEGFR-2 antibody, and the anti-PD-1 antibody or the anti-PD-L1 antibody are administered according to a dose as above described.

In some embodiments, in the uses as disclosed herein, the administration of the antibody-drug conjugate comprising an anti-CEACAM5-antibody and/or the anti-VEGFR-2 antibody and/or the anti-PD-1 antibody or the anti-PD-L1 antibody may be carried out by parenteral route. A suitable parenteral route may be intravenous infusion.

The present disclosure further relates to a pharmaceutical composition comprising an antibody-drug conjugate comprising an anti-CEACAM5-antibody, and further comprising an anti-VEGFR-2 antibody and/or an anti-PD-1 antibody or the anti-PD-L1 antibody.

The present disclosure further relates to a pharmaceutical composition comprising an antibody-drug conjugate comprising an anti-CEACAM5-antibody, an anti-VEGFR-2 antibody and/or an anti-PD-1 antibody or the anti-PD-L1 antibody, and at least one pharmaceutically acceptable excipient.

A pharmaceutical composition may comprise the antibody-drug conjugate comprising an anti-CEACAM5-antibody, and ramucirumab and/or pembrolizumab, and a pharmaceutically acceptable excipient.

A pharmaceutical composition may comprise the antibody-drug conjugate comprising an anti-CEACAM5-antibody, and ramucirumab and/or sintilimab, and a pharmaceutically acceptable excipient.

A pharmaceutical composition may comprise tusamitamab ravtansine, and ramucirumab, and/or pembrolizumab, and a pharmaceutically acceptable excipient.

According to some embodiments, the antibody-drug conjugate comprising an anti-CEACAM5-antibody, the anti-VEGFR-2 antibody, and the anti-PD-1 antibody or the anti-PD-L1 antibody may be formulated in the form of three separate pharmaceutical compositions, wherein (i) one pharmaceutical composition comprises the antibody-drug conjugate comprising an anti-CEACAM5-antibody, (ii) one pharmaceutical composition comprises the anti-VEGFR-2 antibody, and (iii) one pharmaceutical composition comprises the anti-PD-1 antibody or the anti-PD-L1 antibody.

According to some embodiments, the antibody-drug conjugate comprising an anti-CEACAM5-antibody, the anti-VEGFR-2 antibody, and the anti-PD-1 antibody or the anti-PD-L1 antibody may be formulated in the form of three separate pharmaceutical compositions, wherein (i) one pharmaceutical composition comprises the antibody-drug conjugate comprising an anti-CEACAM5-antibody and at least one pharmaceutically acceptable excipient, (ii) one pharmaceutical composition comprises the anti-VEGFR-2 antibody and at least one pharmaceutically acceptable excipient, and (iii) one pharmaceutical composition comprises the anti-PD-1 antibody or the anti-PD-L1 antibody and at least one pharmaceutically acceptable excipient.

The present disclosure further relates to a kit comprising (i) a pharmaceutical composition comprising the antibody-drug conjugate comprising an anti-CEACAM5-antibody, (ii) a pharmaceutical composition comprising an anti-VEGFR-2 antibody, in separate or combined formulations, and (iii) a pharmaceutical composition comprises the anti-PD-1 antibody or the anti-PD-L1 antibody.

The present disclosure further relates to a kit comprising (i) one pharmaceutical composition comprises the antibody-drug conjugate comprising an anti-CEACAM5-antibody and at least one pharmaceutically acceptable excipient,

(ii) one pharmaceutical composition comprises the anti-VEGFR-2 antibody and at least one pharmaceutically acceptable excipient, and (iii) one pharmaceutical composition comprises the anti-PD-1 antibody or the anti-PD-L1 antibody and at least one pharmaceutically acceptable excipient, in separate or combined formulations.

**"Pharmaceutical excipient"** or **"pharmaceutically acceptable excipient"** refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

As used herein, "pharmaceutically-acceptable carriers or excipients" includes any and all solvents, dispersion media, coatings, antibacterial and antifungal agents, and the like that are physiologically compatible. Examples of suitable carriers, diluents and/or excipients include one or more of water, amino acids, saline, phosphate buffered saline, buffer phosphate, acetate, citrate, succinate; amino acids and derivates such as histidine, arginine, glycine, proline, glycylglycine; inorganic salts NaCl, calcium chloride; sugars or polyalcohols such as dextrose, glycerol, ethanol, sucrose, trehalose, mannitol; surfactants such as Polysorbate 80, polysorbate 20, poloxamer 188; and the like, as well as combination thereof. In many cases, it will be preferable to include isotonic agents, such as sugars, polyalcohols, or sodium chloride in the composition, and formulation may also contain an antioxidant such as tryptamine and a stabilizing agent such as Tween 20.

The form of the pharmaceutical compositions, the route of administration, the dosage and the regimen naturally depend upon the condition to be treated, the severity of the illness, the age, weight, and gender of the subject, etc.

The pharmaceutical compositions of the disclosure can be formulated for a topical, oral, parenteral, intranasal, intravenous, intramuscular, subcutaneous or intraocular administration and the like. In an embodiment, the pharmaceutical compositions and combinations of the disclosure are formulated for intravenous administration.

In particular, the pharmaceutical compositions contain vehicles or excipients, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical composition can be administrated through drug combination devices.

The doses used for the administration can be adapted as a function of various parameters, and in particular as a function of the mode of used, of the relevant pathology, or alternatively of the desired duration of treatment.

To prepare pharmaceutical compositions, an effective amount of antibody-drug conjugate comprising an anti-CEACAM5-antibody and of an anti-VEGFR-2 antibody may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and injectable with the appropriate device or system for delivery without degradation. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant. Dispersions can also be prepared in glycerol, liquid polyethylene glycols, and mixtures thereof and in oils. Under ordinary conditions of storage and use, these preparations contain a preservative to prevent the growth of microorganisms.

The antibody-drug conjugate comprising an anti-CEACAM5-antibody can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, glycine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the subsequent of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which

contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The antibody-drug conjugate comprising an anti-CEACAM5-antibody formulated for parenteral administration, such as intravenous or intramuscular injection, other pharmaceutically acceptable forms include, e.g., tablets or other solids for oral administration; time release capsules; and any other form currently used.

In certain embodiments, the use of liposomes and/or nanoparticles is contemplated for the introduction of polypeptides into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 μm) are generally designed using polymers able to be degraded in vivo. Biodegradable polyalkyl-cyanoacrylate nanoparticles, or biodegradable polylactide or polylactide co glycolide nanoparticules that meet these requirements are contemplated for use in the present disclosure, and such particles may be easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 μm. Sonication of MLVs results in the formation of small unilamellar vesicles (SUVs) with diameters in the range of 200 to 500 Å, containing an aqueous solution in the core. The physical characteristics of liposomes depend on pH, ionic strength and the presence of divalent cations.

## [EXAMPLES]

The following examples illustrate the embodiments of the disclosure that are presently best known. However, it is to be understood that the following are only exemplary or illustrative of the application of the principles of the present disclosure. Numerous modifications and alternative compositions, methods, and systems may be devised by those skilled in the art without departing from the spirit and scope of the present disclosure. Thus, while the present disclosure has been described above with particularity, the following examples provide further detail in connection with what are presently deemed to be the most practical and preferred embodiments of the disclosure.

### Example 1: Open-label, single-arm trial to evaluate tusamitamab ravtansine (SAR408701) used in combination with ramucirumab and pembrolizumab in metastatic, non-squamous, non-small-cell lung cancer (NSQ NSCLC) patients with CEACAM5-positive tumors

#### *Rational*

The aim of this study is to evaluate the safety, tolerability and antitumor activity (efficacy) of tusamitamab ravtansine in combination with ramucirumab and pembrolizumab in participants with CEACAM5-positive (CEACAM5 ≥50%) NSQ NSCLC tumors.

The combination of tusamitamab ravtansine with ramucirumab may represent a treatment regimen with an improved safety profile as compared to the combination of docetaxel with ramucirumab approved for the treatment of advanced NSCLC. Addition of pembrolizumab could be a new option for the treatment of advanced NSQ NSCLC after platinum and immune check-point inhibitor (ICI).

*Objectives and endpoints*

**Table 1 - Objectives and endpoints**

| **Triplet cohort**<br>**Objectives** | **Endpoints** |
|---|---|
| **Primary** | |
| To assess the tolerability and to confirm the recommended dose of tusamitamab ravtansine in combination with ramucirumab and pembrolizumab in the NSQ NSCLC population | Incidence of study drug-related dose-limiting toxicity (DLT) at Cycle 1 (C1D1 to C1D21). Anticipated DLT includes, but is not limited to, corneal toxicity. |
| **Secondary** | |
| To assess the safety and tolerability of tusamitamab ravtansine in combination with ramucirumab and pembrolizumab | Incidence of treatment emergent adverse events (TEAEs) and serious adverse events (SAEs) and laboratory abnormalities according to National Cancer Institute (NCI) Common Terminology Criteria for Adverse Events (CTCAE) V5.0 |
| To assess the antitumor activity of tusamitamab ravtansine in combination with ramucirumab and pembrolizumab in the NSQ NSCLC population | Objective response rate (ORR) defined as proportion of participants with confirmed complete response (CR) or partial response (PR) as best overall response (BOR) determined per Response Evaluation Criteria in Solid Tumors (RECIST) v1.1 |
| To assess the immunogenicity of tusamitamab ravtansine (SAR408701) when given in combination with ramucirumab and pembrolizumab | Incidence of anti-therapeutic antibodies (ATAs) against tusamitamab ravtansine (SAR408701) |
| **Tertiary/Exploratory** | |
| To explore circulating carcinoembryonic antigen (CEA) as a potential biomarker for activity and to evaluate circulating CEA levels at prescreening | Circulating CEA at prescreening, screening, and during the treatment period |
| Abbreviations: ATA=antitherapeutic antibody; BOR=best overall response; CR=complete response; DLT=dose-limiting toxicity; NCI CTCAE=National Cancer Institute Common Terminology Criteria for Adverse Events; NSQ NSCLC=non-squamous, non small-cell lung cancer; PK=pharmacokinetic; PR=partial response; RECIST=Response Evaluation Criteria in Solid Tumors. | |

*Study design*

This is a Phase 2, open-label, single-arm, 1 cohort, multi-center study assessing safety, efficacy (antitumor activity), and pharmacokinetic (PK) of the combination of tusamitamab ravtansine, ramucirumab, and pembrolizumab **(Triplet cohort)** in patients with metastatic, CEACAM5-positive (defined as CEACAM5 immunohistochemical [IHC] intensity ≥2+ in ≥50% of cells) NSQ NSCLC tumors, previously treated with platinum-based chemotherapy and an immune check-point inhibitor (ICI).

The prescreening phase will correspond to the period for collection of a participant's tumor sample to allow determination of CEACAM5 status by central IHC. Only participants with NSQ NSCLC determined to be CEACAM5-positive by central IHC will go through protocol screening procedures during the screening phase. Once the results for CEACAM5 available, only participants with positive results (defined as CEACAM5 expression of ≥2+ in intensity involving at least 50% of the tumor cell population), in archival tumor sample (or if not available fresh biopsy sample) will enter in screening phase.

During the screening phase, all inclusion/exclusion criteria will be checked to confirm the participants' eligibility for treatment part.

Once the participant is screened, the participant may be determined to be eligible for a treatment phase.

***The triplet cohort will have only 1 part***

Participants will receive ramucirumab 10 mg/kg, tusamitamab ravtansine followed by pembrolizumab 200 mg,

every 3 weeks to assess the tolerability of the combination to be used in future clinical studies. The first 3 participants will receive ramucirumab at 10 mg/kg, tusamitamab ravtansine 150 mg/$m^2$ followed by pembrolizumab 200 mg every 3 weeks (Q3W). Administration of tusamitamab ravtansine will be initiated at least 1 hour after the completion of ramucirumab infusion.

The starting dose for tusamitamab ravtansine is 150 mg/$m^2$ and was defined based on findings from first-in-human study TED13751. In this study, the RP2D for tusamitamab ravtansine given Q3W was 170 mg/$m^2$. Given that tusamitamab ravtansine exposure PK parameters at Cycle 1 of administration as a single agent of 170 mg/$m^2$ Q2W were significantly associated with the occurrence of Grade ≥2 corneal events, it is anticipated that 150 mg/$m^2$ may result in less ocular toxicity, and overall will be better tolerated than 170 mg/$m^2$. In addition, this dose corresponds to the same dose intensity as with the recommended 100 mg/$m^2$ Q2W dose.

A minimum delay of 1 week is required between the initial dose in the first participant treated in a DL cohort and dosing the next 2 participants treated at the same DL.

- If ≤1/3 participants treated at the starting dose experiences a DLT, 3 additional participants will be treated to confirm the tolerability of the combination.
  - If ≤1/6 participants treated at the starting dose experiences a DLT, the starting dose will be the RP2D.
  - If ≥2/6 participants treated at the starting dose experience DLTs, the dose will be de-escalated to DL -1.
- If ≥2/3 participants treated at the starting dose experience DLTs, the dose will be de-escalated to DL minus 1 (DL -1).
- If ≤1 of the first 3 participants treated at DL -1 experiences a DLT, 3 additional participants will be treated at this DL.
  - If ≤1/6 participants treated at DL -1 experiences a DLT, DL-1 will be the RP2D.
  - If ≥2/6 participants treated at DL -1 experience a DLT, an alternative dosage might be considered or the triplet cohort may be stopped.
- If ≥2/3 participants treated at DL -1 experience a DLT, an alternative dosage might be considered or the triplet cohort may be stopped.

Note: If ≥2/6 or ≥2/3 participants treated at DL -1 experience DLTs, an alternative dosage might be considered from a safety viewpoint by the Sponsor after consulting the Study Committee (SC).

Table 4 shows dose reduction in the case of DLTs occurring in 2 or more of the first 3 participants or of 6 participants in a DL triplet cohort. **Figure 2** shows the decision tree for doses to be administered.

**Table 2** - **Dose reduction for the triplet cohort**

| Dose level (DL) | Tusamitamab ravtansine | ramucirumab | pembrolizumab |
|---|---|---|---|
| Starting dose | 150 mg/$m^2$ Q3W | 10 mg/kg Q3W | 200 mg Q3W |
| Minus -1 (DL-1) | 120 mg/$m^2$ Q3W | 10 mg/kg Q3W | 200 mg Q3W |

DL=dose level; Q3W=every 3 weeks.

Infusion of tusamitamab ravtansine will be administered at least 1 hour after the end of ramucirumab infusion. For patients with a BSA >2.20 $m^2$, the tusamitamab ravtansine dose will be calculated based on a BSA of 2.20 $m^2$.

The study cut-off for the secondary objective response rate (ORR) endpoint analysis of triplet cohort corresponds to the date on which all evaluable treated patients have had at least 2 post baseline tumor assessments, experienced confirmed objective response, or have discontinued the study for any reason. This study cut-off will occur approximately 4.5 months after the date of the first IMP administration of the last participant: 3 months for 2 tumor assessments and 1.5 months if a confirmation of response is needed.

***Dose adiustment/dose delay***

Dose adjustment and/or cycle delay are permitted in case of adverse reaction. In case of toxicity, cycle delays and dose modifications should be implemented. Every effort will be made to administer the full dose regimen and maximize dose intensity.

In case a dose reduction is necessary, the study intervention will be administered as shown in **Table 3:**

**Table 3 - Dose modification for toxicity in triplet cohort**

| Drug name | Starting dose | 1st dose reduction | 2nd dose reduction |
|---|---|---|---|
| tusamitamab ravtansine | 150 mg/m2 Q3W | 120 mg/m2 Q3W | (not permitted) |
| ramucirumab | 10 mg/kg Q3W | 8 mg/kg Q3W | 6 mg/kg Q3W |
| pembrolizumab | 200 mg Q3W | (not permitted) | (not permitted) |

Any patient who requires a tusamitamab ravtansine or ramucirumab dose reduction will continue to receive a reduced dose until discontinuation from tusamitamab ravtansine/ramucirumab/pembrolizumab or discontinuation from the study.

***Number of participants***

In the triplet cohort, approximately 74 participants will be prescreened to achieve up to approximately 12 DLT evaluable participants.

***Intervention groups and duration***

The duration of the study for a participant will include:

- Screening period: up to 28 days.
- Treatment period: once successfully screened, enrolled participants may receive study intervention until disease progression, unacceptable AE, or the participant's or investigator's decision to stop the treatment. Each cycle of treatment will have a duration of 3 weeks in triplet cohort. After discontinuing study intervention, participants will return to the study site approximately 30 days after the last investigational medicinal product (IMP) administration or before the participant receives another anti-cancer therapy, whichever is earlier, for end-of-treatment assessments.
- Safety follow-up visit: will be performed approximately 90 days after the last dose of IMP. If any ongoing related AE/SAE is resolved or stabilized, no further follow-up visit is needed. The expected duration of study intervention for participants may vary, based on progression date; median expected duration of study per participant is estimated as 11 months (up to 1 month for screening, a median of 6 months for treatment, and a median of 4 months for end-of-treatment assessments and safety follow-up visit).

***Study interventions***

Study interventions include tusamitamab ravtansine, ramucirumab, and pembrolizumab. To prevent hypersensitivity reactions, each administration will be preceded by premedication. Each cycle of treatment will have a duration of 3 weeks in the triplet cohort.

Study drugs to be administered for triplet cohort are detailed in **Table 4.**

**Table 4 - Overview of study interventions administered - Triplet cohort**

| Enter Arm Name (single arm) | tusamitamab ravtansine | ramucirumab | pembrolizumab |
|---|---|---|---|
| Type | Drug/Biologic | Biologic | Biologic |
| Dose formulation | Concentrated solution for IV | Concentrated solution for IV | Concentrate for solution for infusion |
| Unit dose strength(s) | 5 mg/mL | 10 mg/mL | 25 mg/mL |
| Dosage level (s) | 150 (120) mg/$m^2$ every 3 weeks | 10 mg/kg every 3 weeks | 200 mg every 3 weeks |

(continued)

| Enter Arm Name (single arm) | tusamitamab ravtansine | ramucirumab | pembrolizumab |
|---|---|---|---|
| Route of administration | IV infusion | IV infusion | IV infusion |
| IMP (single-arm) | IMP | IMP | IMP |

| Enter Arm Name (single arm) | tusamitamab ravtansine | ramucirumab | pembrolizumab |
|---|---|---|---|
| Packaging and labeling | Supplied in a 30 mL glass vial with a plastic flip-off cap, containing 125 mg/25 mL tusamitamab ravtansine, and labelled with a multilingual booklet | Supplied in a single-dose vial (100 mg/10 mL or 500 mg/50 mL) individually packaged in a carton. | Supplied in single-dose vials containing 100 mg/4 mL pembrolizumab labelled with a multilingual booklet. 1 vial per treatment box |
| [Current/ Former name (s) or alias(es)] | None | Cyramza | Keytruda |

***Investigational medicinal products***

On Day 1 of each treatment cycle, the patient's BSA will be determined using the current weight and baseline height; dose may not be adjusted if body weight change is ≤5%.

*Ramucirumab*

Ramucirumab will be administered prior to administration of tusamitamab ravtansine.

- Formulation: CYRAMZA® (ramucirumab) is a concentrate for solution for infusion supplied in 10 mL or 50 mL single-use vial. Each vial contains either 100 mg ramucirumab in 10 mL (10 mg/mL) or 500 mg ramucirumab in 50 mL (10 mg/mL).
- Route of administration: intravenous (IV) infusion.
- Dose regimen:
  - In the triplet cohort, ramucirumab will be administered as a 10 mg/kg IV infusion over 1 hour on Day 1 of every 3-week cycle. Each administration will be preceded by premedication (as per the approved product label) to prevent hypersensitivity reaction.

*Tusamitamab ravtansine*

Tusamitamab ravtansine infusion will start ≥1 hour after the end of ramucirumab infusion.

- Formulation: Tusamitamab ravtansine is supplied as a 25 mL extractable volume of concentrate for solution for infusion of 125 mg contained in a 30 mL type I glass vial.
- Route of administration: IV infusion.
- Dose regimen:
  - In the triplet cohort, tusamitamab ravtansine 150 mg/$m^2$ (or 120 mg/$m^2$, if deemed the appropriate dose by the SC) will be administered via IV infusion over 1 hour, 30 minutes on Day 1, and then every 3 weeks.

- For patients with a body surface area (BSA) >2.20 $m^2$, the dose of tusamitamab ravtansine will be calculated based on a BSA of 2.20 $m^2$.

*Pembrolizumab*

In the triplet cohort, pembrolizumab will be administered after ramucirumab and tusamitamab ravtansine.

- Formulation: Keytruda® (pembrolizumab) is a concentrate for solution for infusion supplied in 100 mg/4 mL (25 mg/mL) solution in single-dose vials.
- Route of administration: intravenous (IV) infusion.
- Dose regimen: pembrolizumab will be administered as a 200 mg IV infusion over 30 minutes on Day 1 and then Q3W.

***Non-investigational medicinal products***

*Premedication:*

Both tusamitamab ravtansine and ramucirumab have potential risk of infusion-related allergic reaction; therefore, premedication with an IV histamine H1 antagonist (diphenhydramine 50 mg IV or equivalent, e.g., cetirizine, promethazine, dexchlorpheniramine, according to local approval and availability) given approximately at least 15 minutes before ramucirumab administration is required for all participants. If a participant previously experienced an infusion-related reaction (IRR) following a dose of ramucirumab or tusamitamab ravtansine, premedication for subsequent infusions will also include dexamethasone 10 mg IV and acetaminophen (paracetamol). All drugs used as premedication will be entered on the concomitant premedication page.

***Statistical considerations:***

***Sample size calculations:***

**Triplet cohort:** the cohort will have only 1 part: a safety run-in. 6 to 12 DLT-evaluable participants will be enrolled in order to confirm the RP2D.

Part 1 (safety run-in) aims to establish the recommended dose (RD) of tusamitamab ravtansine in combination with ramucirumab according to DLTs observed.

The triplet cohort (safety run-in only) is designed to assess the tolerability and to confirm the recommended dose of tusamitamab ravtansine in combination with ramucirumab and pembrolizumab in the NSQ NSCLC population.

***Main analysis populations***

All-treated population: All registered participants exposed to the study treatment, regardless of the amount of treatment administered. This population is the primary population for analysis of all efficacy parameters.

DLT-Evaluable population: For the triplet cohort: Participants who received 1 cycle with at least 80% of the intended dose for each IMP of the combination. Participants should have completed Cycle 1 unless they experienced a DLT before the end of Cycle 1.

ATA population: All treated participants with at least 1 post-baseline ATA result (negative, positive, or inconclusive).

**Table 5 - Populations for analyses**

| Population | Description |
|---|---|
| **Pre-screened** | All participants who signed the prescreening informed consent for CEACAM5 assay assessment of their biopsy. |
| **Screened** | All participants who signed informed consent for study participation |
| **All-treated** | All registered participants exposed to the study treatment, regardless of the amount of treatment administered. This population is the primary population for analysis of all efficacy parameters. |

(continued)

| Population | Description |
|---|---|
| **DLT-Evaluable** | For the triplet cohort: Participants who received 1 cycle with at least 80% of the intended dose for each IMP of the combination. Participants should have completed Cycle 1 unless they experienced a DLT before the end of Cycle 1. |
| **PK** | All participants from the all-treated population who have actually received at least 1 dose or a part of a dose of tasumitamab ravtansine (SAR408701) or ramucirumab with at least 1 evaluable post-baseline concentration. |
| **ATA** | All treated participants with at least 1 post-baseline ATA result (negative, positive, or inconclusive). |

***Statistical analyses***

*Efficacy analyses*

All efficacy analyses will be performed on the all-treated population (primary population for analysis of all efficacy parameters). All efficacy analyses will be summarized only for the subgroup of participants treated at the recommended dose (ie, excluding participants treated at the starting dose if this differs from the RP2D).

A summary of efficacy analyses is provided in **Table 6.**

**Table 6** - **Efficacy analyses**

| Endpoint | Statistical Analysis Methods |
|---|---|
| **Secondary for triplet cohort**<br>ORR | Descriptive statistics and 95% CIs using the Clopper-Pearson method |
| **Tertiary/exploratory**<br>Circulating CEA at prescreening, screening, and during the treatment period | |
| ORR=objective response rate. | |

*Analysis of secondary efficacy endpoints for the triplet cohort*

The ORR will be estimated by dividing the number of participants with confirmed objective response (CR or PR as BOR), derived according to RECIST v1.1, by the number of participants from the analysis population.

The BOR is the best tumor response observed from the date of the first administration of IMP until disease progression, death, or initiation of post-treatment anticancer therapy, whichever occurs first.

ORR will be summarized for the all-treated population with descriptive statistics. In addition, two-sided 95% CIs will be computed using the Clopper-Pearson method.

The DLTs observed during the DLT observation period (Cycle 1 and Cycle 2), will be summarized on the DLT-evaluable population, by dose level. In addition, AEs that meet the DLT criteria in subsequent cycles will be summarized on the all-treated population. Details will be provided by participant.

*Analysis of Primary endpoint for the triplet cohort*

The DLTs observed during the DLT observation period (Cycle 1), will be summarized on the DLT-evaluable population, by dose level. In addition, AEs that meet the DLT criteria in subsequent cycles will be summarized on the all-treated population. Details will be provided by participant.

*Analysis of safety endpoints*

All safety analyses will be performed on the all-treated population for the Part 1 (safety run-in part) for the triplet cohort, by dose level and overall.

Summary of Safety data will be performed by participant. For each safety parameter, a baseline value will be defined as the last value or measurement taken up to the first administration of the IMP.

A summary of safety analyses is provided in **Table 7.**

**Table 7 - Safety analyses**

| Endpoint | Statistical Analysis Methods |
|---|---|
| **Primary**<br>For the triplet cohort: Incidence of study drug-related DLTs at Cycle 1 | Descriptive statistics |
| **Secondary**<br>Incidence of TEAEs and SAEs and laboratory abnormalities according to NCI CTCAE V5.<br>Incidence of ATAs against tusamitamab ravtansine (SAR408701) | Descriptive statistics |
| **Exploratory** | No exploratory endpoint is defined for safety analyses |
| ATA=anti-therapeutic antibodies; TEAE=treatment emergent adverse event; SAE=serious adverse event; SAP=statistical analysis plan. | |

The observation period will be divided into 4 segments:

- The prescreening period is defined as the time from when the participants give prescreening informed consent or provide an addendum to informed consent (for participants who already have available CEACAM5 expression result) to the day before the screening informed consent.
- The screening period is defined as the time from when the participants give screening informed consent to the first administration of the IMP.
- The treatment period is defined as the time from the first administration of IMP up to 30 days after the last administration of IMP.
- The post-treatment period is defined as the time from the 31 st day after the last administration of IMP to study closure or death, whichever occurs first.

Number and percentage of participants experiencing treatment-emergent adverse event (TEAEs) by primary system organ class (SOC) and preferred term (PT) will be summarized by NCI-CTCAE V5.0 Grade (all grades, and Grade ≥3) for the all-treated population. Similar summaries will be prepared for treatment-related TEAEs, TEAEs leading to definitive discontinuation, TEAEs leading to dose modification, serious TEAEs, TEAEs with fatal outcome, adverse events of special interest (AESIs), and AEs/SAEs occurring during the post-treatment period. In addition, the number (%) of participants with any Grade 5 AE (TEAE and post-treatment) will be summarized.

Hematology and clinical chemistry results will be graded according to the NCI-CTCAE V5.0, when applicable. Number and percentage of participants with laboratory abnormalities (all grades and by grade) using the worst grade during the treatment period will be provided for the all-treated population.

*Analysis of primary safety endpoint*

Dose-limiting toxicities observed during the DLT observation period (Cycle 1 for the triplet cohort) will be summarized on the DLT-evaluable population, by dose level. In addition, AEs that meet the DLT criteria in subsequent cycles will be summarized on the all-treated population. Details will be provided by patient.

*Analysis of adverse events*

Adverse events will be collected from the time prescreening informed consent is signed until at least 30 days after the last infusion of the study treatment. All AEs will be categorized according to NCI-CTCAE V5.0 and classified by system organ class (SOC)/ preferred term (PT) according to the latest available version of the MedDRA dictionary.

- Prescreening AEs are defined as AEs occurring during the prescreening period.
- Screening AEs are defined as any AEs occurring during the screening period.

- Treatment-emergent AEs are defined as AEs that develop, worsen (according to the Investigator's opinion), or become serious during the treatment period.

- Post-treatment AEs are defined as AEs that are reported during the post-treatment period.

The NCI-CTCAE grade will be taken into account in the summary. For participants with multiple occurrences of the same PT, the maximum grade will be used.

The primary focus of AE reporting will be on TEAEs. Pre-screening, screening and post-treatment AEs will be described separately.

Treatment-emergent adverse events:

An overall summary of TEAEs will be provided. The number and percentage of participants experiencing any of the following will be provided:

- TEAEs
- Grade ≥3 TEAEs
- Grade 5 TEAEs (any TEAE with a fatal outcome during the treatment period)
- Serious TEAEs
- TEAEs leading to definitive treatment discontinuation
- Treatment-related TEAEs
- Treatment-related TEAEs Grade ≥3
- Serious treatment-related TEAEs
- AESI

Number and percentage of participants experiencing TEAEs by primary SOC and PT will be summarized by NCI-CTCAE V5.0 grade (all grades and Grade ≥3) for the all-treated population.

Similar summaries will be prepared for treatment-related TEAEs, TEAEs leading to definitive discontinuation, TEAEs leading to dose modification, serious TEAEs, TEAEs with fatal outcome, AESIs, and AEs/SAEs occurring during the post-treatment period. In addition, the number (%) of participants with any Grade 5 AE (TEAE and post-treatment) will be summarized.

*Analysis of clinical laboratory evaluations*

Clinical laboratory values will be analyzed after conversion into standard international units. International units will be used in all listings and tables.

Hematology and clinical chemistry results will be graded according to the NCI-CTCAE V5.0, when applicable. Number and percentage of participants with laboratory abnormalities (all grades and by grade) using the worst grade during the treatment period will be provided for the all-treated population.

When the NCI-CTCAE V5.0 grading scale is not applicable, the number of participants with laboratory abnormality out-of-normal laboratory range value will be displayed.

*Analysis of immunogenicity*

Immunogenicity analyses and the potential impact on PK, safety and efficacy will be described in the SAP, and will be performed on the ATA population.

*Other analyses*

Analyses of PK and pharmacodynamics and exploratory biomarker analyses will be described in the statistical analysis plan, to be finalized before database lock.

The population PK methodology will be described in a separate report provided by the Pharmacokinetics,

Dynamics, and Metabolism (PKDM) Modeling and Simulation group.

***Study population***

***Inclusion criteria***

Participants are eligible to be included in the study only if all the following criteria apply:

*Age*

Participants must be ≥18 years of age (or country's legal age of majority, if >18 years), at the time of signing the informed consent.

*Type of participant and disease characteristics*

Histologically or cytologically proven diagnosis of NSQ NSCLC.
Metastatic disease progression fulfilling both of the following 2 criteria:

a) Having progressive disease during or after platinum-based chemotherapy (at least 2 cycles). Maintenance therapy following platinum-based chemotherapy is not considered as a separate regimen. Adjuvant/neoadjuvant treatment for a patient who had a relapse with metastatic disease during or within 6 months of completing treatment will be considered as first-line treatment.
AND
b) Having progressive disease during or after 1 immune checkpoint inhibitor (anti-PD1/PD-L1); this could be given as monotherapy or in combination with platinum-based chemotherapy (whatever the order).

For a tumor genotype with a sensitizing EGFR mutation or BRAF mutation or ALK/ROS alteration, demonstrated disease progression while receiving approved treatment for that genotype in addition to platinum-based chemotherapy and immune checkpoint inhibitor.
Expression of CEACAM5 as demonstrated prospectively by a centrally assessed immunohistochemical (IHC) assay of ≥2+ in intensity involving at least 50% of the tumor cell population in archival tumor sample (or if not available fresh biopsy sample). At least 5 slides of formalin-fixed, paraffin embedded (FFPE) tumor tissue sectioned at a thickness of 4 μm are required. If less material is available, the patient could still be considered eligible after discussion with the Sponsor, who may assess and confirm that the available material is sufficient for key evaluations.
At least one measurable lesion by RECIST v1.1 as determined by local site Investigator radiology assessment. An irradiated lesion can be considered measurable only if progression has been demonstrated on the irradiated lesion.
Eastern Cooperative Oncology Group (ECOG) performance status 0-1.

***Exclusion criteria***

Participants are excluded from the study if any of the following criteria applies:

*Medical conditions*

Untreated brain metastases and history of leptomeningeal disease. Patients with previously treated brain metastases may participate provided they are stable (ie, without evidence of progression by imaging for at least 4 weeks prior to the first administration of study intervention, and any neurologic symptoms have returned to baseline); there is no evidence of new or enlarging brain metastases; and the patient does not require any systemic corticosteroids to manage brain metastases within 3 weeks prior to the first dose of study intervention.
Significant concomitant illness, including any severe medical condition that, in the opinion of the investigator or Sponsor, would impair the patient's participation in the study or interpretation of the results.
History within the last 3 years of an invasive malignancy other than that treated in this study, with the exception of resected/ablated basal or squamous-cell carcinoma of the skin or carcinoma in situ of the cervix, or other local tumors considered cured by local treatment.
History of known acquired immunodeficiency syndrome (AIDS)-related illnesses or known human immunodeficiency virus (HIV) disease requiring antiretroviral treatment; or active infection with hepatitis A, B (defined as either positive HBs antigen or positive hepatitis B viral DNA test above the lower limit of detection of the assay), or C (defined as known positive result for antibodies to hepatitis C and known quantitative hepatitis C virus [HCV] RNA results greater

than the lower limit of detection of the assay).

Non-resolution of any prior treatment-related toxicity to < Grade 2 according to NCI CTCAE V5.0, with the exception of alopecia, vitiligo, or active thyroiditis controlled with hormone replacement therapy.

Unresolved corneal disorder or any previous corneal disorder considered by an ophthalmologist to predict higher risk of drug-induced keratopathy. The use of contact lenses is not permitted. Patients using contact lenses who are not willing to stop wearing them for the duration of the study intervention are excluded.

Radiographic evidence of major airway or blood vessel invasion or intratumor cavitation, regardless of tumor histology.

History of uncontrolled hereditary or acquired arterial thrombotic disorder or history of aneurism.

Major surgery within 28 days prior to Day 1/first IMP infusion, or subcutaneous venous access device placement within 7 days prior to Day1. Postoperative bleeding complications or wound complications from a surgical procedure performed in the last 2 months.

History of gross hemoptysis (defined as bright red blood or ≥1/2 teaspoon or ≥2.5 mL) within 2 months before the first administration of study intervention.

Clinically relevant congestive heart failure (CHF; NYHA II-IV, or LVEF less than 50%) or symptomatic or poorly controlled cardiac arrhythmia.

Any arterial thrombotic event, including myocardial infarction, unstable angina, cerebrovascular accident, or transient ischemic attack, within 6 months before the first administration of study intervention.

Uncontrolled arterial hypertension (systolic ≥150 mmHg or diastolic ≥90 mmHg) despite standard medical management. A participant with systolic pressure >150 mmHg or diastolic pressure >90 mmHg is ineligible for the study.

Serious or nonhealing wound, skin ulcer, or bone fracture within 28 days before the first administration of study intervention.

Gastrointestinal (GI) perforation and/or fistulae within 6 months prior to first administration of study intervention.

Significant bleeding disorders, vasculitis, or Grade 3-4 gastrointestinal (GI) bleeding within 3 months before the first administration of study intervention.

Bowel obstruction, history or presence of inflammatory enteropathy or extensive intestinal resection Crohn's disease, ulcerative colitis, or chronic diarrhea.

Medical condition requiring concomitant administration of a medication with a narrow therapeutic window and metabolized by CYP 450 (See Appendix 9, Section 10.9); and for which a dose reduction cannot be considered.

Medical conditions requiring concomitant administration of a strong CYP3A inhibitor (see Appendix 10; Section 10.10), unless it can be discontinued at least 2 weeks before first administration of study intervention.

*Prior/concomitant therapy*

Concurrent treatment with any other anticancer therapy.

More than 1 line previous chemotherapy in metastatic setting.

Prior treatment with ramucirumab or docetaxel.

Prior therapy targeting CEACAM5.

Prior maytansinoid treatment (DM1 or DM4 antibody-drug conjugate).

Washout period before the first administration of study intervention of less than 3 weeks or less than 5 times the half-life, whichever is shorter, for prior antitumor therapy (chemotherapy, targeted agents, immunotherapy and radiotherapy, or any investigational treatment).

Contraindication to use of corticosteroid premedication.

Current therapeutic anticoagulation with warfarin, low-molecular-weight heparin, or similar agents. Patients receiving prophylactic, low-dose anticoagulation therapy are eligible provided that the coagulation parameters defined in the inclusion criteria (INR ≤1.5 or PT ≤1.5 × ULN, and PTT/aPTT ≤1.5 × ULN) are met.

*Diagnostic assessments*

Poor organ function as defined by any one of the following prior to IMP administration:

a) Serum creatinine >1.5 × upper limit of normal (ULN) or 1.0 to 1.5 × ULN with estimated glomerular filtration rate (eGFR) <60 mL/min/1.73 m 2 as estimated using a Modification of Diet in Renal Disease (MDRD) formula.

b) Total bilirubin >1.0 × ULN.
aspartate aminotransferase (AST), alanine aminotransferase (ALT) >2.5 × ULN or AST, ALT >5 × ULN in case of documented liver metastasis or AST, ALT >1.5 × ULN concomitant with alkaline phosphatase (ALP) >2.5 × ULN.
ALP >5 × ULN with normal ALT/AST, for patients with bone metastases.

c) Neutrophils <1.5 × 10 9 /L or platelet count <100 × 10 9 /L or hemoglobin <9 g/dL (blood infusion-free for at least 2 weeks).

Urine dipstick or routine analysis indicating proteinuria of 2+ or higher, unless a 24 hour urine collection demonstrates <1000 mg of protein.

*Triplet cohort exclusions*

History of active autoimmune disease that has required systemic treatment in the past 2 years.
History of allogeneic tissue/solid organ transplantation.
Active infection requiring IV systemic therapy within 2 weeks prior to first study intervention administration or active tuberculosis.
Interstitial lung disease or history of pneumonitis that has required oral or IV steroids.
Symptomatic herpes zoster within 3 months prior to screening.
Significant allergies to humanized monoclonal antibodies.
Any radiation therapy to lung >30 Gy within 6 months of first study intervention administration.
Has received or will receive a live vaccine within 30 days prior to the first study intervention administration.
Thyroid-stimulating hormone (TSH) out of normal limits. If TSH is not within normal limits at baseline, the subject may still be eligible if T3 and free T4 are within the normal limits.

***Efficacy assessments***

**For the triplet cohort:**

Tumor assessment will be made every 6 weeks (±7 days window), and a scheduled assessment time point will not be modified in case of a cycle delay.
Thoracic-abdominal-pelvic CT-scan or MRI and any other examinations as clinically indicated will be performed to assess disease status at baseline; then every 6 weeks during the study treatment period until IMP discontinuation; and at the end of study treatment, except if already done at last cycle.
Confirmatory radiological evaluation will be performed at least 4 weeks after initial documentation of response.
After IMP discontinuation, tumor assessment should be performed at EOT for patients without imaging performed within past 4 weeks.
Brain CT-scan or MRI should be performed at baseline and followed only for patients with brain lesions at baseline. Imaging assessments during the on-treatment period are to be scheduled using the Cycle 1, Day 1 date as the reference date for all time points and are not to be scheduled based on the date of the previous imaging time point. Delay of an imaging assessment to conform to treatment delay is not permitted. The same tumor assessment technique must be used throughout the study for a given lesion/participant.

***Safety assessments***

***Physical examinations***

A complete physical examination will include, at a minimum, temperature, blood pressure, heart rate, and assessments of the major body systems, including cardiovascular and central nervous systems. Weight will also be measured and recorded before premedication and IMP administration at all treatment visits, and at the end of treatment and follow-up visits. Height will be recorded only at Screening.
ECOG performance status should be assessed before each IMP administration and at the follow-up visit.
Investigators should pay special attention to clinical signs related to previous serious illnesses.
Any new finding or worsening of previous finding should be reported as a new AE.

***Specific ocular tests***

Specific complete ocular tests at baseline will include: assessment of ocular/visual symptoms and ocular exams including visual acuity, slit lamp under dilatation, and Schirmer's test.
Standard specific ocular tests include:

- Assessment of ocular/visual symptoms, (ie, blurred vision, photophobia, dry eye, etc) at each visit before each study intervention. Start and end dates of symptoms will be collected.

- Visual acuity at screening and whenever clinically indicated.
- Slit lamp under dilatation at screening and whenever clinically indicated.
- Schirmer's test at screening and whenever clinically indicated.

In participants with any ocular/visual symptom (eg, blurred vision, photophobia), complete ocular tests will be repeated at the time of the occurrence of the ocular toxicity, if any regardless of the grade. Thereafter, visual acuity, slit lamp examination under dilatation, and Schirmer's test will be repeated once weekly (if not recommended to have less frequent assessment by ophthalmologist based on lesion characteristics) until resolution to Grade 1. In case of recurrent ocular toxicity observed in subsequent cycles, visual acuity and slit lamp examination under dilatation, and Schirmer's test will be performed at the time of the event onset, then weekly until resolution to Grade 1.

***Vital signs***

- Temperature, blood pressure, heart rate will be assessed during each physical examination.
- For blood pressure assessments, manual techniques will be used only if an automated device is not available.
- Blood pressure and pulse measurements should be preceded by at least 5 minutes of rest for the participant in a quiet setting without distractions (eg, television, cell phones).

***Cardiac assessment***

*Electrocardiograms*

- A single 12-lead ECG is required at baseline screening; before starting and after completing the first IMP administration (within 30 minutes after the end of the infusion); before IMP administration at each cycle; and at the end of treatment evaluation (between Day 22 and Day 30 after the last IMP administration). This test can be performed on the same day before the study intervention administration, or on the day before. An ECG is to be repeated as clinically indicated.
- A single 12-lead ECG will be obtained using an ECG machine that automatically calculates the heart rate and measures PR, QRS, and QT intervals.
- ECGs will be interpreted by a qualified physician at the site as soon after the time of ECG collection as possible, and ideally while the patient is still present, should additional ECGs be performed or for immediate patient management should any clinically relevant findings be identified.

*Echocardiogram or MUGA scan*

An echocardiogram or multigated acquisition (MUGA) scan to evaluate left ventricular ejection fraction (LVEF) will be evaluated during screening period, and whenever clinically indicated.

***Clinical safety laboratory assessments***

In general, these tests will be done at each cycle; during the first 2 cycles, hematology and liver function tests will be assessed weekly.

If Grade 4 neutropenia occurs, assess ANC every 2 to 3 days until ANC $\geq 0.5 \times 10\ 9$ /L.

In case of Grade ≥3 abnormal liver function tests, additional tests will be done every 2 to 3 days until recovery to the baseline value. Additional tests will be performed when clinically appropriate. This test can be performed before the study intervention administration on the same day or the day before.

The Investigator must review the laboratory reports and must document this review in the source documents.

***Pharmacokinetics***

Blood samples will be collected for the measurement of tusamitamab ravtansine and ramucirumab concentrations.

***Non-compartmental analysis***

Pharmacokinetic parameters of tusamitamab ravtansine will be calculated using non-compartmental methods from concentrations assayed in Part 1 (safety run-in). The parameters will include, but may not be limited to, those listed in **Table 8:**

**Table 8 - List of pharmacokinetic parameters and definitions**

| Parameters | Definition |
|---|---|
| $C_{eoi}$ | Concentration observed at the end of IV infusion |
| $C_{max}$ | Maximum concentration observed after infusion |
| $t_{max}$ | Time to reach C max |
| $C_{last}$ | Last concentration observed above the lower limit of quantification after infusion |
| $t_{last}$ | Time of C last |
| $C_{trough}$ | Concentration observed just before treatment administration during repeated dosing |
| $AUC_{0-14d}$ | Area under the plasma concentration versus time curve calculated using the trapezoidal method from time 0 to 14 days. |

***Population approach***

Data from plasma concentrations of tusamitamab ravtansine will be used for population PK analysis by non-linear mixed-effects modeling. Empirical Bayesian estimation of individual exposure parameters such as maximum concentration (C max ), trough concentration (C trough ) and area under the curve (AUC) will be derived. The population PK analysis will be reported in a standalone report (s).

***Pharmacodynamics***

Approximately 3 mL venous blood samples for measurement of circulating CEA will be collected at prescreening, at baseline, and during the treatment period as close as possible to tumor assessments.

For CEA sample collection scheduled on the day of a treatment visit, samples should be drawn prior to initiation of ramucirumab infusion and tusamitamab ravtansine infusion.

***Biomarkers***

Collection of samples for other biomarker research is also part of this study. The following samples for biomarker research are required and will be collected from all participants in this study:

- Tumor tissue samples will be collected and assayed for CEACAM5 expression to determine eligibility for this study.
- Blood samples will be collected for IgG measurement to explore the impact of IgG level on PK of tusamitamab ravtansine.

The level of IgG in blood drawn pre-infusion on Day 1 of Cycle 1 will be determined by a central laboratory. For this test, 2 mL of blood, corresponding to 1 mL of serum, will be collected. Instructions for the collection and handling of biological samples will be provided by the Sponsor in a separate laboratory manual. Samples may be stored for a maximum of 15 years (or according to local regulations) following the last participant's last visit for the study at a facility selected by the Sponsor to enable further analysis of biomarker responses to tusamitamab ravtansine.

***Immunogenicity assessments***

Blood samples will be collected for assessing the presence of ATA against tusamitamab ravtansine in plasma

from all participants

Plasma samples will be screened for antibodies binding to tusamitamab ravtansine and the titer of confirmed positive samples will be reported.

## Claims

1. A combination of (i) an antibody-drug conjugate (ADC) comprising an anti-CEACAM5 antibody, (ii) an anti-VEGFR2 antibody, and (iii) an anti-PD-1 antibody or anti-PD-L1 antibody, for use in the treatment of a cancer.

2. The combination for use according to claim 1, wherein the anti-CEACAM5 antibody comprises a CDR-H1 having the amino acid sequence of SEQ ID NO: 1, a CDR-H2 having the amino acid sequence of SEQ ID NO: 2, a CDR-H3 having the amino acid sequence of SEQ ID NO: 3, a CDR-L1 having the amino acid sequence of SEQ ID NO: 4, a CDR-L2 having the amino acid sequence NTR, and a CDR-L3 having the amino acid sequence of SEQ ID NO: 5.

3. The combination for use according to claim 1 or 2, wherein the anti-CEACAM5 antibody is tusamitamab.

4. The antibody-drug conjugate for the use of any of claims 1 to 3, wherein the antibody-drug conjugate comprises at least one chemotherapeutic agent.

5. The combination for use according to claim 4, wherein the chemotherapeutic agent is a maytansinoid selected from N2'-deacetyl-N2'-(3-mercapto-1-oxopropyl)-maytansine (DM1), N2'-deacetyl-N2'-(4-methyl-4-mercapto-1-oxopentyl)-maytansine (DM4), and combinations thereof.

6. The combination for use according to claim 4 or 5, wherein the anti-CEACAM5 antibody is covalently attached via non-cleavable linker to the at least one chemotherapeutic agent, the linker being selected from N-succinimidyl pyridyldithiobutyrate (SPDB), 4-(pyridin-2-yldisulfanyl)-2-sulfo-butyric acid (sulfo-SPDB), and succinimidyl(N-maleimidomethyl) cyclohexane-1-carboxylate (SMCC).

7. The combination for use according to any one of claims 1 to 6, wherein the antibody-drug conjugate is tusamitamab ravtansine.

8. The combination for use according to any one of claims 1 to 7, wherein the cancer is a CEACAM5 positive cancer having a CEACAM5 immunohistochemical intensity ≥ 2+ in ≥ 1% and in < 50% of cancer cells or is a CEACAM5 positive cancer having a CEACAM5 immunohistochemical intensity ≥ 2+ in ≥ 50% of cancer cells.

9. The combination for use according to any one of claims 1 to 8, wherein the cancer is selected from hepatocellular carcinoma, colorectal cancer, gastric cancer, gastroesophageal junction adenocarcinoma (GEJ), esophageal cancer, lung cancer, uterine cervix cancer, pancreatic cancer, ovarian cancer, thyroid cancer, bladder cancer, endometrial cancer, breast cancer, liver cancer, biliary tract cancer, prostate cancer, neuroendocrine cancer, and skin cancer.

10. The combination for use according to claim 9, wherein the lung cancer is a non-squamous non-small cell lung cancer (NSQ NSCLC).

11. The combination for use according to claim 10, wherein the non-squamous non-small cell lung cancer has no epidermal growth factor receptor (EGFR) sensitizing mutation or v-raf murine sarcoma viral oncogene homolog B1 (BRAF) mutation or anaplastic lymphoma kinase/c-ros oncogene 1 (ALK/ROS) alterations.

12. The combination for use according to any one of claims 1 to 11, wherein the anti-VEGFR-2 antibody is administered before the antibody-drug conjugate.

13. The combination for use according to any one of claims 1 to 12, wherein the antibody-drug conjugate is administered before the anti-PD-1 antibody or anti-PD-L1 antibody.

14. The combination for use according to any one of claims 1 to 13, wherein (i) the antibody-drug conjugate, (ii) the anti-VEGFR2 antibody, and (iii) the anti-PD-1 antibody or anti-PD-L1 antibody are administered at day 1 of a first and at least one additional cycles of treatment.

**15.** The combination for use according to claim 14, wherein a cycle of treatment is 3 weeks.

**16.** The combination for use according to any one of claims 1 to 15, wherein the antibody-drug conjugate is administered at a dose of 80 mg/$m^2$ to 170 mg/$m^2$, or at a dose of 80 mg/$m^2$ to 150 mg/$m^2$, or at a dose of 100 mg/$m^2$ to 120 mg/$m^2$, or at a dose of 150 mg/$m^2$ or at a dose of 120 mg/$m^2$.

**17.** The combination for use according to any one of claims 1 to 16, wherein the anti-VEGFR2 antibody is administered at a dose of 6 mg/kg to 10 mg/kg, or at a dose of 8 mg/k to 10 mg/kg, or at a dose of 6 mg/kg, or at a dose of 8 mg/kg, or at a dose of 10 mg/kg.

**18.** The combination for use according to any one of claims 1 to 17, wherein the anti-PD-1 antibody or the anti-PD-L1 antibody is administered at a dose of 150 mg to 400 mg, or at a dose of 150 mg to 300 mg, or at a dose of 200 mg.

**19.** The combination for use according to anyone of claims 1 to 18, wherein at day 1 of a first cycle of treatment and at day 1 of an at least one additional cycle of treatment, (i) the antibody-drug conjugate is administered at a dose of 150 mg/$m^2$, (ii) the anti-VEGFR-2 antibody is administered at a dose of 10 mg/kg, and (iii) the anti-PD-1 antibody or the anti-PD-L1 antibody is administered at a dose of 200 mg, and the cycle of treatment is 3 weeks.

**20.** The combination for use according to any one of claims 1 to 19, wherein the anti-VEGFR-2 antibody is ramucirumab.

**21.** The combination for use according to any one of claims 1 to 20, wherein the anti-PD-1 antibody is selected from the group consisting of pembrolizumab, nivolumab, cemiplimab, sintilimab, dostarlimab, and tislelizumab.

**22.** The combination for use according to claim 21, wherein the anti-PD-1 antibody is pembrolizumab or sintilimab.

**23.** A pharmaceutical composition comprising (i) an antibody-drug conjugate of anyone of claims 1 to 7, (ii) an anti-VEGFR-2 antibody, (iii) an anti-PD-1 antibody or an anti-PD-L1 antibody, and a pharmaceutically acceptable excipient.

**24.** A kit comprising (i) a pharmaceutical composition comprising an antibody-drug conjugate of anyone of claims 1 to 7 and a pharmaceutically acceptable excipient, (ii) a pharmaceutical composition comprising an anti-VEGFR2 antibody and a pharmaceutically acceptable excipient, and (iii) a pharmaceutical composition comprising an anti-PD-1 antibody or an anti-PD-L1 antibody and a pharmaceutically acceptable excipient.

**25.** The pharmaceutical composition according to anyone of claims 23 to 24, or the kit according to claim 68, for a use for treating a cancer.

**26.** Use of a combination of (i) an antibody-drug conjugate of anyone of claims 1 to 7, (ii) an anti-VEGFR2 antibody, and (iii) an anti-PD-1 antibody or anti-PD-L1 antibody, for the manufacture of a pharmaceutical composition or a kit, for the treatment of a cancer.

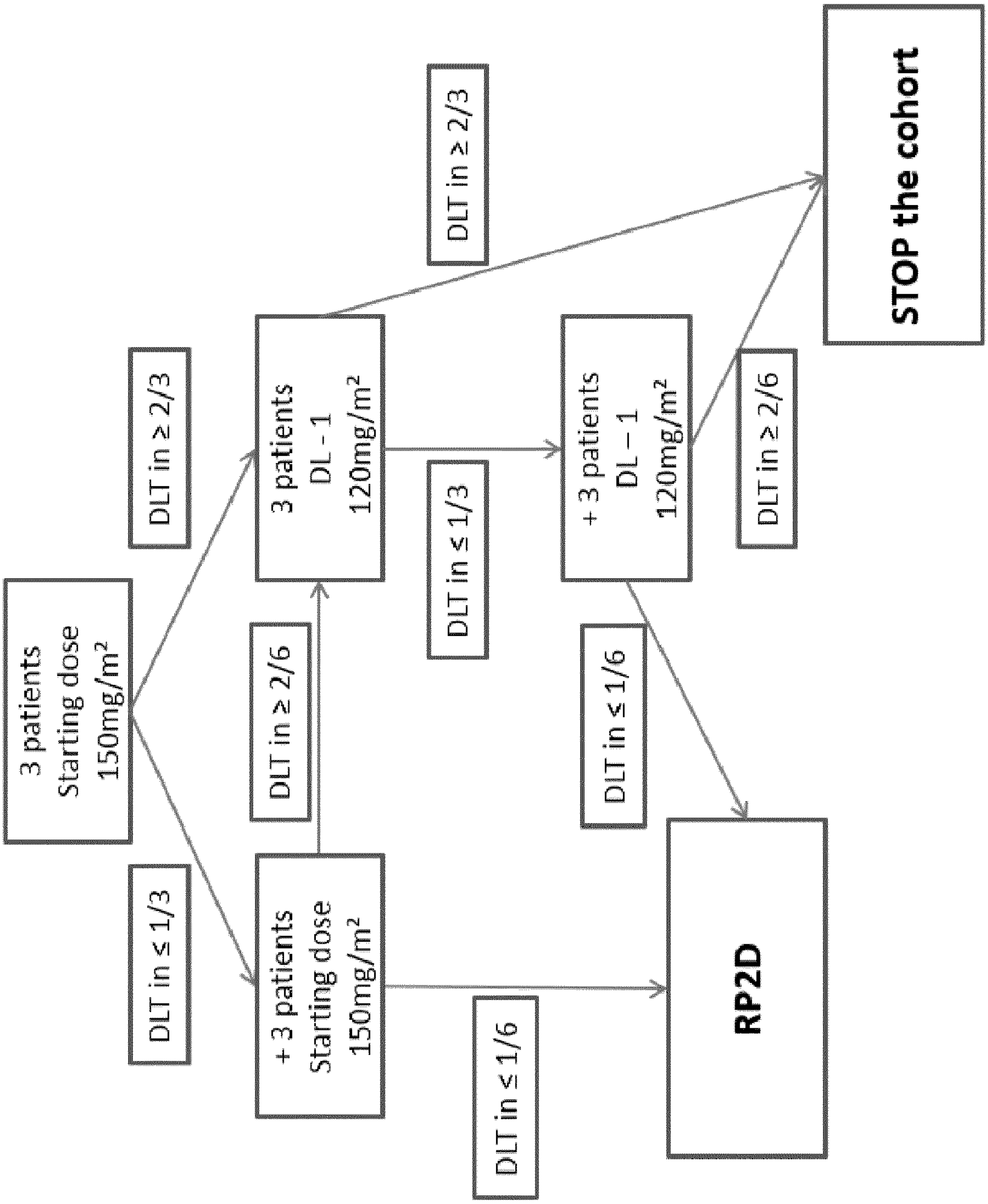
3 patients
Starting dose
150mg/m$^2$
DLT in ≥ 2/3
DLT in ≤ 1/3
3 patients
DL - 1
120mg/m$^2$
+ 3 patients
Starting dose
150mg/m$^2$
DLT in ≥ 2/6
DLT in ≤ 1/3
DLT in ≤ 1/6
DLT in ≥ 2/3
+ 3 patients
DL – 1
120mg/m$^2$
DLT in ≤ 1/6
DLT in ≥ 2/6
RP2D
STOP the cohort

FIGURE 1

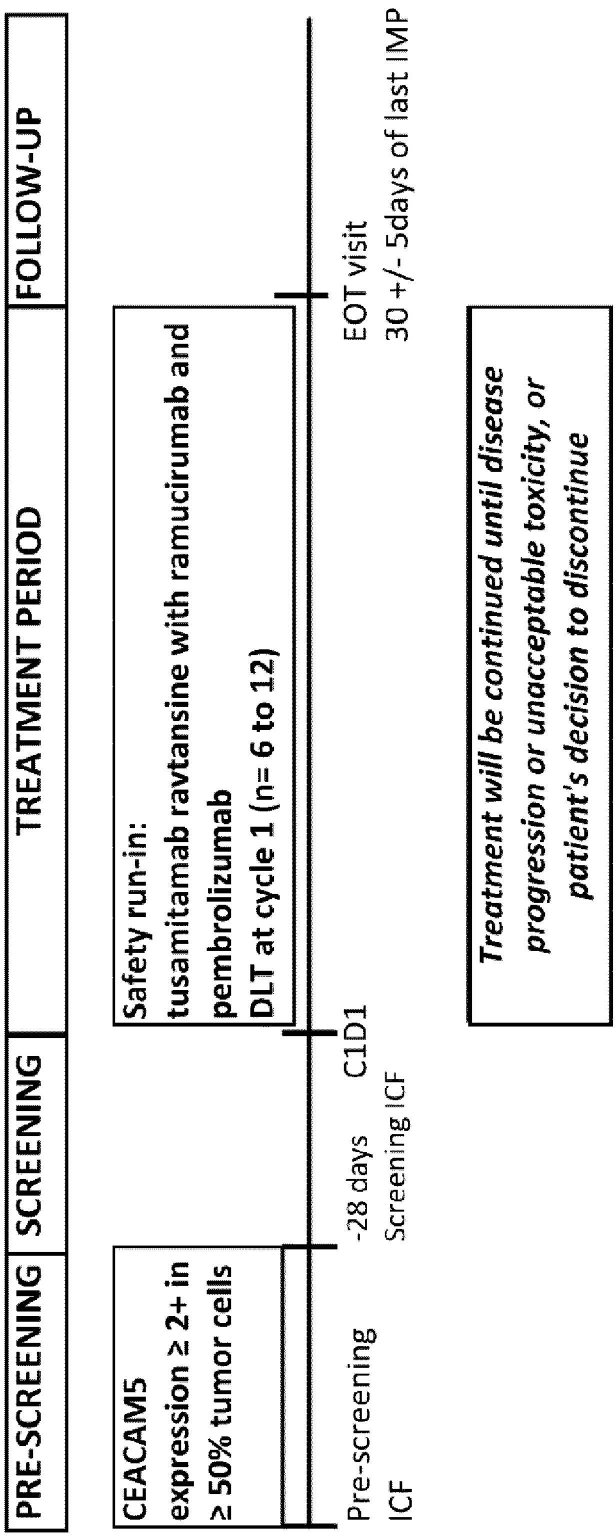
PRE-SCREENING
SCREENING
TREATMENT PERIOD
FOLLOW-UP
CEACAM5 expression ≥ 2+ in ≥ 50% tumor cells
Safety run-in:
tusamitamab ravtansine with ramucirumab and pembrolizumab
DLT at cycle 1 (n= 6 to 12)
Pre-screening ICF
-28 days
Screening ICF
C1D1
EOT visit
30 +/- 5days of last IMP
Treatment will be continued until disease progression or unacceptable toxicity, or patient's decision to discontinue

FIGURE 2

European Patent Office

# EUROPEAN SEARCH REPORT

Application Number
EP 23 30 5290

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2023/099683 A1 (SANOFI SA [FR]) 8 June 2023 (2023-06-08) * claims 1, 6, 10, 12, 16, page 64, lines 18-30, page 77, lines 7-10 * ----- | 1-11,16, 20-26 | INV. A61K47/68 A61P35/00 A61K39/395 |
| X | CRISCITIELLO CARMEN ET AL: "Antibody-drug conjugates in solid tumors: a look into novel targets", JOURNAL OF HEMATOLOGY & ONCOLOGY, vol. 14, no. 1, 1 January 2021 (2021-01-01), page 20, XP093020709, DOI: 10.1186/s13045-021-01035-z Retrieved from the Internet: URL:https://jhoonline.biomedcentral.com/counter/pdf/10.1186/s13045-021-01035-z.pdf> * page 10, left column * ----- | 1-26 | |
| X | GAZZAH A. ET AL: "Safety, pharmacokinetics, and antitumor activity of the anti-CEACAM5-DM4 antibody-drug conjugate tusamitamab ravtansine (SAR408701) in patients with advanced solid tumors: first-in-human dose-escalation study", ANNALS OF ONCOLOGY, vol. 33, no. 4, 1 April 2022 (2022-04-01), pages 416-425, XP055950615, DOI: 10.1016/j.annonc.2021.12.012 * page 423, right column, lower part * ----- -/-- | 1-26 | TECHNICAL FIELDS SEARCHED (IPC) A61K |

The present search report has been drawn up for all claims

1

| Place of search | Date of completion of the search | Examiner |
|---|---|---|
| The Hague | 17 August 2023 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 23 30 5290

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Anonymous: "Tusamitamab Ravtansine (SAR408701) in Combination With Pembrolizumab and Tusamitamab Ravtansine (SAR408701) in Combination With Pembrolizumab and Platinum-based Chemotherapy With or Without Pemetrexed in Patients With NSQ NSCLC (CARMEN-LC05)", , 12 December 2022 (2022-12-12), XP093074465, Retrieved from the Internet: URL:https://classic.clinicaltrials.gov/ct2/history/NCT04524689?A=24&B=24&C=merged#StudyPageTop [retrieved on 2023-08-17] * the whole document * ----- | 1-26 | |
| Y | Anonymous: "Compare Comparison Format: Merged Side-by-Side History of Changes for Study: NCT04394624 SAR408701 in Combination With Ramucirumab in Pre-treated Patients With Non Squamous Non-small Cell Lung Cancer (NSQ NSCLC) (CARMEN-LC04)", , 9 January 2023 (2023-01-09), XP093074466, Retrieved from the Internet: URL:https://classic.clinicaltrials.gov/ct2/history/NCT04394624?A=30&B=30&C=merged#StudyPageTop [retrieved on 2023-08-17] * the whole document * ----- -/-- | 1-26 | TECHNICAL FIELDS SEARCHED (IPC) |

The present search report has been drawn up for all claims

1

| Place of search | Date of completion of the search | Examiner |
|---|---|---|
| The Hague | 17 August 2023 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 30 5290

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PAZ-ARES LUIS ET AL: "75TiP - Open-label, phase II study of tusamitamab ravtansine (SAR408701) in combination with pembrolizumab and with pembrolizumab + platinum-based chemotherapy +/- pemetrexed in patients with CEACAM5-positive nonsquamous NSCLC (CARMEN-LC05)", , vol. 33, 1 April 2022 (2022-04-01), pages 1-1, XP093021030, DOI: 10.1016/annonc/annonc856 Retrieved from the Internet: URL:https://oncologypro.esmo.org/meeting-resources/european-lung-cancer-congress/open-label-phase-ii-study-of-tusamitamab-ravtansine-sar408701-in-combination-with-pembrolizumab-and-with-pembrolizumab-platinum-based-chemothe> * the whole document * ----- | 1-26 | |
| | | | TECHNICAL FIELDS SEARCHED (IPC) |

The present search report has been drawn up for all claims

1

| Place of search | Date of completion of the search | Examiner |
|---|---|---|
| The Hague | 17 August 2023 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 30 5290

This annex lists the patent family members relating to the patent documents cited in the above-mentioned European search report. The members are as contained in the European Patent Office EDP file on
The European Patent Office is in no way liable for these particulars which are merely given for the purpose of information.

17-08-2023

| Patent document cited in search report | Publication date | Patent family member(s) | Publication date |
|---|---|---|---|
| WO 2023099683 A1 | 08-06-2023 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014079886 A
- WO 2020161214 A
- EP 2050764 A1
- US 20050003403 A1
- WO 2009032661 A
- EP 239400 A
- WO 9109967 A
- US 5225539 A
- US 5530101 A
- US 5585089 A
- EP 592106 A
- EP 519596 A
- US 5565332 A
- EP 125023 A
- WO 9602576 A
- WO 9845322 A
- WO 8702671 A
- US 5859205 A
- US 4816567 A
- EP 0173494 A
- US 4424219 A
- US 4256746 A
- US 4294757 A
- US 4307016 A
- US 4313946 A
- US 4315929 A
- US 4331598 A
- US 4361650 A
- US 4362663 A
- US 4364866 A
- US 4450254 A
- US 4322348 A
- US 4371533 A
- US 6333410 B
- US 5475092 A
- US 5585499 A
- US 5846545 A
- WO 2008010101 A
- WO 2004091668 A
- WO 9411026 A
- US 5208020 A


### Non-patent literature cited in the description


- **HAMMARSTRÖM et al.** Tumor markers, Physiology, Pathobiology, Technology and Clinical Applications. AACC Press, 2002, 375
- **YOUNES A ; GOPAL AK ; SMITH SE ; ANSELL SM ; ROSENBLATT JD ; SAVAGE KJ et al.** Results of a pivotal phase II study of brentuximab vedotin for patients with relapsed or refractory Hodgkin's lymphoma. *J Clin Oncol.,* 2012, vol. 30 (18), 2183-9
- **VERMA S ; MILES D ; GIANNI L ; KROP IE ; WELSLAU M ; BASELGA J et al.** Trastuzumab emtansine for HER2-positive advanced breast cancer. *N Engl J Med.,* 2012, vol. 19, 1783-91
- **JUO, PEI-SHOW.** Concise Dictionary of Biomedicine and Molecular Biology. CRC Press, 2002
- The Dictionary of Cell and Molecular Biology. Academic Press, 1999
- Oxford Dictionary Of Biochemistry And Molecular Biology. Oxford University Press, 2000
- **LEFRANC et al.** *Dev. Comp. Immunol.,* 2003, vol. 27 (1), 55-77, www.imgt.org
- **HARMSEN ; DE HAARD HJ.** *Appl. Microbiol. Biotechnol.,* November 2007, vol. 77 (1), 13-22
- **NEEDLEMAN ; WUNSCH.** *J. Mol. Biol.,* 1970, vol. 48, 443
- *CHEMICAL ABSTRACTS,* 2254086-60-5
- **WANG ; GOSH.** *J. Membrane Sci.,* 2008, vol. 318, 311-316
- **CROMWELL et al.** *AAPS Journal,* 2006, vol. 8 (3), E572-E579
- **WALTER et al.** *Anal. Biochem.,* 1993, vol. 212 (2), 469-480
- Therapeutic Antibodies and Protocols. Springer Science, 2009, vol. 525, 445
- *CHEMICAL ABSTRACTS,* 947687-13-0
- Remington's Pharmaceutical Sciences. 1035-1038, 1570-1580